# EUROPEAN PATENT SPECIFICATION

(11) **EP 4 218 752 B1**
(45) Date of publication and mention of the grant of the patent: **05.11.2025**
(21) Application number: 21871680.1
(22) Date of filing: 27.09.2021
(51) Int. Cl.: A61K 31/365, A61K 31/7042, A61K 31/7056, A61K 45/06, G01N 33/577, G01N 33/574, A61P 35/00

(54) **ANTI-CANCER EFFECTS OF MITOCHONDRIAL OXIDATIVE PHOSPHORYLATION PATHWAY INHIBITORS AND MARKERS THEREOF**
ANTIKREBSWIRKUNG VON MITOCHONDRIALEN OXIDATIVEN PHOSPHORYLIERUNGSWEGINHIBITOREN UND DEREN MARKERS
ACTIVITÉ ANTICANCÉREUSE DES INHIBITEURS DE LA VOIE DE PHOSPHORYLATION OXYDATIVE MITOCHONDRIALE ET LEURS MARQUEURS

(30) Priority: 27.09.2020 CN 202011034567
(43) Date of publication of application: 02.08.2023
(73) Proprietor: Shanghai Shijiang Biotechnology Co., Ltd, Shanghai 200333 (CN)
(72) Inventor: SHI, Yufeng, Nanjing, Jiangsu 200120 (CN); MA, Wenjiang, Nanjing, Jiangsu 200120 (CN); JIANG, Cizhong, Nanjing, Jiangsu 200120 (CN); LIU, Wenju, Nanjing, Jiangsu 200120 (CN); WU, Changqing, Nanjing, Jiangsu 200120 (CN); LIU, Yu'e, Nanjing, Jiangsu 200120 (CN); LU, Shaojuan, Nanjing, Jiangsu 200120 (CN)
(74) Representative: reuteler & cie SA
(86) International application number: PCT/CN2021/121088
(87) International publication number: WO 2022/063311

(56) References cited:
- EP-A1- 4 265 249
- EP-A1- 4 265 613
- WO-A2-2020/086830
- CN-A- 106 248 946
- CN-A- 106 248 946
- KR-A- 20180 044 617
- WU CHANGQING ET AL: "NNMT-DNMT1 Axis is Essential for Maintaining Cancer Cell Sensitivity to Oxidative Phosphorylation Inhibition", vol. 10, no. 1, 1 January 2023 (2023-01-01), XP093129087, ISSN: 2198-3844, Retrieved from the Internet <URL:https://onlinelibrary.wiley.com/doi/full-xml/10.1002/advs.202202642> DOI: 10.1002/advs.202202642
- KANAKKANTHARA ARUN ET AL: "BRCA1 Deficiency Upregulates NNMT, Which Reprograms Metabolism and Sensitizes Ovarian Cancer Cells to Mitochondrial Metabolic Targeting Agents", vol. 79, no. 23, 1 December 2019 (2019-12-01), US, pages 5920 - 5929, XP055939523, ISSN: 0008-5472, Retrieved from the Internet <URL:https://aacrjournals.org/cancerres/article-pdf/79/23/5920/2786362/5920.pdf> DOI: 10.1158/0008-5472.CAN-19-1405
- LIU YU'E, SHI YUFENG: "Mitochondria as a target in cancer treatment", MEDCOMM, vol. 1, no. 2, 1 September 2020 (2020-09-01), pages 129 - 139, XP055914469, ISSN: 2688-2663, DOI: 10.1002/mco2.16
- RAMSDEN DAVID B., ROSEMARY H WARING, DAVID J BARLOW, RICHARD B PARSONS: "Nicotinamide N-Methyltransferase in Health and Cancer", INTERNATIONAL JOURNAL OF TRYPTOPHAN RESEARCH, vol. 10, 30 June 2017 (2017-06-30), pages 1 - 19, XP055914471, DOI: 10.1177/1178646917691739
- ZHANG JUN, WANG YANZHONG, LI GUILING, YU HAITAO, XIE XINYOU: "Down-Regulation of Nicotinamide N-methyltransferase Induces Apoptosis in Human Breast Cancer Cells via the Mitochondria-Mediated Pathway", PLOS ONE, vol. 9, no. 2, 18 February 2014 (2014-02-18), pages e89202 - 13019, XP055914472, DOI: 10.1371/journal.pone.0089202

## Description

### FIELD OF THE INVENTION

The present invention relates to the field of medicine. Specifically, the present invention relates to marker for determining anti-cancer effects of mitochondrial oxidative phosphorylation pathway inhibitor.

### BACKGROUND TECHNOLOGY

Mitochondria is ubiquitous in eukaryotic cells, which provides energy for the activities of cells and other intermediate products necessary for cell growth. Mitochondria, as the energy factory and material source in cell, is an indispensable organelle for tumorigenesis of tumor cell. The inhibition of mitochondrial function can effectively inhibit the occurrence and development of tumor, reduce the malignant degree of tumor and prolong the survival period of patient.

Oxidative Phosphorylation (OXPHOS) is one of the most important pathways in mitochondria, which utilizes NADH and FADH derived from pathways such as the tricarboxylic acid cycle and fat oxidation to produce ATP. The mitochondrial oxidative phosphorylation pathway is composed of more than 90 proteins, which form five protein complexes, complexes I, II, III, IV and V, respectively. The first four protein complexes (complexes I, II, III and IV), also known as the electron transport chain, receive electron from electron donor NADH and FADH, and transfer them to oxygen. In the process of electron transfer, hydrogen ion is pumped from the mitochondrial inner membrane to the intermembrane space between the mitochondrial inner membrane and the mitochondrial outer membrane, thereby forming a hydrogen ion gradient and potential difference inside and outside the inner membrane. The energy stored in the mitochondria membrane potential drives complex V in the oxidative phosphorylation pathway to generate ATP. Tumor cells with high malignancy and stem cell properties extremely depend on this pathway for survival, the inhibition of this pathway can effectively kill such tumor cells, thus overcoming the recurrence of malignant cancer. The drugs targeting tumor energy metabolism become an important direction in the development of new anticancer drugs.

In recent years, a number of small molecules targeting target mitochondrial oxidative phosphorylation pathway have been discovered. These small molecules have achieved significant anticancer effects in various tumor models and clinical experiments, especially for some recurrent or metastatic malignant cancers, and can provide effective solutions for unmet clinical needs. For example, a study published in Nature has found that the tumor cells causing the recurrence of pancreatic cancer are very sensitive to Oligomycin, an oxidative phosphorylation pathway inhibitor, which can effectively kill tumor cells and inhibit tumor recurrence, see Viale, A., Pettazzoni, P., Lyssiotis, C. et al. Oncogene ablation-resistant pancreatic cancer cells depend on mitochondrial function. Nature 514, 628-632 (2014). Another study published in Nature has found that Gboxin, a mitochondrial oxidative phosphorylation pathway inhibitor, has excellent killing effect on brain tumors and other tumors, and can effectively inhibit tumor growth, see Shi, Y., Lim, S.K., Liang, Q. et al. Gboxin is an oxidative phosphorylation inhibitor that targets glioblastoma. Nature 567, 341-346 (2019). A study published in Nature Medicine has found that IACS-010759, a mitochondrial oxidative phosphorylation pathway inhibitor, has excellent inhibitory effect on brain tumors and acute myeloid leukemia, see Molina, J.R., Sun, Y., Protopopova, M. et al. An inhibitor of oxidative phosphorylation exploits cancer vulnerability. Nat Med 24, 1036-1046 (2018).* CN106248946 describes an enzyme-linked immunoassay kit composed of a nicotinamide Nmethyltransferase standard substance, a contrast buffer solution, an ELISA plate wrapped by a nicotinamide N-Methyltransferase monoclonal antibody A, a nicotinamide NMethyltransferase monoclonal antibody B solution marked by horse radish peroxidase and an auxiliary reagent.

Many studies and clinical cases have found that various types of anti-tumor drugs cannot be effective in all tumors, some tumor cells are not sensitive to specific anti-tumor drugs. The specific anti-tumor drugs used in tumor cells or tumor patients that are not sensitive to drugs can not have a good therapeutic effect, and the treatment opportunity is delayed, causing a great negative impact on tumor patients. In particular, up to now, specific tumor markers for determining anti-cancer effects of novel anticancer drugs such as mitochondrial oxidative phosphorylation pathway inhibitor is little known.

However, at present, the mechanism of the sensitivity of tumor cells to oxidative phosphorylation pathway inhibitor is not clear, and tumor markers related to oxidative phosphorylation pathway have not been reported. The discovery of related tumor markers can provide precise guidance for the use of specific anti-tumor drugs such as mitochondrial oxidative phosphorylation pathway inhibitors, thus achieving the precise treatment on cancer patients, significantly improving the clinical treatment effect of drugs on cancer patients, avoiding the use of drugs for patients who are not suitable for using such specific anti-tumor drugs, and avoiding delaying the treatment time. Therefore, there is a need in the art to develop a marker that can effectively guide the use of anti-tumor drugs such as mitochondrial oxidative phosphorylation pathway inhibitors, so as to accurately guide the use of such drugs and significantly improve the therapeutic effect.

### SUMMARY OF THE INVENTION

It is an object of the present invention to provide a marker for determining whether a mitochondrial oxidative phosphorylation pathway inhibitor is suitable for use in the prevention and/or treatment of tumor patient to achieve precise treatment on tumors, the marker is the expression level or activity of mitochondrial oxidative phosphorylation pathway, the expression level of NNMT gene, the expression level of DNA methylase, the expression level of UHRF1, the methylation level of nucleotide site of NNMT gene, and/or the methylation level of DNA CpG site of NNMT gene. The mitochondrial oxidative phosphorylation pathway inhibitor has significant treatment effect on tumors with up-regulation of mitochondrial oxidative phosphorylation pathway, low or no expression of NNMT gene, high expression of DNA methylase, high expression of UHRFl, high methylation level of nucleotide site of NNMT gene, and/or high methylation level of DNA CpG site of NNMT gene.

In the first aspect of the present invention, it provides a mitochondrial oxidative phosphorylation pathway inhibitor as defined in claim 1 useful in the preparation of a composition or a preparation for preventing and/or treating tumor.

In another preferred embodiment, the tumor is human-derived tumor.

In another preferred embodiment, the tumor is human tumor.

In another preferred embodiment, the tumor comprises tumor with up-regulation of mitochondrial oxidative phosphorylation pathway.

In another preferred embodiment, the up-regulation of mitochondrial oxidative phosphorylation pathway means that the expression level or activity of mitochondrial oxidative phosphorylation pathway in a cell ( e.g., tumor cell ) is higher than the expression level or activity of mitochondrial oxidative phosphorylation pathway in the same type of cell or a normal cell ( e.g., para-tumor tissue cell).

In another preferred embodiment, the up-regulation of mitochondrial oxidative phosphorylation pathway comprises the high expression level or high activity of mitochondrial oxidative phosphorylation pathway.

In another preferred embodiment, the up-regulation of mitochondrial oxidative phosphorylation pathway means that the ratio (H1/H0) of the expression level or activity H1 of mitochondrial oxidative phosphorylation pathway in a cell ( e.g., tumor cell ) to the expression level or activity H0 of mitochondrial oxidative phosphorylation pathway in the same type of cell or a normal cell (e.g., para-tumor tissue cell) is >1.0, preferably ≥1.2, more preferably ≥1.5, more preferably ≥2, more preferably ≥3, more preferably ≥5, more preferably ≥8, more preferably ≥10, more preferably ≥15, more preferably ≥20, more preferably ≥30, more preferably ≥50.

In another preferred embodiment, the same type of cell refers to the cell ( e.g., the same type of tumor cell ) with normal expression or normal activity of mitochondrial oxidative phosphorylation pathway.

In another preferred embodiment, the same type of cell refers to the same type of cell with normal expression or normal activity of mitochondrial oxidative phosphorylation pathway.

In another preferred embodiment, the normal cell refers to normal tissue cell (e.g., tumor origin cell, tumor-adjacent cell or para-tumor tissue cell) with normal expression or normal activity of mitochondrial oxidative phosphorylation pathway.

In another preferred embodiment, the tumor comprises tumor with low or no expression of NNMT gene.

In another preferred embodiment, the NNMT gene is human-derived NNMT gene.

In another preferred embodiment, the NNMT gene is human NNMT gene.

In another preferred embodiment, the tumor comprises tumor with high expression of DNA methylase.

In another preferred embodiment, the DNA methylase is selected from the group consisting of DNMT1, DNMT3a, DNMT3b, and combinations thereof.

In another preferred embodiment, the tumor comprises tumor with high expression of DNMT1.

In another preferred embodiment, the tumor comprises tumor with high expression of DNMT3a.

In another preferred embodiment, the tumor comprises tumor with high expression of DNMT3b.

In another preferred embodiment, the tumor comprises tumor with high expression of UHRFl.

In another preferred embodiment, the tumor comprises tumor with high methylation level of nucleotide site of NNMT gene and/or high methylation level of DNA CpG site of NNMT gene.

In another preferred embodiment, the tumor comprises tumor with high methylation level of nucleotide site of NNMT gene.

In another preferred embodiment, the tumor comprises tumor with high methylation level of DNA CpG site of NNMT gene.

In another preferred embodiment, the tumor with low or no expression of NNMT gene means that no NNMT protein can be detected in 1 µg of protein extracted from tumor using NNMT antibody, preferably in 5 µg of protein extracted from tumor, more preferably in 10 µg of protein extracted from tumor, more preferably in 100 µg of protein extracted from tumor, preferably in 1000 µg of protein extracted from tumor.

In another preferred embodiment, the tumor with low or no expression of NNMT gene means the expression level of NNMT gene in tumor cell is lower than that in the same type of cell or a normal cell (e.g., para-tumor tissue cell).

In another preferred embodiment, the tumor with low or no expression of NNMT gene means the ratio (E1/E0) of the expression level E1 of NNMT gene in the tumor cell to the expression level E0 of NNMT gene in the same type of cell or a normal cell (e.g., para-tumor tissue cell) is <1.0.

In another preferred embodiment, the low or no expression of NNMT gene means the ratio (E1/E0) of the expression level E1 of NNMT gene in a cell ( e.g., tumor cell ) to the expression level E0 of NNMT gene in the same type of cell or a normal cell (e.g., para-tumor tissue cell) is <1.0. preferably ≤ 0.7, more preferably ≤ 0.6, more preferably ≤ 0.5, more preferably ≤ 0.4, more preferably ≤ 0.3, more preferably ≤ 0.2, more preferably ≤ 0.1, more preferably ≤ 0.05, more preferably ≤ 0.01, more preferably ≤ 0.005, more preferably ≤ 0.001, more preferably ≤ 0.0001, more preferably ≤ 0.00001 , more preferably ≤ 0.000001, more preferably ≤ 0.0000001.

In another preferred embodiment, the same type of cell refers to the cell ( e.g., the same type of tumor cell ) with normal expression of NNMT gene.

In another preferred embodiment, the same type of cell refers to the same type of cell with normal expression of NNMT gene.

In another preferred embodiment, the normal cell refers to normal tissue cell (e.g., tumor origin cell, tumor-adjacent cell or para-tumor tissue cell) with normal expression of NNMT gene.

In another preferred embodiment, E0 refers to the expression level of NNMT gene in the cell with normal expression of NNMT gene.

In another preferred embodiment, the cell with normal expression of NNMT gene comprises the cell that is not sensitive to mitochondrial oxidative phosphorylation pathway inhibitor.

In another preferred embodiment, the tumor with high expression of DNA methylase means that DNA methylase can be detected in 20 µg of protein extracted from tumor using DNA methylase antibody, preferably in 5 µg of protein extracted from tumor, more preferably in 1 µg of protein extracted from tumor, more preferably in 0.2 µg of protein extracted from tumor, more preferably in 0.05 µg of protein extracted from tumor, more preferably in 0.01 µg of protein extracted from tumor.

In another preferred embodiment, the tumor with high expression of DNA methylase means the expression level of DNA methylase in tumor cell is higher than that in the same type of cell or a normal cell (e.g., para-tumor tissue cell).

In another preferred embodiment, the tumor with high expression of DNA methylase means the ratio (A1/A0) of the expression level A1 of DNA methylase in the tumor cell to the expression level A0 of DNA methylase in the same type of cell or a normal cell (e.g., para-tumor tissue cell) is > 1.0, preferably ≥ 1.2, more preferably ≥ 1.5, more preferably ≥ 2, more preferably ≥ 3, more preferably ≥ 5, more preferably ≥ 8, more preferably ≥ 10, more preferably ≥ 15, more preferably ≥ 20, more preferably ≥ 30, more preferably ≥ 50.

In another preferred embodiment, the same type of cell refers to the cell ( e.g., the same type of tumor cell ) with normal expression of DNA methylase.

In another preferred embodiment, the same type of cell refers to the same type of cell with normal expression of DNA methylase.

In another preferred embodiment, the normal cell refers to normal tissue cell (e.g., tumor origin cell, tumor-adjacent cell or para-tumor tissue cell) with normal expression of DNA methylase.

In another preferred embodiment, A0 refers to the expression level of DNA methylase in the cell with normal expression of DNA methylase.

In another preferred embodiment, the cell with normal expression of DNA methylase comprises the cell that is not sensitive to mitochondrial oxidative phosphorylation pathway inhibitor.

In another preferred embodiment, the tumor with high expression of DNMT1 means that DNMT1 protein can be detected in 20 µg of protein extracted from tumor using DNMT1 antibody, preferably in 5 µg of protein extracted from tumor, more preferably in 1 µg of protein extracted from tumor, more preferably in 0.2 µg of protein extracted from tumor, more preferably in 0.05 µg of protein extracted from tumor, more preferably in 0.01 µg of protein extracted from tumor.

In another preferred embodiment, the tumor with high expression of DNMT1 means the expression level of DNMT1 in tumor cell is higher than that in the same type of cell or a normal cell (e.g., para-tumor tissue cell).

In another preferred embodiment, the tumor with high expression of DNMT1 means the ratio (B1/B0) of the expression level B1 of DNMT1 in the tumor cell to the expression level B0 of DNMT1 in the same type of cell or a normal cell (e.g., para-tumor tissue cell) is > 1.0, preferably ≥ 1.2, more preferably ≥ 1.5, more preferably ≥ 2, more preferably ≥ 3, more preferably ≥ 5, more preferably ≥ 8, more preferably ≥ 10, more preferably ≥ 15, more preferably ≥ 20, more preferably ≥ 30, more preferably ≥ 50.

In another preferred embodiment, the same type of cell refers to the cell ( e.g., the same type of tumor cell ) with normal expression of DNMT1.

In another preferred embodiment, the same type of cell refers to the same type of cell with normal expression of DNMT1.

In another preferred embodiment, the normal cell refers to normal tissue cell (e.g., tumor origin cell, tumor-adjacent cell or para-tumor tissue cell) with normal expression of DNMT1.

In another preferred embodiment, B0 refers to the expression level of DNMT1 in the cell with normal expression of DNMT1.

In another preferred embodiment, the cell with normal expression of DNMT1 comprises the cell that is not sensitive to mitochondrial oxidative phosphorylation pathway inhibitor.

In another preferred embodiment, the tumor with high expression of DNMT3a means that DNMT3a protein can be detected in 20 µg of protein extracted from tumor using DNMT3a antibody, preferably in 5 µg of protein extracted from tumor, more preferably in 1 µg of protein extracted from tumor, more preferably in 0.2 µg of protein extracted from tumor, more preferably in 0.05 µg of protein extracted from tumor, more preferably in 0.01 µg of protein extracted from tumor.

In another preferred embodiment, the tumor with high expression of DNMT3a means the expression level of DNMT3a in tumor cell is higher than that in the same type of cell or a normal cell (e.g., para-tumor tissue cell).

In another preferred embodiment, the tumor with high expression of DNMT3a means the ratio (C1/C0) of the expression level C1 of DNMT3a in the tumor cell to the expression level C0 of DNMT3a in the same type of cell or a normal cell (e.g., para-tumor tissue cell) is > 1.0, preferably ≥ 1.2, more preferably ≥ 1.5, more preferably ≥ 2, more preferably ≥ 3, more preferably ≥ 5, more preferably ≥ 8, more preferably ≥ 10, more preferably ≥ 15, more preferably ≥ 20, more preferably ≥ 30, more preferably ≥ 50.

In another preferred embodiment, the same type of cell refers to the cell ( e.g., the same type of tumor cell ) with normal expression of DNMT3a.

In another preferred embodiment, the same type of cell refers to the same type of cell with normal expression of DNMT3a.

In another preferred embodiment, the normal cell refers to normal tissue cell (e.g., tumor origin cell, tumor-adjacent cell or para-tumor tissue cell) with normal expression of DNMT3a.

In another preferred embodiment, C0 refers to the expression level of DNMT3a in the cell with normal expression of DNMT3a.

In another preferred embodiment, the cell with normal expression of DNMT3a comprises the cell that is not sensitive to mitochondrial oxidative phosphorylation pathway inhibitor.

In another preferred embodiment, the tumor with high expression of DNMT3b means that DNMT3b protein can be detected in 20 µg of protein extracted from tumor using DNMT3b antibody, preferably in 5 µg of protein extracted from tumor, more preferably in 1 µg of protein extracted from tumor, more preferably in 0.2 µg of protein extracted from tumor, more preferably in 0.05 µg of protein extracted from tumor, more preferably in 0.01 µg of protein extracted from tumor.

In another preferred embodiment, the tumor with high expression of DNMT3b means the expression level of DNMT3b in tumor cell is higher than that in the same type of cell or a normal cell (e.g., para-tumor tissue cell).

In another preferred embodiment, the tumor with high expression of DNMT3b means the ratio (D1/D0) of the expression level D1 of DNMT3b in the tumor cell to the expression level D0 of DNMT3b in the same type of cell or a normal cell (e.g., para-tumor tissue cell) is > 1.0, preferably ≥ 1.2, more preferably ≥ 1.5, more preferably ≥ 2, more preferably ≥ 3, more preferably ≥ 5, more preferably ≥ 8, more preferably ≥ 10, more preferably ≥ 15, more preferably ≥ 20, more preferably ≥ 30, more preferably ≥ 50.

In another preferred embodiment, the same type of cell refers to the cell ( e.g., the same type of tumor cell ) with normal expression of DNMT3b.

In another preferred embodiment, the same type of cell refers to the same type of cell with normal expression of DNMT3b.

In another preferred embodiment, the normal cell refers to normal tissue cell (e.g., tumor origin cell, tumor-adjacent cell or para-tumor tissue cell) with normal expression of DNMT3b

In another preferred embodiment, D0 refers to the expression level of DNMT3b in the cell with normal expression of DNMT3b.

In another preferred embodiment, the cell with normal expression of DNMT3b comprises the cell that is not sensitive to mitochondrial oxidative phosphorylation pathway inhibitor.

In another preferred embodiment, the tumor with high expression of UHRF1 means that UHRF1 protein can be detected in 20 µg of protein extracted from tumor using UHRF1 antibody, preferably in 5 µg of protein extracted from tumor, more preferably in 1 µg of protein extracted from tumor, more preferably in 0.2 µg of protein extracted from tumor, more preferably in 0.05 µg of protein extracted from tumor, more preferably in 0.01 µg of protein extracted from tumor.

In another preferred embodiment, the tumor with high expression of UHRF1 means the expression level of UHRF1 in tumor cell is higher than that in the same type of cell or a normal cell (e.g., para-tumor tissue cell).

In another preferred embodiment, the tumor with high expression of UHRF1 means the ratio (F1/F0) of the expression level F1 of UHRF1 in the tumor cell to the expression level F0 of UHRF1 in the same type of cell or a normal cell (e.g., para-tumor tissue cell) is > 1.0, preferably ≥ 1.2, more preferably ≥ 1.5, more preferably ≥ 2, more preferably ≥ 3, more preferably ≥ 5, more preferably ≥ 8, more preferably ≥ 10, more preferably ≥ 15, more preferably ≥ 20, more preferably ≥ 30, more preferably ≥ 50.

In another preferred embodiment, the same type of cell refers to the cell ( e.g., the same type of tumor cell ) with normal expression of UHRF1.

In another preferred embodiment, the same type of cell refers to the same type of cell with normal expression of UHRF1.

In another preferred embodiment, the normal cell refers to normal tissue cell (e.g., tumor origin cell, tumor-adjacent cell or para-tumor tissue cell) with normal expression of UHRF1

In another preferred embodiment, F0 refers to the expression level of UHRF1 in the cell with normal expression of UHRF1.

In another preferred embodiment, the cell with normal expression of UHRF1 comprises the cell that is not sensitive to mitochondrial oxidative phosphorylation pathway inhibitor.

In another preferred embodiment, the high methylation level of nucleotide site of NNMT gene means the methylation level of nucleotide site of NNMT gene in a cell ( e.g., tumor cell ) is higher than that in the same type of cell or a normal cell (e.g., para-tumor tissue cell).

In another preferred embodiment, the high methylation level of nucleotide site of NNMT gene means the ratio (L1/L0) of the methylation level L1 of nucleotide site of NNMT gene in a cell ( e.g., tumor cell ) to the methylation level L0 of nucleotide site of NNMT gene in the same type of cell or a normal cell (e.g., para-tumor tissue cell) is > 1.0, preferably ≥ 1.2, more preferably ≥ 1.5, more preferably ≥ 2, more preferably ≥ 3, more preferably ≥ 5, more preferably ≥ 8, more preferably ≥ 10, more preferably ≥ 15, more preferably ≥ 20, more preferably ≥ 30, more preferably ≥ 50.

In another preferred embodiment, the high methylation level of nucleotide site of NNMT gene means the methylation level of nucleotide site of NNMT gene in a cell ( e.g., tumor cell ) is ≥ 1%, more preferably ≥ 3%, more preferably ≥ 5%, more preferably ≥ 10%, more preferably ≥ 15%, more preferably ≥ 20%, more preferably ≥ 25%, more preferably ≥ 30%, more preferably ≥ 40%, more preferably ≥ 50%.

In another preferred embodiment, the same type of cell refers to the cell ( e.g., the same type of tumor cell ) with normal methylation level of nucleotide site of NNMT gene.

In another preferred embodiment, the same type of cell refers to the same type of cell with normal methylation level of nucleotide site of NNMT gene.

In another preferred embodiment, the normal cell refers to normal tissue cell (e.g., tumor origin cell, tumor-adjacent cell or para-tumor tissue cell) with normal methylation level of nucleotide site of NNMT gene.

In another preferred embodiment, the cell with normal methylation level of nucleotide site of NNMT gene comprises the cell that is not sensitive to mitochondrial oxidative phosphorylation pathway inhibitor.

In another preferred embodiment, the high methylation level of nucleotide site of NNMT gene means the methylation level (M%) of nucleotide site of NNMT gene in a cell ( e.g., tumor cell ) is ≥ 3% and ≤ M1%, wherein M1 is any positive integer from 3 to 100.

In another preferred embodiment, M1 is 5, 10, 15, 20, 25, 30, 35, 40, 45, 50, 55, 60, 65, 70, 80, 85, 90, 95 or 100.

In another preferred embodiment, the methylation level of nucleotide site of NNMT gene refers to the ratio of the number of methylated nucleotides to the number of all nucleotides in the NNMT gene.

In another preferred embodiment, the methylation level of nucleotide site of NNMT gene comprises the methylation level of nucleotide site in promoter region of NNMT gene.

In another preferred embodiment, the nucleotide sequence of the promoter region of NNMT gene is as shown in SEQ ID NO: 1.

In another preferred embodiment, the methylation level of nucleotide site of NNMT gene comprises the methylation level of nucleotide sites from 1050 bp before the transcription start site to 499 bp after the transcription start site in NNMT gene.

In another preferred embodiment, the nucleotide sites from 1050 bp before the transcription start site to 499 bp after the transcription start site in NNMT gene is sites 951-2500 of nucleotide sequence as shown in SEQ ID NO: 1.

In another preferred embodiment, the methylation level of nucleotide site of NNMT gene comprises the methylation level of nucleotide sites from 1050 bp to 193 bp before the transcription start site in NNMT gene.

In another preferred embodiment, the nucleotide sites from 1050 bp to 193 bp before the transcription start site in NNMT gene is sites 951-1808 of nucleotide sequence as shown in SEQ ID NO: 1.

In another preferred embodiment, the methylation level of nucleotide site of NNMT gene comprises the methylation level of nucleotide sites from 840 bp to 469 bp before the transcription start site in NNMT gene.

In another preferred embodiment, the sites from 840 bp to 469 bp before the transcription start site in NNMT gene is sites 1161-1532 of nucleotide sequence as shown in SEQ ID NO: 1.

In another preferred embodiment, the methylation level of nucleotide site of NNMT gene comprises the methylation level of nucleotide sites between any two sites (including the two sites) selected from group consisting of site 114165695, site 114165730, site 114165769, site 114165804, site 114165938, site 114166050 and site 114166066 on human chromosome 11.

In another preferred embodiment, the methylation level of nucleotide site of NNMT gene comprises the methylation level of nucleotide site of one or more (e.g., 2, 3, 4, 5, 6, or 7) of site 114165695, site 114165730, site 114165769, site 114165804, site 114165938, site 114166050 and site 114166066 on human chromosome 11.

In another preferred embodiment, the methylation level of nucleotide site of NNMT gene comprises the methylation level of nucleotide sites selected from group consisting of site 114165695 on human chromosome 11, site 114165730 on human chromosome 11, site 114165769 on human chromosome 11, site 114165804 on human chromosome 11, site 114165938 on human chromosome 11, site 114166050 on human chromosome 11, site 114166066 on human chromosome 11, and combinations thereof.

In another preferred embodiment, the methylation level of nucleotide site of NNMT gene comprises the methylation level of nucleotide sites between any two sites (including the two sites) selected from group consisting of site 1161, site 1196, site 1235, site 1270, site 1404, site 1516 and site 1532 in nucleotide sequence of SEQ ID NO: 1.

In another preferred embodiment, the methylation level of nucleotide site of NNMT gene comprises the methylation level of nucleotide site of one or more (e.g., 2, 3, 4, 5, 6, or 7) of site 1161, site 1196, site 1235, site 1270, site 1404, site 1516 and site 1532 in nucleotide sequence of SEQ ID NO: 1.

In another preferred embodiment, the methylation level of nucleotide site of NNMT gene comprises the methylation level of nucleotide sites selected from group consisting of site 1161 in SEQ ID NO: 1, site 1196 in SEQ ID NO: 1, site 1235 in SEQ ID NO: 1, site 1270 in SEQ ID NO: 1, site 1404 in SEQ ID NO: 1, site 1516 in SEQ ID NO: 1, site 1532 in SEQ ID NO: 1, and combinations thereof.

In another preferred embodiment, the high methylation level of DNA CpG site of NNMT gene means the methylation level of DNA CpG site of NNMT gene in a cell ( e.g., tumor cell ) is higher than that in the same type of cell or a normal cell (e.g., para-tumor tissue cell).

In another preferred embodiment, the high methylation level of DNA CpG site of NNMT gene means the ratio (W1/WO) of the methylation level W1 of DNA CpG site of NNMT gene in a cell ( e.g., tumor cell ) to the methylation level WO of DNA CpG site of NNMT gene in the same type of cell or a normal cell (e.g., para-tumor tissue cell) is > 1.0, preferably ≥ 1.2, more preferably ≥ 1.5, more preferably ≥ 2, more preferably ≥ 3, more preferably ≥ 5, more preferably ≥ 8, more preferably ≥ 10, more preferably ≥ 15, more preferably ≥ 20, more preferably ≥ 30, more preferably ≥ 50.

In another preferred embodiment, the high methylation level of DNA CpG site of NNMT gene means the methylation level of DNA CpG site of NNMT gene in a cell ( e.g., tumor cell ) is ≥ 1%, more preferably ≥ 3%, more preferably ≥ 5%, more preferably ≥ 10%, more preferably ≥ 15%, more preferably ≥ 20%, more preferably ≥ 25%, more preferably ≥ 30%, more preferably ≥ 40%, more preferably ≥ 50%.

In another preferred embodiment, the same type of cell refers to the cell ( e.g., the same type of tumor cell ) with normal methylation level of DNA CpG site of NNMT gene.

In another preferred embodiment, the same type of cell refers to the same type of cell with normal methylation level of DNA CpG site of NNMT gene.

In another preferred embodiment, the normal cell refers to normal tissue cell (e.g., tumor origin cell, tumor-adjacent cell or para-tumor tissue cell) with normal methylation level of DNA CpG site of NNMT gene.

In another preferred embodiment, the cell with normal methylation level of DNA CpG site of NNMT gene comprises the cell that is not sensitive to mitochondrial oxidative phosphorylation pathway inhibitor.

In another preferred embodiment, the high methylation level of DNA CpG site of NNMT gene means the methylation level (M%) of DNA CpG site of NNMT gene in a cell ( e.g., tumor cell ) is ≥ 3% and ≤ M2%, wherein M2 is any positive integer from 3 to 100.

In another preferred embodiment, M2 is 5, 10, 15, 20, 25, 30, 35, 40, 45, 50, 55, 60, 65, 70, 80, 85, 90, 95 or 100.

In another preferred embodiment, the methylation level of CpG site refers to the ratio of the number of methylated CpG nucleotides to the number of all nucleotides in a gene.

In another preferred embodiment, the methylation level of DNA CpG site of NNMT gene refers to the ratio of the number of methylated CpG nucleotides to the number of all nucleotides in the NNMT gene.

In another preferred embodiment, the methylation level of CpG site refers to the ratio of the number of methylated CpG nucleotides to the number of all CpG nucleotides in a gene.

In another preferred embodiment, the methylation level of DNA CpG site of NNMT gene refers to the ratio of the number of methylated CpG nucleotides to the number of all CpG nucleotides in the NNMT gene.

In another preferred embodiment, the methylation level of DNA CpG site refers to the ratio of the number of methylated CpG sites to the number of all CpG sites in a DNA.

In another preferred embodiment, the methylation level of DNA CpG site refers to the ratio of the number of methylated CpG nucleotides to the number of all nucleotides in a DNA.

In another preferred embodiment, the methylation level of DNA CpG site refers to the ratio of the number of methylated CpG nucleotides to the number of all CpG nucleotides in a DNA.

In another preferred embodiment, the methylation level of DNA CpG site of NNMT gene refers to the ratio of the number of methylated CpG sites to the number of all CpG sites in the NNMT gene.

In another preferred embodiment, the methylation level of DNA CpG site of NNMT gene refers to the ratio of the number of methylated CpG nucleotides to the number of all CpG nucleotides in the NNMT gene.

In another preferred embodiment, the methylation level of DNA CpG site of NNMT gene comprises the methylation level of DNA CpG site in promoter region of NNMT gene.

In another preferred embodiment, the nucleotide sequence of the promoter region of NNMT gene is as shown in SEQ ID NO: 1.

In another preferred embodiment, the methylation level of DNA CpG site of NNMT gene comprises the methylation level of the DNA CpG sites from 1050 bp before the transcription start site to 499 bp after the transcription start site in NNMT gene.

In another preferred embodiment, the sites from 1050 bp before the transcription start site to 499 bp after the transcription start site in NNMT gene is sites 951-2500 of nucleotide sequence as shown in SEQ ID NO: 1.

In another preferred embodiment, the methylation level of DNA CpG site of NNMT gene comprises the methylation level of the DNA CpG sites from 1050 bp to 193 bp before the transcription start site in NNMT gene.

In another preferred embodiment, the sites from 1050 bp to 193 bp before the transcription start site in NNMT gene is sites 951-1808 of nucleotide sequence as shown in SEQ ID NO: 1.

In another preferred embodiment, the methylation level of DNA CpG site of NNMT gene comprises the methylation level of the DNA CpG sites from 840 bp to 469 bp before the transcription start site in NNMT gene.

In another preferred embodiment, the sites from 840 bp to 469 bp before the transcription start site in NNMT gene is sites 1161-1532 of nucleotide sequence as shown in SEQ ID NO: 1.

In another preferred embodiment, the methylation level of DNA CpG site of NNMT gene comprises the methylation level of DNA CpG sites between any two sites (including the two sites) selected from group consisting of site 114165695, site 114165730, site 114165769, site 114165804, site 114165938, site 114166050 and site 114166066 on human chromosome 11.

In another preferred embodiment, the methylation level of DNA CpG site of NNMT gene comprises the methylation level of nucleotide site of one or more (e.g., 2, 3, 4, 5, 6, or 7) of site 114165695, site 114165730, site 114165769, site 114165804, site 114165938, site 114166050 and site 114166066 on human chromosome 11.

In another preferred embodiment, the methylation level of DNA CpG site of NNMT gene comprises the methylation level of nucleotide sites selected from group consisting of site 114165695 on human chromosome 11, site 114165730 on human chromosome 11, site 114165769 on human chromosome 11, site 114165804 on human chromosome 11, site 114165938 on human chromosome 11, site 114166050 on human chromosome 11, site 114166066 on human chromosome 11, and combinations thereof.

In another preferred embodiment, the methylation level of DNA CpG site of NNMT gene comprises the methylation level of nucleotide sites between any two sites (including the two sites) selected from group consisting of site 1161, site 1196, site 1235, site 1270, site 1404, site 1516 and site 1532 in nucleotide sequence of SEQ ID NO: 1.

In another preferred embodiment, the methylation level of DNA CpG site of NNMT gene comprises the methylation level of the nucleotide site of one or more (e.g., 2, 3, 4, 5, 6, or 7) of site 1161, site 1196, site 1235, site 1270, site 1404, site 1516 and site 1532 in nucleotide sequence of SEQ ID NO: 1.

In another preferred embodiment, the methylation level of DNA CpG site of NNMT gene comprises the methylation level of nucleotide sites selected from group consisting of site 1161 in SEQ ID NO: 1, site 1196 in SEQ ID NO: 1, site 1235 in SEQ ID NO: 1, site 1270 in SEQ ID NO: 1, site 1404 in SEQ ID NO: 1, site 1516 in SEQ ID NO: 1, site 1532 in SEQ ID NO: 1, and combinations thereof.

In another preferred embodiment, the tumor is selected from the group consisting of lung cancer, renal carcinoma, breast cancer, colon cancer, rectal cancer, colorectal cancer, lymphoma, leukemia, pancreatic cancer, brain tumor, liver cancer, prostate cancer, melanoma, and combinations thereof.

In another preferred embodiment, the lung cancer is selected from the group consisting of non-small cell lung cancer, small cell lung cancer, metastatic lung cancer, and combinations thereof.

In another preferred embodiment, the colon cancer comprises colon adenocarcinoma.

In another preferred embodiment, the rectal cancer comprises rectal adenocarcinoma.

In another preferred embodiment, the colorectal cancer comprises colorectal adenocarcinoma.

In another preferred embodiment, the lymphoma is selected from the group consisting of B-cell lymphoma, T-cell lymphoma, skin T-cell lymphoma, large cell lymphoma, histiocytic lymphoma, and combinations thereof.

In another preferred embodiment, the lymphoma comprises diffuse large B-cell lymphoma.

In another preferred embodiment, the brain tumor is selected from the group consisting of glioblastoma, neuroglioma, and combination thereof.

In another preferred embodiment, the glioblastoma comprises glioblastoma multiforme.

In another preferred embodiment, the brain tumor comprises brain medulloblastoma.

In another preferred embodiment, the renal carcinoma is selected from the group consisting of clear cell renal cell adenocarcinoma, metastatic renal carcinoma, and combination thereof.

In another preferred embodiment, the renal carcinoma cell comprises Wilms cells.

In another preferred embodiment, the leukemia is selected from the group consisting of T-lymphocyte leukemia, myeloid leukemia, and combinations thereof.

In another preferred embodiment, the T-lymphocytic leukemia comprises acute T-lymphocytic leukemia.

In another preferred embodiment, the myeloid leukemia comprises acute myeloid leukemia.

In another preferred embodiment, the myeloid leukemia comprises M4 type acute myeloid leukemia.

In another preferred embodiment, the myeloid leukemia comprises FAB M4 type acute myeloid leukemia.

In another preferred embodiment, the expression comprises protein expression and/or mRNA expression.

In another preferred embodiment, the prostate cancer is selected from the group consisting of metastatic prostate cancer.

In another preferred embodiment, the metastatic prostate cancer is selected from the group consisting of brain-metastatic prostate cancer, bone-metastatic prostate cancer, and combinations thereof.

In another preferred embodiment, the breast cancer is selected from the group consisting of breast ductal carcinoma , metastatic breast cancer, and combinations thereof.

In another preferred embodiment, the breast ductal carcinoma comprises primary breast ductal carcinoma.

In another preferred embodiment, the breast ductal carcinoma comprises primary breast ductal carcinoma of grade 3.

In another preferred embodiment, the pancreatic cancer comprises liver-metastatic pancreatic cancer.

In another preferred embodiment, the mitochondrial oxidative phosphorylation pathway inhibitor comprises a compound of formula I, or an optical isomer thereof, or a racemate thereof, or a solvate thereof, or a pharmaceutically acceptable salt thereof; 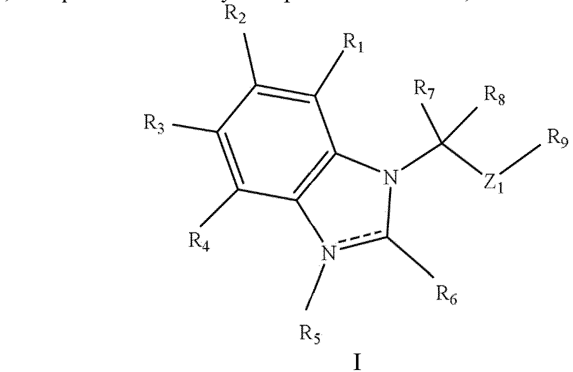 wherein,
R₁, R₂, R₃, R₄, R₆, R₇, R₈ and R₉ are each independently hydrogen, halogen, hydroxyl, sulfhydryl, amino, substituted or unsubstituted C₁-C₁₂ alkyl, substituted or unsubstituted C3-C12 cycloalkyl, substituted or unsubstituted 3-12 membered heterocycloalkyl, substituted or unsubstituted C1-C12 alkoxyl, substituted or unsubstituted C1-C12 alkylthio, substituted or unsubstituted C6-C12 aryl, substituted or unsubstituted 5-12 membered heteroaryl;
R₅ is none, hydrogen, halogen, hydroxyl, sulfhydryl, amino, substituted or unsubstituted C1-12 alkyl, substituted or unsubstituted C3-C12 cycloalkyl, substituted or unsubstituted 3-12 membered heterocycloalkyl, substituted or unsubstituted C1-C12 alkoxyl, substituted or unsubstituted C1-C12 alkylthio, substituted or unsubstituted C6-C12 aryl, substituted or unsubstituted 5-12 membered heteroaryl;
Z₁ is 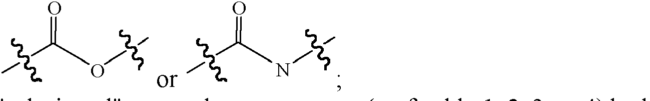
each "substituted" means that one or more (preterably 1, 2, 3, or 4) hydrogen atoms on the group are substituted by a substituent selected from the group consisting of C1-C8 alkyl, C3-C8 cycloalkyl, C1-C8 haloalkyl (e.g., trifluoromethyl), C3-C8 halocycloalkyl, halogen, nitro, -CN, hydroxyl, sulfhydryl, amino, C1-C8 alkoxyl, C1-C8 alkylthio, C3-C8 cycloalkoxyl, C3-C8 cycloalkylthio, C1-C8 haloalkoxyl, C1-C8 haloalkylthio, C6-C12 aryl, 5-10 membered heteroaryl, methylsulfonyl, sulfonyl;
the heterocyclic ring of the heterocycloalkyl and heteroaryl each independently contains 1-4 (preferably 1, 2, 3 or 4) heteroatoms selected from the group consisting of N, O and S.

In another preferred embodiment, R₅ is none, the "- - - -" is double bond.

In another preferred embodiment, R₅ is not none, the "- - - -" is single bond.

In another preferred embodiment, R₅ is not none, the "- - - -" is double bond, and the N atom connected with R5 is N⁺.

In another preferred embodiment, R₅ is none, hydrogen or C1-C3 alkyl.

In another preferred embodiment, R₁, R₂, R₃, R₄, R₅, R₆, R₇, R₈ and R₉ are each independently hydrogen, halogen, hydroxyl, sulfhydryl, amino, substituted or unsubstituted C₁-C10 alkyl, substituted or unsubstituted C3-C10 cycloalkyl, substituted or unsubstituted 3-10 membered heterocycloalkyl, substituted or unsubstituted C1-C10 alkoxyl, substituted or unsubstituted C1-C10 alkylthio, substituted or unsubstituted C6-C10 aryl, substituted or unsubstituted 5-10 membered heteroaryl.

In another preferred embodiment, R₁, R₂, R₃, R₄, R₅, R₆, R₇, R₈ and R₉ are each independently hydrogen, halogen, hydroxyl, sulfhydryl, amino, substituted or unsubstituted C1-C8 alkyl, substituted or unsubstituted C3-C8 cycloalkyl, substituted or unsubstituted 3-8 membered heterocycloalkyl, substituted or unsubstituted C1-C8 alkoxyl, substituted or unsubstituted C1-C8 alkylthio, substituted or unsubstituted C6-C8 aryl, substituted or unsubstituted 5-8 membered heteroaryl.

In another preferred embodiment, R₁, R₂, R₃, R₄, R₅, R₆, R₇, R₈ and R₉ are each independently hydrogen, halogen, hydroxyl, sulfhydryl, amino, substituted or unsubstituted C1-C6 alkyl, substituted or unsubstituted C5-C8 cycloalkyl, substituted or unsubstituted 5-8 membered heterocycloalkyl, substituted or unsubstituted C1-C6 alkoxyl, substituted or unsubstituted C1-C6 alkylthio, substituted or unsubstituted C6-C8 aryl, substituted or unsubstituted 5-8 membered heteroaryl.

In another preferred embodiment, R₁, R₂, R₃, R₄, R₅, R₆, R₇, R₈ and R₉ are each independently hydrogen, halogen, hydroxyl, sulfhydryl, amino, substituted or unsubstituted C1-C4 alkyl, substituted or unsubstituted C3-C8 cycloalkyl, substituted or unsubstituted 3-8 membered heterocycloalkyl, substituted or unsubstituted C1-C4 alkoxyl, substituted or unsubstituted C1-C4 alkylthio, substituted or unsubstituted C6-C8 aryl, substituted or unsubstituted 5-8 membered heteroaryl.

In another preferred embodiment, R₁, R₂, R₃, R₄, R₅, R₆, R₇, R₈ and R₉ are each independently hydrogen, halogen, hydroxyl, sulfhydryl, amino, substituted or unsubstituted C1-C4 alkyl, substituted or unsubstituted C5-C8 cycloalkyl, substituted or unsubstituted 5-8 membered heterocycloalkyl, substituted or unsubstituted C1-C4 alkoxyl, substituted or unsubstituted C1-C4 alkylthio, substituted or unsubstituted C6-C8 aryl, substituted or unsubstituted 5-8 membered heteroaryl.

In another preferred embodiment, R₁, R₂, R₃, R₄, R₅, R₆, R₇, R₈ and R₉ are each independently hydrogen, halogen, hydroxyl, sulfhydryl, amino, substituted or unsubstituted C1-C4 alkyl, substituted or unsubstituted C5-C8 cycloalkyl, substituted or unsubstituted 5-8 membered heterocycloalkyl, substituted or unsubstituted C1-C4 alkoxyl, substituted or unsubstituted C1-C4 alkylthio, substituted or unsubstituted C6 aryl, substituted or unsubstituted C7 aryl, substituted or unsubstituted C8 aryl, substituted or unsubstituted 5-8 membered ( e.g., 5, 6, 7 or 8 membered ) heteroaryl.

In another preferred embodiment, R₁, R₂, R₃, R₄, R₇ and R₈ are each independently hydrogen. In another preferred embodiment, R₅ is hydrogen, methyl, ethyl, propyl or butyl.

In another preferred embodiment, R₆ is hydrogen, methyl, ethyl, propyl, butyl, phenyl, trifluoromethyl-phenyl-.

In another preferred embodiment, the trifluoromethyl-phenyl- is mono-substituted trifluoromethyl-phenyl-.

In another preferred embodiment, in the trifluoromethyl-phenyl-, the ortho, meta or para position of phenyl is substituted by trifluoromethyl.

In another preferred embodiment, the trifluoromethyl-phenyl- is

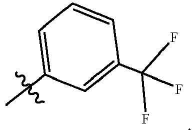

In another preferred embodiment, R₆ is hydrogen, methyl, ethyl, propyl, butyl, unsubstituted phenyl or substituted phenyl.

In another preferred embodiment, the substituted phenyl means one or more (preferably 2, 3, or 4) hydrogen atoms on the phenyl are substituted by trifluoromethyl.

In another preferred embodiment, the substituted phenyl means that one hydrogen atom on the phenyl is substituted by trifluoromethyl.

In another preferred embodiment, the substituted phenyl means one hydrogen atom on the ortho, meta or para position of phenyl is substituted by trifluoromethyl.

In another preferred embodiment, R₆ is hydrogen, methyl, ethyl, propyl, butyl or

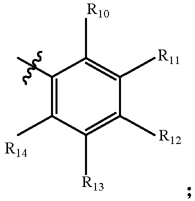

R₁₀, R₁₁, R₁₂, R₁₃ and R₁₄ are each independently hydrogen, C1-C8 alkyl, C3-C8 cycloalkyl, C1-C8 haloalkyl (e.g., trifluoromethyl), C3-C8 halocycloalkyl, halogen, nitro, -CN, hydroxyl, sulfhydryl, amino, C1-C8 alkoxyl, C1-C8 alkylthio, C3-C8 cycloalkoxyl, C3-C8 cycloalkylthio, C1-C8 haloalkoxyl, C1-C8 haloalkylthio, C6-C12 aryl, 5-10 membered heteroaryl.

In another preferred embodiment, R₁₀, R₁₁, R₁₂, R₁₃ and R₁₄ are each independently hydrogen, C1-C6 alkyl, C3-C8 cycloalkyl, C1-C6 haloalkyl (e.g., trifluoromethyl), C3-C8 halocycloalkyl, halogen, nitro, -CN, hydroxyl, sulfhydryl, amino, C1-C6 alkoxyl, C1-C6 alkylthio, C3-C8 cycloalkoxyl, C3-C8 cycloalkylthio, C1-C6 haloalkoxyl, C1-C6 haloalkylthio, C6-C10 aryl, 5-8 membered heteroaryl.

In another preferred embodiment, R₁₀, R₁₁, R₁₂, R₁₃ and R₁₄ are each independently hydrogen, C1-C4 alkyl, C3-C8 cycloalkyl, C1-C4 haloalkyl (e.g., trifluoromethyl), C3-C8 halocycloalkyl, halogen, nitro, -CN, hydroxyl, sulfhydryl, amino, C1-C4 alkoxyl, C1-C6 alkylthio, C3-C8 cycloalkoxyl, C3-C8 cycloalkylthio, C1-C4 haloalkoxyl, C1-C4 haloalkylthio, C6-C10 aryl, 5-8 membered heteroaryl.

In another preferred embodiment, R₁₀, R₁₁, R₁₂, R₁₃ and R₁₄ are each independently hydrogen, C1-C4 haloalkyl (e.g., trifluoromethyl).

In another preferred embodiment, R₁₀, R₁₁, R₁₂, R₁₃ and R₁₄ are each independently hydrogen, trifluoromethyl.

In another preferred embodiment, R₁₀, R₁₁, R₁₂ and R₁₄ are each independently hydrogen.

In another preferred embodiment R₁₃ is trifluoromethyl.

In another preferred embodiment, Z₁ is

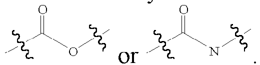

In another preferred embodiment, Z₁ is

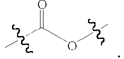

In another preferred embodiment, Z₉ is substituted or unsubstituted cyclohexyl.

In another preferred embodiment, the substituted cyclohexyl means one or more (preferably 2, 3, or 4) hydrogen atoms on the cyclohexyl are each independently substituted by C1-C4 alkyl.

In another preferred embodiment, the substituted cyclohexyl means one or more (preferably 2, 3, or 4) hydrogen atoms on the cyclohexyl are each independently substituted by methyl, ethyl, propyl, butyl.

In another preferred embodiment, the substituted cyclohexyl means the hydrogen at positions 1 and 4 on the cyclohexyl are substituted by C1-C4 alkyl.

In another preferred embodiment, the substituted cyclohexyl means the hydrogen at positions 1 and 4 on the cyclohexyl are each independently substituted by methyl, ethyl, propyl, butyl.

In another preferred embodiment, R₉ is 1-propyl-4-methyl-cyclohexyl -.

In another preferred embodiment, R₉ is 1-isopropyl-4-methyl-cyclohexyl -.

In another preferred embodiment, R₉ is

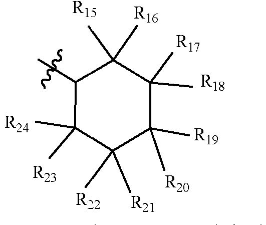

R₁₅, R₁₆, R₁₇, R₁₈, R₁₉, R₂₀, R₂₁, R₂₂, R₂₃ and R₂₄ are each independently hydrogen, C1-C8 alkyl, C3-C8 cycloalkyl, C1-C8 haloalkyl (e.g., trifluoromethyl), C3-C8 halocycloalkyl, halogen, nitro, - CN, hydroxyl, sulfhydryl, amino, C1-C8 alkoxyl, C1-C8 alkylthio, C3-C8 cycloalkoxyl, C3-C8 cycloalkylthio, C1-C8 haloalkoxyl, C1-C8 haloalkylthio, C6-C12 aryl, 5-10 membered heteroaryl.

In another preferred embodiment, R₁₅, R₁₆, R₁₇, R₁₈, R₁₉, R₂₀, R₂₁, R₂₂, R₂₃ and R₂₄ are each independently hydrogen, C1-C6 alkyl, C3-C8 cycloalkyl, C1-C6 haloalkyl (e.g., trifluoromethyl), C3-C8 halocycloalkyl, halogen, nitro, -CN, hydroxyl, sulfhydryl, amino, C1-C6 alkoxyl, C1-C6 alkylthio, C3-C8 cycloalkoxyl, C3-C8 cycloalkylthio, C1-C6 haloalkoxyl, C1-C6 haloalkylthio, C6-C10 aryl, 5-10 membered heteroaryl.

In another preferred embodiment, R₁₅, R₁₆, R₁₇, R₁₈, R₁₉, R₂₀, R₂₁, R₂₂, R₂₃ and R₂₄ are each independently hydrogen, C1-C4 alkyl, C3-C8 cycloalkyl, C1-C4 haloalkyl (e.g., trifluoromethyl), C3-C8 halocycloalkyl, halogen, nitro, -CN, hydroxyl, sulfhydryl, amino, C1-C4 alkoxyl, C1-C4 alkylthio, C3-C8 cycloalkoxyl, C3-C8 cycloalkylthio, C1-C4 haloalkoxyl, C1-C4 haloalkylthio, C6-C10 aryl, 5-10 membered heteroaryl.

In another preferred embodiment, R₁₅, R₁₆, R₁₇, R₁₈, R₁₉, R₂₀, R₂₁, R₂₂, R₂₃ and R₂₄ are each independently hydrogen, methyl, ethyl, propyl, butyl.

In another preferred embodiment, the propyl is isopropyl.

In another preferred embodiment, R₉ is 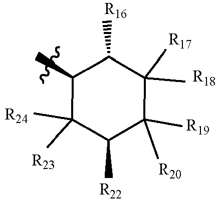 wherein, R₁₆, R₁₇, R₁₈, R₁₉, R₂₀, R₂₂, R₂₃ and R₂₄ are as defined above.

In another preferred embodiment, R₉ is

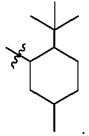

In another preferred embodiment, R₉ is

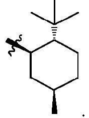

In another preferred embodiment, the heterocyclic ring of the heterocycloalkyl and heteroaryl each independently contains 1-4 (preferably 1, 2, 3 or 4) heteroatoms selected from the group consisting of N, O and S.

In another preferred embodiment, when R₅ is none, the compound of formula I has the following structure of formula I-1: 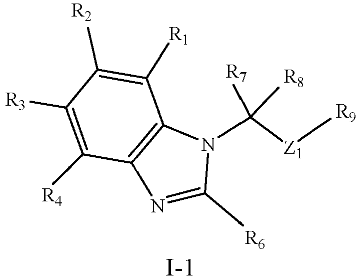 wherein, R₁, R₂, R₃, R₄, R₆, R₇, R₈, R₉ and Z1 are as defined above.

In another preferred embodiment, when R₅ is not none, the compound of formula I has the following structure of formula I-2: 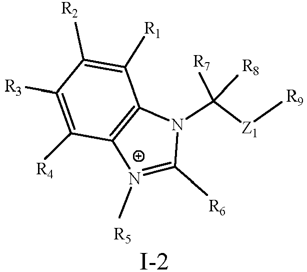 wherein, R₁, R₂, R₃, R₄, R₅, R₆, R₇, R₈, R₉ and Z1 are as defined above.

In another preferred embodiment, the compound of formula I has the following structure of formula I-3: 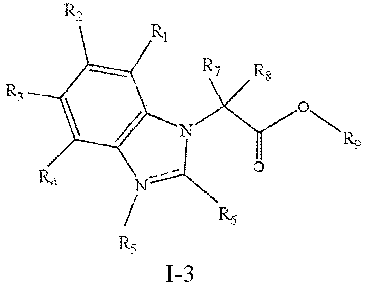 wherein, R₁, R₂, R₃, R₄, R₅, R₆, R₇, R₈, R₉ and "- - - -" are as defined above.

In another preferred embodiment, the mitochondrial oxidative phosphorylation pathway inhibitor comprises a compound of formula II, or an optical isomer thereof, or a racemate thereof, or a solvate thereof, or a pharmaceutically acceptable salt thereof; 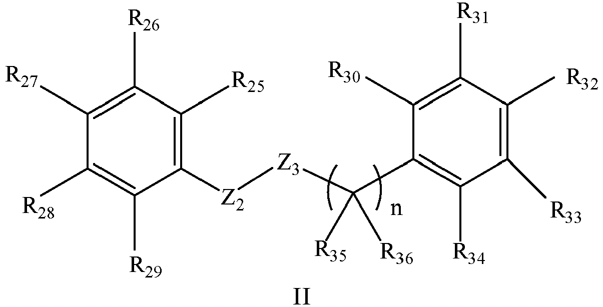
R₂₅, R₂₆, R₂₇, R₂₈, R₂₉, R₃₀, R₃₁, R₃₂, R₃₃, R₃₄, R₃₅ and R₃₆ are each independently hydrogen, halogen, hydroxyl, sulfhydryl, amino, substituted or unsubstituted C1-C12 alkyl, substituted or unsubstituted C3-C12 cycloalkyl, substituted or unsubstituted 3-12 membered heterocycloalkyl, substituted or unsubstituted C1-C12 alkoxyl, substituted or unsubstituted C1-C12 alkylthio, substituted or unsubstituted C1-C12 haloalkoxyl, substituted or unsubstituted C1-C12 haloalkylthio, substituted or unsubstituted C6-C12 aryl, substituted or unsubstituted 5-12 membered heteroaryl;
Z₂ and Z₃ are each independently substituted or unsubstituted C6-C12 arylene, substituted or unsubstituted 3-12 membered heteroarylene;
n is 0, 1, 2, 3, 4, 5, 6, 7, 8, 9, 10, 11 or 12;
each "substituted" means that one or more (preferably 1, 2, 3, or 4) hydrogen atoms on the group are substituted by a substituent selected from the group consisting of C1-C8 alkyl, C3-C8 cycloalkyl, C1-C8 haloalkyl (e.g., trifluoromethyl), C3-C8 halocycloalkyl, halogen, nitro, -CN, hydroxyl, sulfhydryl, amino, C1-C8 alkoxyl, C1-C8 alkylthio, C3-C8 cycloalkoxyl, C3-C8 cycloalkylthio, C1-C8 haloalkoxyl, C1-C8 haloalkylthio, C6-C12 aryl, 5-10 membered heteroaryl, methylsulfonyl, sulfonyl;
the heterocyclic ring of the heterocycloalkyl, heteroaryl, arylene and heteroarylene independently contains 1-4 (preferably 1, 2, 3 or 4) heteroatoms selected from the group consisting of N, O and S.

In another preferred embodiment, R₂₅, R₂₆, R₂₇, R₂₈, R₂₉, R₃₀, R₃₁, R₃₂, R₃₃, R₃₄, R₃₅ and R₃₆ are each independently hydrogen, halogen, hydroxyl, sulfhydryl, amino, substituted or unsubstituted C1-C10 alkyl, substituted or unsubstituted C3-C10 cycloalkyl, substituted or unsubstituted 3-10 membered heterocycloalkyl, substituted or unsubstituted C1-C10 alkoxyl, substituted or unsubstituted C1-C10 alkylthio, substituted or unsubstituted C1-C10 haloalkoxyl, substituted or unsubstituted C1-C10 haloalkylthio, substituted or unsubstituted C6-C10 aryl, substituted or unsubstituted 5-10 membered heteroaryl.

In another preferred embodiment, R₂₅, R₂₆, R₂₇, R₂₈, R₂₉, R₃₀, R₃₁, R₃₂, R₃₃, R₃₄, R₃₅ and R₃₆ are each independently hydrogen, halogen, hydroxyl, sulfhydryl, amino, substituted or unsubstituted C1-C8 alkyl, substituted or unsubstituted C3-C10 cycloalkyl, substituted or unsubstituted 3-10 membered heterocycloalkyl, substituted or unsubstituted C1-C8 alkoxyl, substituted or unsubstituted C1-C8 alkylthio, substituted or unsubstituted C1-C8 haloalkoxyl, substituted or unsubstituted C1-C8 haloalkylthio, substituted or unsubstituted C6-C10 aryl, substituted or unsubstituted 5-10 membered heteroaryl.

In another preferred embodiment, R₂₅, R₂₆, R₂₇, R₂₈, R₂₉, R₃₀, R₃₁, R₃₂, R₃₃, R₃₄, R₃₅ and R₃₆ are each independently hydrogen, halogen, hydroxyl, sulfhydryl, amino, substituted or unsubstituted C1-C6 alkyl, substituted or unsubstituted C3-C10 cycloalkyl, substituted or unsubstituted 3-10 membered heterocycloalkyl, substituted or unsubstituted C1-C6 alkoxyl, substituted or unsubstituted C1-C6 alkylthio, substituted or unsubstituted C1-C6 haloalkoxyl, substituted or unsubstituted C1-C6 haloalkylthio, substituted or unsubstituted C6-C10 aryl, substituted or unsubstituted 5-10 membered heteroaryl.

In another preferred embodiment, R₂₅, R₂₆, R₂₇, R₂₈, R₂₉, R₃₀, R₃₁, R₃₂, R₃₃, R₃₄, R₃₅ and R₃₆ are each independently hydrogen, halogen, hydroxyl, sulfhydryl, amino, substituted or unsubstituted C1-C4 alkyl, substituted or unsubstituted C3-C8 cycloalkyl, substituted or unsubstituted 3-8 membered heterocycloalkyl, substituted or unsubstituted C1-C4 alkoxyl, substituted or unsubstituted C1-C4 alkylthio, substituted or unsubstituted C1-C4 haloalkoxyl, substituted or unsubstituted C1-C4 haloalkylthio, substituted or unsubstituted C6-C8 aryl, substituted or unsubstituted 5-8 membered heteroaryl.

In another preferred embodiment, R₂₅, R₂₆, R₂₈, R₂₉, R₃₀, R₃₁, R₃₂, R₃₄, R₃₅ and R₃₆ are each independently hydrogen.

In another preferred embodiment, R₂₇ is substituted or unsubstituted C1-C4 haloalkoxyl, substituted or unsubstituted C1-C4 haloalkylthio.

In another preferred embodiment, R₂₇ is substituted or unsubstituted C1-C3 haloalkoxyl, substituted or unsubstituted C1-C3 haloalkylthio.

In another preferred embodiment, R₂₇ is substituted or unsubstituted C1-C2 haloalkoxyl, substituted or unsubstituted C1-C2 haloalkylthio.

In another preferred embodiment, R₂₇ is trifluoromethyl-O-, trifluoromethyl-S-.

In another preferred embodiment, R₃₃ is substituted or unsubstituted 3-10-membered (e.g., 5, 6, 7, 8, 9, 10 membered) heterocycloalkyl.

In another preferred embodiment, the heterocycloalkyl is fully saturated heterocycloalkyl.

In another preferred embodiment, R₃₃ is substituted or unsubstituted hexahydropyridyl.

In another preferred embodiment, R₃₃ is substituted or unsubstituted hexahydropyridyl, the "substituted" means that one or more (preferably 2, 3, 4, 5 or 6) hydrogen atoms on the hexahydropyridyl are each independently substituted by a substituent selected from the group consisting of methylsulfonyl, sulfonyl.

In another preferred embodiment, R₃₃ is

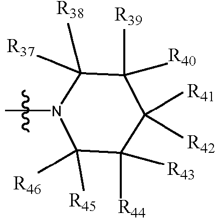

R₃₇, R₃₈, R₃₉, R₄₀, R₄₁, R₄₂, R₄₃, R₄₄, R₄₅ and R₄₆ are each independently hydrogen, C1-C4 alkyl, C3-C6 cycloalkyl, methylsulfonyl, sulfonyl.

In another preferred embodiment, R₃₇, R₃₈, R₃₉, R₄₀, R₄₁, R₄₃, R₄₄, R₄₅ and R₄₆ are each independently hydrogen.

In another preferred embodiment, R₄₂ is methylsulfonyl, sulfonyl.

In another preferred embodiment, n is 0, 1, 2, 3, 4, 5, 6, 7 or 8.

In another preferred embodiment, n is 1.

In another preferred embodiment, Z₂ and Z₃ are each independently substituted or unsubstituted C6-C10 arylene, substituted or unsubstituted 3-10 membered heteroarylene.

In another preferred embodiment, Z₂ and Z₃ are each independently substituted or unsubstituted C6-C8 arylene, substituted or unsubstituted 3-8 membered heteroarylene.

In another preferred embodiment, Z₂ and Z₃ are each independently substituted or unsubstituted C6-C8 arylene, substituted or unsubstituted 3-7 membered heteroarylene.

In another preferred embodiment, Z₂ and Z₃ are each independently substituted or unsubstituted C6 arylene, substituted or unsubstituted C7 arylene, substituted or unsubstituted C8 arylene, substituted or unsubstituted 3 membered heteroarylene, substituted or unsubstituted 4 membered heteroarylene, substituted or unsubstituted 5 membered heteroarylene, substituted or unsubstituted 6 membered heteroarylene, substituted or unsubstituted 7 membered heteroarylene, substituted or unsubstituted 8 membered heteroarylene, substituted or unsubstituted 9 membered heteroarylene, substituted or unsubstituted 10 membered heteroarylene.

In another preferred embodiment, Z₂ and Z₃ are each independently phenylene, substituted or unsubstituted oxadiazolylene, substituted or unsubstituted triazolylene.

In another preferred embodiment, oxadiazolylene is 1,2,4-oxadiazolylene.

In another preferred embodiment, triazolylene is 1H-1,2,4-triazolylene.

In another preferred embodiment, Z₂ and Z₃ are each independently 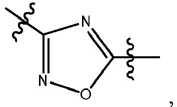 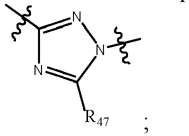 wherein, R₄₇ is hydrogen, C1-C8 alkyl, C3-C8 cycloalkyl.

In another preferred embodiment, R₄₇ is hydrogen, C1-C8 alkyl, C3-C8 cycloalkyl.

In another preferred embodiment, R₄₇ is hydrogen, C1-C6 alkyl, C3-C8 cycloalkyl.

In another preferred embodiment, R₄₇ is hydrogen, C1-C4 alkyl, C3-C8 cycloalkyl.

In another preferred embodiment, R₄₇ is hydrogen, C1-C2 alkyl, C3-C8 cycloalkyl.

In another preferred embodiment, R₄₇ is hydrogen, methyl, ethyl, propyl, or butyl.

In another preferred embodiment, Z₂ is

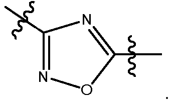

In another preferred embodiment, Z₃ is 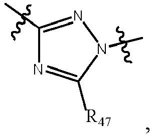 R₄₇ is as defined above.

In another preferred embodiment, the heterocyclic ring of the heterocycloalkyl, heteroaryl, arylene and heteroarylene independently contains 1-4 (preferably 1, 2, 3 or 4) heteroatoms selected from the group consisting of N, O and S.

In another preferred embodiment, the compound of formula II has the following structure of formula II-1: 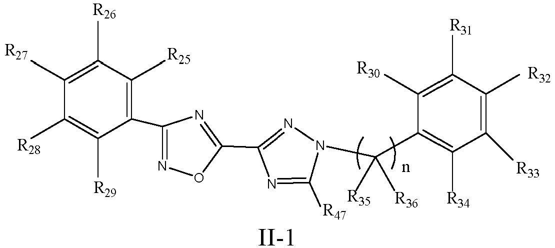 wherein, R₂₅, R₂₆, R₂₇, R₂₈, R₂₉, R₃₀, R₃₁, R₃₂, R₃₃, R₃₄, R₃₅, R₃₆, R₄₇ and n are as defined above.

In another preferred embodiment, the mitochondrial oxidative phosphorylation pathway inhibitor comprises a compound of formula III, or an optical isomer thereof, or a racemate thereof, or a solvate thereof, or a pharmaceutically acceptable salt thereof; 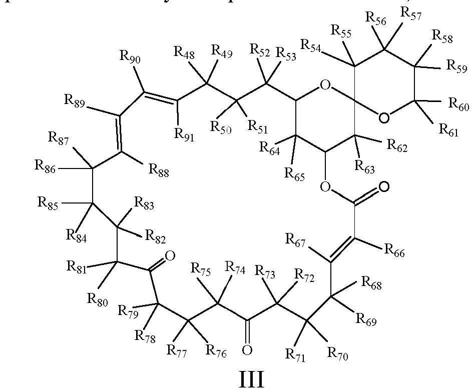
wherein, R₄₈, R₄₉, R₅₀, R₅₁, R₅₂, R₅₃, R₅₄, R₅₅, R₅₆, R₅₇, R₅₈, R₅₉, R₆₀, R₆₁, R₆₂, R₆₃, R₆₄, R₆₅, R₆₆, R₆₇, R₆₈, R₆₉, R₇₀, R₇₁, R₇₂, R₇₃, R₇₄, R₇₅, R₇₆, R₇₇, R₇₈, R₇₉, R₈₀, R₈₁, R₈₂, R₈₃, R₈₄, R₈₅, R₈₆, R₈₇, R₈₈, R₈₉, R₉₀ and R₉₁ are each independently hydrogen, halogen, hydroxyl, hydroxyl-(C1-C12 alkyl)-, sulfhydryl, amino, substituted or unsubstituted C1-C12 alkyl, substituted or unsubstituted C3-C12 cycloalkyl, substituted or unsubstituted 3-12 membered heterocycloalkyl, substituted or unsubstituted C1-C12 alkoxyl, substituted or unsubstituted C1-C12 alkylthio, substituted or unsubstituted C6-C12 aryl, substituted or unsubstituted 5-12 membered heteroaryl;
each "substituted" means that one or more (preferably 1, 2, 3, or 4) hydrogen atoms on the group are substituted by a substituent selected from the group consisting of C1-C8 alkyl, C3-C8 cycloalkyl, C1-C8 haloalkyl (e.g., trifluoromethyl), C3-C8 halocycloalkyl, halogen, nitro, -CN, hydroxyl, sulfhydryl, amino, C1-C8 alkoxyl, C1-C8 alkylthio, C3-C8 cycloalkoxyl, C3-C8 cycloalkylthio, C1-C8 haloalkoxyl, C1-C8 haloalkylthio, C6-C12 aryl, 5-10 membered heteroaryl, methylsulfonyl, sulfonyl;
the heterocyclic ring of the heterocycloalkyl and heteroaryl each independently contains 1-4 (preferably 1, 2, 3 or 4) heteroatoms selected from the group consisting of N, O and S.

In another preferred embodiment, R₄₈, R₄₉, R₅₀, R₅₁, R₅₂, R₅₃, R₅₄, R₅₅, R₅₆, R₅₇, R₅₈, R₅₉, R₆₀, R₆₁, R₆₂, R₆₃, R₆₄, R₆₅, R₆₆, R₆₇, R₆₈, R₆₉, R₇₀, R₇₁, R₇₂, R₇₃, R₇₄, R₇₅, R₇₆, R₇₇, R₇₈, R₇₉, R₈₀, R₈₁, R₈₂, R₈₃, R₈₄, R₈₅, R₈₆, R₈₇, R₈₈, R₈₉, R₉₀ and R₉₁ are each independently hydrogen, halogen, hydroxyl, hydroxyl-(C1-C10 alkyl)-, sulfhydryl, amino, substituted or unsubstituted C1-C10 alkyl, substituted or unsubstituted C3-C10 cycloalkyl, substituted or unsubstituted 3-10 membered heterocycloalkyl, substituted or unsubstituted C1-C10 alkoxyl, substituted or unsubstituted C1-C10 alkylthio, substituted or unsubstituted C6-C10 aryl, substituted or unsubstituted 5-10 membered heteroaryl.

In another preferred embodiment, R₄₈, R₄₉, R₅₀, R₅₁, R₅₂, R₅₃, R₅₄, R₅₅, R₅₆, R₅₇, R₅₈, R₅₉, R₆₀, R₆₁, R₆₂, R₆₃, R₆₄, R₆₅, R₆₆, R₆₇, R₆₈, R₆₉, R₇₀, R₇₁, R₇₂, R₇₃, R₇₄, R₇₅, R₇₆, R₇₇, R₇₈, R₇₉, R₈₀, R₈₁, R₈₂, R₈₃, R₈₄, R₈₅, R₈₆, R₈₇, R₈₈, R₈₉, R₉₀ and R₉₁ are each independently hydrogen, halogen, hydroxyl, hydroxyl-(C1-C8 alkyl)-, sulfhydryl, amino, substituted or unsubstituted C1-C8 alkyl, substituted or unsubstituted C3-C8 cycloalkyl, substituted or unsubstituted 3-8 membered heterocycloalkyl, substituted or unsubstituted C1-C8 alkoxyl, substituted or unsubstituted C1-C8 alkylthio, substituted or unsubstituted C6-C10 aryl, substituted or unsubstituted 5-10 membered heteroaryl.

In another preferred embodiment, R₄₈, R₄₉, R₅₀, R₅₁, R₅₂, R₅₃, R₅₄, R₅₅, R₅₆, R₅₇, R₅₈, R₅₉, R₆₀, R₆₁, R₆₂, R₆₃, R₆₄, R₆₅, R₆₆, R₆₇, R₆₈, R₆₉, R₇₀, R₇₁, R₇₂, R₇₃, R₇₄, R₇₅, R₇₆, R₇₇, R₇₈, R₇₉, R₈₀, R₈₁, R₈₂, R₈₃, R₈₄, R₈₅, R₈₆, R₈₇, R₈₈, R₈₉, R₉₀ and R₉₁ are each independently hydrogen, halogen, hydroxyl, hydroxyl-(C1-C6 alkyl)-, sulfhydryl, amino, substituted or unsubstituted C1-C6 alkyl, substituted or unsubstituted C1-C6 alkoxyl, substituted or unsubstituted C1-C6 alkylthio.

In another preferred embodiment, R₄₈, R₄₉, R₅₀, R₅₁, R₅₂, R₅₃, R₅₄, R₅₅, R₅₆, R₅₇, R₅₈, R₅₉, R₆₀, R₆₁, R₆₂, R₆₃, R₆₄, R₆₅, R₆₆, R₆₇, R₆₈, R₆₉, R₇₀, R₇₁, R₇₂, R₇₃, R₇₄, R₇₅, R₇₆, R₇₇, R₇₈, R₇₉, R₈₀, R₈₁, R₈₂, R₈₃, R₈₄, R₈₅, R₈₆, R₈₇, R₈₈, R₈₉, R₉₀ and R₉₁ are each independently hydrogen, hydroxyl, hydroxyl-(C1-C4 alkyl)-, substituted or unsubstituted C1-C4 alkyl, substituted or unsubstituted C1-C4 alkoxyl, substituted or unsubstituted C1-C4 alkylthio.

In another preferred embodiment, R₄₈, R₄₉, R₅₀, R₅₁, R₅₂, R₅₃, R₅₄, R₅₅, R₅₆, R₅₇, R₅₈, R₅₉, R₆₀, R₆₁, R₆₂, R₆₃, R₆₄, R₆₅, R₆₆, R₆₇, R₆₈, R₆₉, R₇₀, R₇₁, R₇₂, R₇₃, R₇₄, R₇₅, R₇₆, R₇₇, R₇₈, R₇₉, R₈₀, R₈₁, R₈₂, R₈₃, R₈₄, R₈₅, R₈₆, R₈₇, R₈₈, R₈₉, R₉₀ and R₉₁ are each independently hydrogen, methyl, ethyl, propyl, butyl, hydroxy-propyl-, sulfhydryl-propyl-, hydroxyl, sulfhydryl.

In another preferred embodiment, hydroxyl-propyl- is monohydroxyl-propyl-.

In another preferred embodiment, hydroxyl-propyl- is

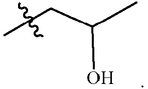

In another preferred embodiment, sulfhydryl-propyl- is monosulfhydryl-propyl-.

In another preferred embodiment, sulfhydryl-propyl- is

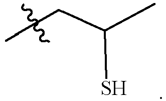

In another preferred embodiment, each "substituted" means that one or more (preferably 1, 2, 3, or 4) hydrogen atoms on the group are substituted by a substituent selected from the group consisting of C1-C6 alkyl, C3-C8 cycloalkyl, C1-C6 haloalkyl (e.g., trifluoromethyl), C3-C8 halocycloalkyl, halogen, nitro, -CN, hydroxyl, sulfhydryl, amino, C1-C6 alkoxyl, C1-C6 alkylthio, C3-C8 cycloalkoxyl, C3-C8 cycloalkylthio, C1-C6 haloalkoxyl, C1-C6 haloalkylthio, C6-C10 aryl, 5-10 membered heteroaryl, methylsulfonyl, sulfonyl.

In another preferred embodiment, each "substituted" means that one or more (preferably 1, 2, 3, or 4) hydrogen atoms on the group are substituted by a substituent selected from the group consisting of C1-C4 alkyl, C3-C8 cycloalkyl, C1-C4 haloalkyl (e.g., trifluoromethyl), C3-C8 halocycloalkyl, halogen, nitro, -CN, hydroxyl, sulfhydryl, amino, C1-C4 alkoxyl, C1-C4 alkylthio, C3-C8 cycloalkoxyl, C3-C8 cycloalkylthio, C1-C4 haloalkoxyl, C1-C4 haloalkylthio, C6-C10 aryl, 5-10 membered heteroaryl, methylsulfonyl, sulfonyl.

In another preferred embodiment, the heterocyclic ring of the heterocycloalkyl and heteroaryl each independently contains 1-4 (preferably 1, 2, 3 or 4) heteroatoms selected from the group consisting of N, O and S.

In another preferred embodiment, the mitochondrial oxidative phosphorylation pathway inhibitor is selected from the following group:

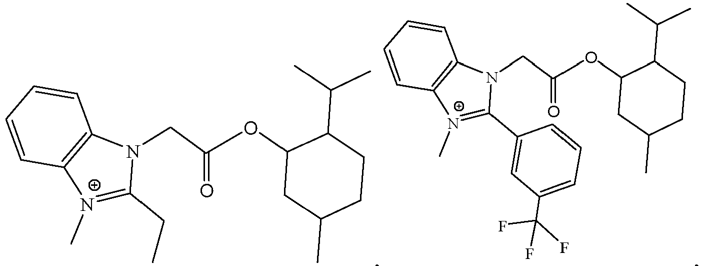

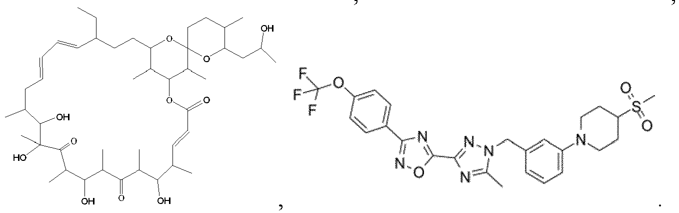

In another preferred embodiment, the mitochondrial oxidative phosphorylation pathway inhibitor is selected from the following group:

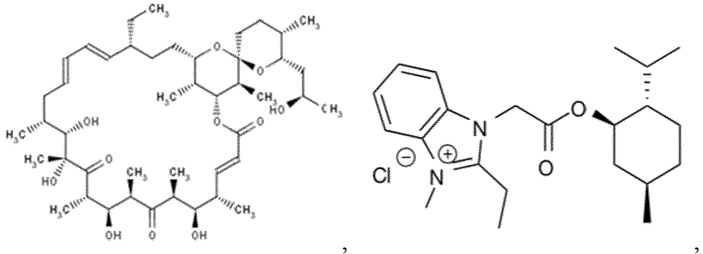

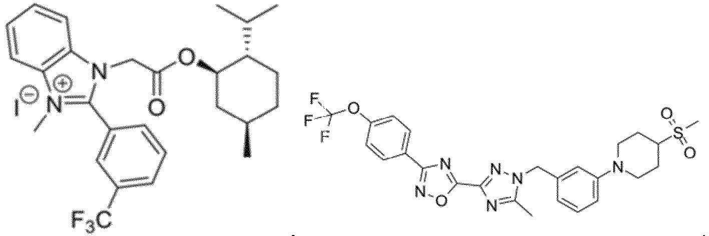

In another preferred embodiment, the composition is a pharmaceutical composition.

In another preferred embodiment, the pharmaceutical composition further comprises a pharmaceutically acceptable carrier.

In another preferred embodiment, the expression is mRNA expression or protein expression.

In another preferred embodiment, the dosage form of the composition or preparation is a solid preparation, liquid preparation or semi-solid preparation.

In another preferred embodiment, the dosage form of the composition or preparation is oral preparation, external preparation or injection preparation

In another preferred embodiment, the dosage form of the composition or preparation is tablet, injection, infusion, paste, gel, solution, microsphere or film.

In the second aspect of the present invention, it provides a marker for determining whether a mitochondrial oxidative phosphorylation pathway inhibitor is suitable for use in the prevention and/or treatment of patient tumor, the marker comprises the expression level or activity of mitochondrial oxidative phosphorylation pathway, the expression level of NNMT gene, the expression level of DNA methylase, the expression level of UHRF1, the methylation level of nucleotide site of NNMT gene, and/or the methylation level of DNA CpG site of NNMT gene.

The present invention further provides a use of the marker (or expression level, activity, or methylation level thereof) or detection reagent thereof in the preparation of a kit for determining whether a mitochondrial oxidative phosphorylation pathway inhibitor is suitable for use in the prevention and/or treatment of patient tumor.

In another preferred embodiment, the methylation level of DNA CpG site of NNMT gene comprises the methylation level of DNA CpG site in promoter region of NNMT gene.

In another preferred embodiment, the mitochondrial oxidative phosphorylation pathway inhibitor is suitable for use in the prevention and/or treatment of patient tumor with up-regulation of mitochondrial oxidative phosphorylation pathway, low or no expression of NNMT gene, high expression of DNA methylase, high expression of UHRFl, high methylation level of nucleotide site of NNMT gene, and/or high methylation level of DNA CpG site of NNMT gene.

In another preferred embodiment, the mitochondrial oxidative phosphorylation pathway inhibitor is not suitable for use in the prevention and/or treatment of patient tumor with down-regulation of mitochondrial oxidative phosphorylation pathway, high expression of NNMT gene, low expression of DNA methylase, low expression of UHRFl, low methylation level of nucleotide site of NNMT gene, and/or low methylation level of DNA CpG site of NNMT gene.

In another preferred embodiment, "the mitochondrial oxidative phosphorylation pathway inhibitor is suitable for use in the prevention and/or treatment of patient tumor" comprises "the patient tumor is sensitive to the mitochondrial oxidative phosphorylation pathway inhibitor".

In another preferred embodiment, "the mitochondrial oxidative phosphorylation pathway inhibitor is not suitable for use in the prevention and/or treatment of patient tumor" comprises "the patient tumor is not sensitive to the mitochondrial oxidative phosphorylation pathway inhibitor".

In another preferred embodiment, the DNA methylase is selected from the group consisting of DNMT1, DNMT3a, DNMT3b, and combinations thereof.

In another preferred embodiment, the tumor with up-regulation of mitochondrial oxidative phosphorylation pathway is as described in the first aspect of the invention.

In another preferred embodiment, the tumor with low or no expression of NNMT gene is as described in the first aspect of the invention.

In another preferred embodiment, the tumor with high expression of DNA methylase ( e.g., DNMT1) is as described in the first aspect of the invention.

In another preferred embodiment, the tumor with high expression of UHRFl is as described in the first aspect of the invention.

In another preferred embodiment, the tumor with high methylation level of nucleotide site of NNMT gene is as described in the first aspect of the invention.

In another preferred embodiment, the tumor with high methylation level of DNA CpG site of NNMT gene is as described in the first aspect of the invention.

In another preferred embodiment, the down-regulation of mitochondrial oxidative phosphorylation pathway means that the ratio (H1/H0) of the expression level or activity H1 of mitochondrial oxidative phosphorylation pathway in a cell ( e.g., tumor cell ) to the expression level or activity H0 of mitochondrial oxidative phosphorylation pathway in the same type of cell or a normal cell (e.g., para-tumor tissue cell) is <1.0. preferably ≤ 0.7, more preferably ≤ 0.6, more preferably ≤ 0.5, more preferably ≤ 0.4, more preferably ≤ 0.3, more preferably ≤ 0.2, more preferably ≤ 0.1, more preferably ≤ 0.05, more preferably ≤ 0.01, more preferably ≤ 0.005, more preferably ≤ 0.001, more preferably ≤ 0.0001, more preferably ≤ 0.00001 , more preferably ≤ 0.000001, more preferably ≤ 0.0000001.

In another preferred embodiment, the high expression of NNMT gene means the ratio (E1/E0) of the expression level E1 of NNMT gene in a cell ( e.g., tumor cell ) to the expression level E0 of NNMT gene in the same type of cell or a normal cell (e.g., para-tumor tissue cell) is >1.0, preferably ≥1.2, more preferably ≥1.5, more preferably ≥2, more preferably ≥3, more preferably ≥5, more preferably ≥8, more preferably ≥10, more preferably ≥15, more preferably ≥20, more preferably ≥30, more preferably ≥50.

In another preferred embodiment, the tumor with low expression of DNA methylase means the ratio (A1/A0) of the expression level A1 of DNA methylase in the tumor cell to the expression level A0 of DNA methylase in the same type of cell or a normal cell (e.g., para-tumor tissue cell) is <1.0. preferably ≤ 0.7, more preferably ≤ 0.6, more preferably ≤ 0.5, more preferably ≤ 0.4, more preferably ≤ 0.3, more preferably ≤ 0.2, more preferably ≤ 0.1, more preferably ≤ 0.05, more preferably ≤ 0.01, more preferably ≤ 0.005, more preferably ≤ 0.001, more preferably ≤ 0.0001, more preferably ≤ 0.00001 , more preferably ≤ 0.000001, more preferably ≤ 0.0000001.

In another preferred embodiment, the tumor with low expression of UHRF1 means the ratio (F1/F0) of the expression level F1 of UHRF1 in the tumor cell to the expression level F0 of UHRF1 in the same type of cell or a normal cell (e.g., para-tumor tissue cell) is <1.0. preferably ≤ 0.7, more preferably ≤ 0.6, more preferably ≤ 0.5, more preferably ≤ 0.4, more preferably ≤ 0.3, more preferably ≤ 0.2, more preferably ≤ 0.1, more preferably ≤ 0.05, more preferably ≤ 0.01, more preferably ≤ 0.005, more preferably ≤ 0.001, more preferably ≤ 0.0001, more preferably ≤ 0.00001 , more preferably ≤ 0.000001, more preferably ≤ 0.0000001.

In another preferred embodiment, the low methylation level of nucleotide site of NNMT gene means the ratio (L1/L0) of the methylation level L1 of nucleotide site of NNMT gene in a cell ( e.g., tumor cell ) to the methylation level L0 of nucleotide site of NNMT gene in the same type of cell or a normal cell (e.g., para-tumor tissue cell) is <1.0. preferably ≤ 0.7, more preferably ≤ 0.6, more preferably ≤ 0.5, more preferably ≤ 0.4, more preferably ≤ 0.3, more preferably ≤ 0.2, more preferably ≤ 0.1, more preferably ≤ 0.05, more preferably ≤ 0.01, more preferably ≤ 0.005, more preferably ≤ 0.001, more preferably ≤ 0.0001, more preferably ≤ 0.00001 , more preferably ≤ 0.000001, more preferably ≤ 0.0000001.

In another preferred embodiment, the low methylation level of DNA CpG site of NNMT gene means the ratio (W1/WO) of the methylation level W1 of DNA CpG site of NNMT gene in a cell ( e.g., tumor cell ) to the methylation level WO of DNA CpG site of NNMT gene in the same type of cell or a normal cell (e.g., para-tumor tissue cell) is <1.0. preferably ≤ 0.7, more preferably ≤ 0.6, more preferably ≤ 0.5, more preferably ≤ 0.4, more preferably ≤ 0.3, more preferably ≤ 0.2, more preferably ≤ 0.1, more preferably ≤ 0.05, more preferably ≤ 0.01, more preferably ≤ 0.005, more preferably ≤ 0.001, more preferably ≤ 0.0001, more preferably ≤ 0.00001 , more preferably ≤ 0.000001, more preferably ≤ 0.0000001.

In the third aspect of the present invention, it provides a detection kit, which comprises:
(i) a detection reagent for detecting the expression level or activity of mitochondrial oxidative phosphorylation pathway, the expression level of NNMT gene, the expression level of DNA methylase, the expression level of UHRF1, the methylation level of nucleotide site of NNMT gene, and/or the methylation level of DNA CpG site of NNMT gene.

In another preferred embodiment, the test sample of the detection kit comprises tumor cell.

In another preferred embodiment, the expression of NNMT gene is the expression of mRNA or protein.

In another preferred embodiment, the methylation level of DNA CpG site of NNMT gene is the methylation level of DNA CpG site in promoter region of NNMT gene.

In another preferred embodiment, the methylation level of DNA CpG site of NNMT gene is the methylation level of the DNA CpG sites from 1050 bp before the transcription start site to 499 bp after the transcription start site in NNMT gene.

In another preferred embodiment, the methylation level of DNA CpG site of NNMT gene is the methylation level of the DNA CpG sites from 1050 bp to 193 bp before the transcription start site in NNMT gene.

In another preferred embodiment, the methylation level of DNA CpG site of NNMT gene is the methylation level of the DNA CpG sites from 840 bp to 469 bp before the transcription start site in NNMT gene.

In the fourth aspect of the present invention, it provides a use of the detection kit according to the third aspect of the present invention in the preparation of concomitant diagnose kit for determining whether a mitochondrial oxidative phosphorylation pathway inhibitor is suitable for use in the prevention and/or treatment of patient tumor.

In another preferred embodiment, the concomitant diagnose kit further comprises instruction or label.

In another preferred embodiment, the instruction or label records that the mitochondrial oxidative phosphorylation pathway inhibitor is suitable for use in the prevention and/or treatment of patient tumor with up-regulation of mitochondrial oxidative phosphorylation pathway, low or no expression of NNMT gene, high expression of DNA methylase, high expression of UHRFl, high methylation level of nucleotide site of NNMT gene, and/or high methylation level of DNA CpG site of NNMT gene.

In another preferred embodiment, the instruction or label records that the mitochondrial oxidative phosphorylation pathway inhibitor is not suitable for use in the prevention and/or treatment of patient tumor with down-regulation of mitochondrial oxidative phosphorylation pathway, high expression of NNMT gene, low expression of DNA methylase, low expression of UHRFl, low methylation level of nucleotide site of NNMT gene, and/or low methylation level of DNA CpG site of NNMT gene.

In the fifth aspect of the present invention, it provides a medicine kit, which comprises:
(i) a detection reagent for detecting the expression level or activity of mitochondrial oxidative phosphorylation pathway, the expression level of NNMT gene, the expression level of DNA methylase, the expression level of UHRF1, the methylation level of nucleotide site of NNMT gene, and/or the methylation level of DNA CpG site of NNMT gene; and
(ii) a mitochondrial oxidative phosphorylation pathway inhibitor.

In another preferred embodiment, the medicine kit further comprises instruction or label.

In another preferred embodiment, the instruction or label records that the mitochondrial oxidative phosphorylation pathway inhibitor is suitable for use in the prevention and/or treatment of patient tumor with up-regulation of mitochondrial oxidative phosphorylation pathway, low or no expression of NNMT gene, high expression of DNA methylase, high expression of UHRFl, high methylation level of nucleotide site of NNMT gene, and/or high methylation level of DNA CpG site of NNMT gene.

In another preferred embodiment, the mitochondrial oxidative phosphorylation pathway inhibitor is not suitable for use in the prevention and/or treatment of patient tumor with down-regulation of mitochondrial oxidative phosphorylation pathway, high expression of NNMT gene, low expression of DNA methylase, low expression of UHRFl, low methylation level of nucleotide site of NNMT gene, and/or low methylation level of DNA CpG site of NNMT gene.

In another preferred embodiment, the tumor of the subject comprises tumor with low or no expression of NNMT gene.

In another preferred embodiment, the tumor of the subject comprises tumor with high methylation level of DNA CpG site of NNMT gene.

In another preferred embodiment, the subject is human and non-human mammals (rodent, rabbit, monkey, livestock, dog, cat, and the like).

In the seventh aspect of the present invention, it provides a device or system, the device or system comprises:
(i) a detection module, the detection module is used to detect the expression level or activity of mitochondrial oxidative phosphorylation pathway, the expression level of NNMT gene, the expression level of DNA methylase, the expression level of UHRF1, the methylation level of nucleotide site of NNMT gene, and/or the methylation level of DNA CpG site of NNMT gene;
(ii) a output module, the output module comprises the output of the information as follows:
   the mitochondrial oxidative phosphorylation pathway inhibitor is suitable for use in the prevention and/or treatment of patient tumor with up-regulation of mitochondrial oxidative phosphorylation pathway, low or no expression of NNMT gene, high expression of DNA methylase, high expression of UHRFl, high methylation level of nucleotide site of NNMT gene, and/or high methylation level of DNA CpG site of NNMT gene; and/or
   the mitochondrial oxidative phosphorylation pathway inhibitor is not suitable for use in the prevention and/or treatment of patient tumor with down-regulation of mitochondrial oxidative phosphorylation pathway, high expression of NNMT gene, low expression of DNA methylase, low expression of UHRFl, low methylation level of nucleotide site of NNMT gene, and/or low methylation level of DNA CpG site of NNMT gene.

In another preferred embodiment, the device comprises a gene detector or protein detector.

In another preferred embodiment, the device or system further comprises sample injection module.

In another preferred embodiment, the injection module is used to inject tumor cell extract.

In another preferred embodiment, the device or system further comprises data processing module.

In another preferred embodiment, the expression level or activity of mitochondrial oxidative phosphorylation pathway, the expression level of NNMT gene, the expression level of DNA methylase, the expression level of UHRF1, the methylation level of nucleotide site of NNMT gene, and/or the methylation level of DNA CpG site of NNMT gene can be obtained by the procession of the data processing module.

In another preferred embodiment, the expression level of NNMT gene and/or the methylation level of DNA CpG site in promoter region of NNMT gene can be obtained by the procession of the data processing module.

In another preferred embodiment, the expression level of NNMT gene and/or the methylation level of DNA CpG sites from 1050 bp before the transcription start site to 499 bp after the transcription start site in NNMT gene can be obtained by the procession of the data processing module.

In another preferred embodiment, the expression level of NNMT gene and/or the methylation level of DNA CpG sites from 1050 bp to 193 bp before the transcription start site in NNMT gene can be obtained by the procession of the data processing module.

In another preferred embodiment, the expression level of NNMT gene and/or the methylation level of DNA CpG sites from 840 bp to 469 bp before the transcription start site in NNMT gene can be obtained by the procession of the data processing module.

It should be understood that, in the present invention, each of the technical features specifically described above and below (such as those in the Examples) can be combined with each other, thereby constituting new or preferred technical solutions which need not be redundantly described one-by-one.

### DESCRIPTION OF THE DRAWINGS

Fig.1 shows the inhibition of different compounds on mitochondrial oxidative phosphorylation pathway (n=3).
Fig.2 shows the expression level of ATF4 and p-s6 protein in tumor cells NCI-H82, G-401 and WSU-DLCL2 treated with mitochondrial oxidative phosphorylation pathway inhibitor Gboxin and Oligomycin A.
Fig.3 shows the expression of ATF4 and p-s6 protein in tumor cells SF126, CFPAC-1 and 786-O treated with mitochondrial oxidative phosphorylation pathway inhibitor Gboxin and Oligomycin A.
Fig.4 shows the degree of difference among cells as indicated by the gene expression of the cells.
Fig.5 shows the functional difference in tumor cells sensitive and insensitive to mitochondrial oxidative phosphorylation pathway inhibitors.
Fig.6 shows the difference of metabolic pathways in tumor cells sensitive and insensitive to mitochondrial oxidative phosphorylation pathway inhibitors.
Fig.7 shows the protein complex of oxidative phosphorylation pathway involved in the expression.
Fig.8 shows the membrane potential difference of mitochondria in cell lines (NCI-H82, G-401 and WSU-DLCL2) sensitive to mitochondrial oxidative phosphorylation pathway inhibitors and cell lines (786-O, CFPAC-1 and SF126) insensitive to mitochondrial oxidative phosphorylation pathway inhibitors.
Fig.9 shows the oxygen consumption rate (OCR) of mitochondria in different tumor cells.
Fig.10 shows the genes with significant differences in expression screened from different cells.
Fig.11 shows the correlation between the mean transcription level of NNMT gene in tumor cells and the sensitivity of the tumors to the mitochondrial oxidative phosphorylation pathway inhibition.
Fig.12 shows the mRNA and protein expression of NNMT gene in different tumor cells, the above Fig. shows the mRNA expression of NNMT gene, and the below Fig shows the protein expression of NNMT gene.
Fig. 13 shows the analysis between the expression of NNMT gene and methylation of promoter region of NNMT gene in different tumor cells.
Fig.14 shows the methylation level of DNA CpG site of the promoter region of NNMT gene in tumor cells sensitive and insensitive to mitochondrial oxidative phosphorylation pathway inhibitors.
Fig.15 shows the methylation level of DNA CpG sites from 1050 bp before the transcription start site to 499 bp after the transcription start site in NNMT gene in tumor cells sensitive and insensitive to mitochondrial oxidative phosphorylation pathway inhibitors.
Fig. 16 shows the methylation level of DNA CpG sites from 1050 bp to 193 bp before the transcription start site in NNMT gene in tumor cells sensitive and insensitive to mitochondrial oxidative phosphorylation pathway inhibitors.
Fig. 17 shows the methylation level of specific DNA CpG sites of NNMT gene, ie, site 114165695, site 114165730, site 114165769, site 114165804, site 114165938, site 114166050, site 114166066 on human chromosome 11, in tumor cells sensitive and insensitive to mitochondrial oxidative phosphorylation pathway inhibitors, black dot indicates that the relevant site is methylated, white dot indicates that the relevant site is not methylated, SST refers to the transcription starting site, and Chr11 refers to human chromosome 11 according to human genome version GCF_ 00000 1405.25 (GRCh37. p13).
Fig.18 shows the level of S-adenosylmethionine (SAM) in tumor cells sensitive and insensitive to mitochondrial oxidative phosphorylation pathway inhibitors.
Fig.19 shows the correlation between the expression of NNMT and the expression of DNMT1, UHRF1, DNMT3a and DNMT3b in tumor cells.
Fig.20 shows the correlation between the transcription level of DNMT1 gene and the sensitivity of the tumors to the mitochondrial oxidative phosphorylation pathway inhibition.

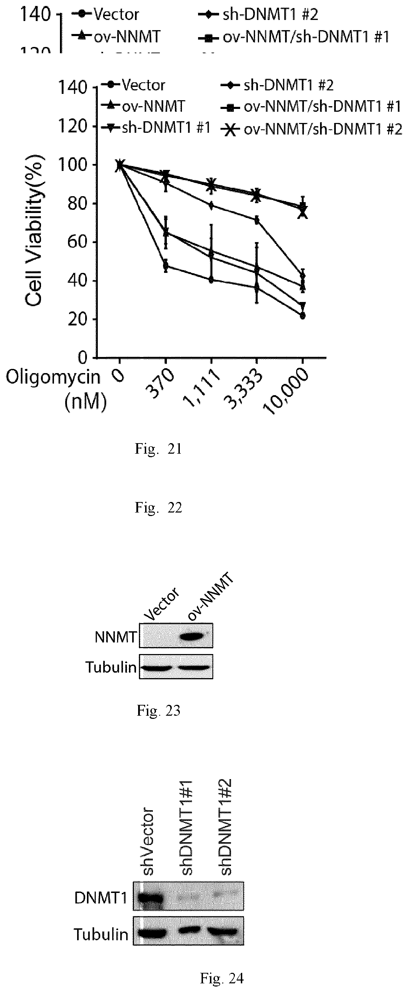

Fig.21 shows the sensitivity of tumor cells to Gboxin after overexpressing NNMT protein of NCI-H82 cell using transgenic method and/or knocking down DNMT1 expression of NCI-H82 cell using shRNA transfection method, wherein, "Vector" refers to NCI-H82 cell with normal expression of NNMT protein and DNMT1; "ov-NNMT" refers to NCI-H82 cell with overexpression of NNMT protein using transgenic method; "sh-DNMT1#1" refers to NCI-H82 cell with knockdown of DNMT1 expression using sh-DNMT1#1 transfection method; "sh-DNMT1#2" refers to NCI-82 cell with knockdown of DNMT1 expression using sh-DNMT1#2 transfection method; "ov-NNMT/sh-DNMT1#1" refers to NCI-H82 cell with overexpression of NNMT protein using transgenic method and knockdown of DNMT1 expression using sh-DNMT1#1 transfection method; "ov-NNMT/sh-DNMT1 #2" refers to NCI-H82 cell with overexpression of NNMT protein using transgenic method and knockdown of DNMT1 expression using sh-DNMT1#2 transfection method.
Fig.22 shows the sensitivity of tumor cells to Oligomycin A after overexpressing NNMT protein of NCI-H82 cell using transgenic method and/or knocking down DNMT1 expression of NCI-H82 cell using shRNA transfection method, wherein, "Vector" refers to NCI-H82 cell with normal expression of NNMT protein and DNMT1; "ov-NNMT" refers to NCI-H82 cell with overexpression of NNMT protein using transgenic method; "sh-DNMT1 # 1" refers to NCI-H82 cell with knockdown of DNMT1 expression using sh-DNMT1 # 1 transfection method; "sh-DNMT1#2" refers to NCI-H82 cell with knockdown of DNMT1 expression using sh-DNMT1#2 transfection method; "ov-NNMT/sh-DNMT1 #1" refers to NCI-H82 cell with overexpression of NNMT protein using transgenic method and knockdown of DNMT1 expression using sh-DNMT1#1 transfection method; "ov-NNMT/sh-DNMT1 #2" refers to NCI-H82 cell with overexpression of NNMT protein using transgenic method and knockdown of DNMT1 expression using sh-DNMT1#2 transfection method
Fig.23 shows the NNMT protein content in NCI-H82 (ov-NNMT) overexpressing NNMT protein using Western Blot test compared with normal NCI-H82 (Vector), wherein, "Vector" refers to NCI-H82 cell with normal expression of NNMT protein and DNMT1; "ov-NNMT" refers to NCI-H82 cell with overexpression of NNMT protein using transgenic method.
Fig.24 shows the DNMT1 protein content in NCI-H82 (sh-DNMT1 # 1 or sh-DNMT1 # 2) with knockdown of DNMT1 expression using sh-DNMT1 # 1 or sh-DNMT1 # 2 transfection method via Western Blot test compared with normal NCI-H82 (shVector), wherein, "shVector" refers to NCI-H82 cell with normal expression of DNMT1 protein; "sh-DNMT1 # 1" refers to NCI-H82 cell with knockdown of DNMT1 expression using sh-DNMT1 # 1 transfection method; "sh-DNMT1 # 2" refers to NCI-H82 cell with knockdown of DNMT1 expression using sh-DNMT1 # 2 transfection method.
Fig.25 shows the inhibitory effect of S-Gboxin, an oxidative phosphorylation pathway inhibitor, on NCI-H82 tumor-bearing mice, wherein NCI-H82 refers to NCI-H82 tumor with normal expression of NNMT protein.
Fig.26 shows the inhibitory effect of S-Gboxin, an oxidative phosphorylation pathway inhibitor, on NCI-H82-NNMT^{ov} tumor-bearing mice. NCI-H82-NNMT^{ov} refers to NCI-H82 tumor with overexpression of NNMT protein using transgene method.
Fig.27 shows the inhibitory effect of S-Gboxin, oxidative phosphorylation pathway inhibitor, on CFPAC-1 tumor-bearing mice.

### EMBODIMENTS FOR CARRYING OUT THE INVENTION

Based on an extensive and intensive research, the inventors have unexpectedly found the mitochondrial oxidative phosphorylation pathway inhibitor has significant inhibitory effect on tumors with up-regulation of mitochondrial oxidative phosphorylation pathway, low or no expression of NNMT gene, high expression of DNA methylase, high expression of UHRFl, high methylation level of nucleotide site of NNMT gene, and/or high methylation level of DNA CpG site of NNMT gene. The expression level or activity of mitochondrial oxidative phosphorylation pathway, the expression level of NNMT gene, the expression level of DNA methylase, the expression level of UHRF1, the methylation level of nucleotide site of NNMT gene, and/or the methylation level of DNA CpG site in promoter region of NNMT gene can be used as a marker for determining whether the mitochondrial oxidative phosphorylation pathway inhibitor is suitable for use in the prevention and/or treatment of patient tumor. On this basis, the inventors has completed the present invention.

### Terms

As used herein, the term "comprise", " comprising", and "containing" are used interchangeably, which not only comprise closed definitions, but also semi-closed and open definitions. In other words, the term comprises "consisting of" and "essentially consisting of".

As used herein, the term "high methylation level of DNA CpG site" and "high level of DNA CpG site methylation" are used interchangeably.

As used herein, the term "low methylation level of DNA CpG site" and "low level of DNA CpG site methylation" are used interchangeably.

As used herein, the term "IC50" and "IC₅₀" are used interchangeably, and refers to 50% inhibiting concentration, ie, the concentration of the inhibitor when 50% inhibitory effect is achieved.

As used herein, the term "methylation of CpG site ", "methylation of CpG nucleotide " and "CpG methylation" are used interchangeably.

As used herein, "Oligomycin A" can be abbreviated as "Oligomycin".

As used herein, the term "P/S" refers to adding penicillin and streptomycin into the culture medium.

As used herein, the term "a cell" refers to a cell (e.g., single tumor cell) or a group of cells containing multiple similar cells ((e.g., a tumor tissue).

As used herein, "the mitochondrial oxidative phosphorylation pathway inhibitor is suitable for use in the prevention and/or treatment of patient tumor" comprises "patient tumor is sensitive to mitochondrial oxidative phosphorylation pathway inhibitor".

As used herein, "the mitochondrial oxidative phosphorylation pathway inhibitor is not suitable for use in the prevention and/or treatment of patient tumor" comprises "patient tumor is sensitive to mitochondrial oxidative phosphorylation pathway inhibitor".

As used herein, "the expression level or activity of mitochondrial oxidative phosphorylation pathway, the expression level of NNMT gene, the expression level of DNA methylase, the expression level of UHRF1, the methylation level of nucleotide site of NNMT gene, and/or the methylation level of DNA CpG site of NNMT gene" refers to one or more of the expression level or activity of mitochondrial oxidative phosphorylation pathway, the expression level of NNMT gene, the expression level of DNA methylase, the expression level of UHRF1, the methylation level of nucleotide site of NNMT gene, and the methylation level of DNA CpG site of NNMT gene.

As used herein, "the up-regulation of mitochondrial oxidative phosphorylation pathway, low or no expression of NNMT gene, high expression of DNA methylase, high expression of UHRFl, high methylation level of nucleotide site of NNMT gene, and/or high methylation level of DNA CpG site of NNMT gene" refers to one or more of the up-regulation of mitochondrial oxidative phosphorylation pathway, low or no expression of NNMT gene, high expression of DNA methylase, high expression of UHRFl, high methylation level of nucleotide site of NNMT gene, and high methylation level of DNA CpG site of NNMT gene.

As used herein, "the down-regulation of mitochondrial oxidative phosphorylation pathway, high expression of NNMT gene, low expression of DNA methylase, low expression of UHRFl, low methylation level of nucleotide site of NNMT gene, and/or low methylation level of DNA CpG site of NNMT gene" refers to one or more of the down-regulation of mitochondrial oxidative phosphorylation pathway, high expression of NNMT gene, low expression of DNA methylase, low expression of UHRFl, low methylation level of nucleotide site of NNMT gene, and low methylation level of DNA CpG site of NNMT gene.

As used herein, the term "NNMT" refers to Nicotinamide N-Methyltransferase.

As used herein, the term "bp" refers to base pair.

As used herein, the term "SST" refers to the transcription start site.

As used herein, the term "Chr11" refers to human chromosome 11 according to human genome version GCF_ 000001405.25 (GRCh37. p13).

As used herein, the term "human chromosome 11" refers to human chromosome 11 according to human genome version GCF_ 000001405.25 (GRCh37. p13).

As used herein, the terms "before the transcription start site" and "after the transcription start site" do not comprise the transcription start site itself.

As used herein, the terms "site 114165695 on human chromosome 11" refers to nucleotide in site 114165695 of human chromosome 11; "site 114165730 on human chromosome 11" refers to nucleotide in site 114165730 of human chromosome 11; "site 114165769 on human chromosome 11" refers to nucleotide in site 114165769 of human chromosome 11; "site 114165804 on human chromosome 11" refers to nucleotide in site 114165804 of human chromosome 11; "site 114165938 on human chromosome 11" refers to nucleotide in site 114165938 of human chromosome 11; "site 114166050 on human chromosome 11" refers to nucleotide in site 114166050 of human chromosome 11; "site 114166066 on human chromosome 11" refers to nucleotide in site 114166066 of human chromosome 11.

As used herein, the S-adenosyl methionine is abbreviated as SAM.

As used herein, the gene expression comprises the protein expression of the gene and/or the mRNA expression of the gene.

As used herein, the term "DNMT3a" refers to DNA methyltransferase 3a.

As used herein, the term "DNMT3b" refers to DNA methyltransferase 3b.

As used herein, the term "DNMT1" refers to DNA methyltransferase 1.

As used herein, the term "UHRF1" refers to ubiquitin-like with PHD and ring finger domain 1.

It should be understood that the skilled in the art can choose the substituents and substituted forms on the compound of the present invention to obtain chemically stable compounds, the compound can be synthesized by the techniques known in the art and the methods described below. If the compound is substituted by more than one substituents, it should be understood that the substituents can be on the same carbon or different carbons, as long as a stable structure is obtained.

As used herein, the term "substitute" or "substituted" means the hydrogen atom on the group is substituted by a non-hydrogen atom group, but it needs to meet its valence requirements and the substituted compound is chemically stable, that is, the substituted compound does not spontaneously undergo transformations such as cyclization and elimination, etc.

As used herein, "R₁", "R1" and "R¹" have the same meaning and can be used interchangeably. The other similar definitions have the same meaning. As used herein, " 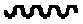 " denotes the linking site of the group.

As used herein, the term "alkyl" refers to a saturated hydrocarbon group with linear chain (ie, unbranched chain) or branched chain, or a combination of linear and branched chains. When the number of carbon atoms is limited in front of the alkyl (e.g., C1-C6 alkyl), it means that the alkyl has 1-6 carbon atoms, for example, C1-C4 alkyl refers to an alkyl having 1-4 carbon atoms. Representative examples comprise but are not limited to methyl, ethyl, propyl, isopropyl, butyl, isobutyl, sec-butyl, tert-butyl, or the like.

As used herein, the term "halogen" refers to F, Cl, Br or I.

As used herein, the term "halo" means the group is substituted by halogen.

As used herein, the term "haloalkyl" means that one or more (preferably 1, 2, 3 or 4) hydrogens on the alkyl are substituted by halogen, the alkyl and halogen are as defined above. When the number of carbon atoms is limited in front of the alkyl (e.g., C1-C8 haloalkyl), it means that the alkyl has 1-8 carbon atoms, for example, C1-C6 haloalkyl refers to an haloalkyl having 1-6 carbon atoms. Representative examples comprise but are not limited to -CF₃, -CHF₂, monofluoroisopropyl, difluorobutyl, or the like.

As used herein, the term "cycloalkyl" refers to a cyclic group having a saturated or partially saturated monocyclic ring, bicyclic ring or polycyclic ring (fused ring, bridged ring or spiro ring). When the number of carbon atoms is limited in front of the cycloalkyl (e.g., C3-C12 cycloalkyl), it means the cycloalkyl has 3-12 ring carbon atoms. In some preferred embodiments, C3-C8 cycloalkyl refers to a saturated or partially saturated monocycloalkyl or dicycloalkyl having 3-8 ring carbon atoms, comprising cyclopropyl, cyclobutyl, cyclopentane , cycloheptyl, or the like.

As used herein, the term "halocycloalkyl" means that one or more (preferably 1, 2, 3 or 4) hydrogens on cycloalkyl are substituted by halogen, the cycloalkyl and halogen are as defined above, When the number of carbon atoms is limited in front of the cycloalkyl (e.g, C3-C8 haloalkyl), it means that the cycloalkyl has 3-8 ring carbon atoms, for example, C3-C8 haloalkyl refers to an halocycloalkyl having 3-6 carbon atoms. Representative examples comprises but are not limited to monofluorocyclopropyl, monochlorocyclobutyl, monofluorocyclopentyl, difluorocycloheptyl, or the like.

As used herein, the term "alkoxyl" refers to R-O- group, wherein R is alkyl, the alkyl is as defined above. When the number of carbon atoms is limited in front of the alkoxyl, for example, C1-C8 alkoxyl means that the alkyl in the alkoxyl has 1-8 carbon atoms. Representative examples of alkoxyl comprise but are not limited to methoxyl, ethoxyl, n-propoxyl, isopropoxyl, tert-butoxyl, or the like.

As used herein, the term "alkylthio" refers to R-S- group, wherein R is alkyl, the alkyl is as defined above. When the number of carbon atoms is limited in front of the alkylthio, for example, C1-C8 alkylthio means that the alkyl in the alkylthio has 1-8 carbon atoms. Representative examples of alkylthio comprise but are not limited to methylthio, ethylthio, n-propylthio, isopropylthio, tert-butylthio, or the like.

As used herein, the term "cycloalkoxyl" refers to R-O- group, wherein R is cycloalkyl, the cycloalkyl is as defined above. When the number of carbon atoms is limited in front of the cycloalkoxyl, for example, C3-C8 cycloalkoxyl means that the cycloalkyl in the cycloalkoxyl has 3-8 carbon atoms. Representative examples of cycloalkoxyl comprise but are not limited to cyclopropyloxyL, cyclobutoxy, or the like.

As used herein, the term "cycloalkylthio" refers to R-S- group, wherein R is cycloalkyl, the cycloalkyl is as defined above. When the number of carbon atoms is limited in front of the cycloalkylthio, for example, C3-C8 cycloalkylthio means that the cycloalkyl in the cycloalkylthio has 3-8 carbon atoms. Representative examples of cycloalkylthio comprise but are not limited to cyclopropylthio, cyclobutythio, or the like.

As used herein, the term "haloalkoxyl" refers to haloalkyl-O-, wherein the haloalkyl is as defined above, for example, C1-C6 haloalkoxyl refers to a haloalkoxyl having 1-6 carbon atoms. Representative examples of haloalkoxyl comprise but are not limited to monofluoromethoxyl, monofluoroethoxyl, bisfluorobutoxyl, or the like.

As used herein, the term "haloalkylthio" refers to haloalkyl-S-, wherein the haloalkyl is as defined above, for example, C1-C6 haloalkylthio refers to a haloalkylthio having 1-6 carbon atoms. Representative examples of haloalkylthio comprise but are not limited to monofluoromethylthio, monofluoroethylthio, difluorobutylthio, or the like.

The term "heterocycloalkyl" refers to fully saturated or partially unsaturated cyclic group (comprising but not limited to such as 3-7 membered monocyclic ring, 7-11 membered bicyclic ring, or 8-16 membered tricyclic ring) , at least one heteroatom is present in a ring with at least one carbon atom. When the number of members is limited in front of the heterocycloalkyl, it refers to the number of ring atoms of the heterocycloalkyl, for example, 3-16 membered heterocycloalkyl refers to a heterocycloalkyl having 3-16 ring atoms. Each heterocyclic ring having heteroatoms can have one or more (e.g., 1, 2, 3 or 4) heteroatoms, each of heteroatoms is independently selected from the group consisting of nitrogen atom, oxygen atom or sulfur atom, wherein the nitrogen atom or sulfur atom can be oxidized, and the nitrogen atom can also be quaternized. Heterocycloalkyl can be attached to any heteroatom or carbon atom residue of ring or ring system molecule. Representative examples of monocyclic heterocycloalkyl comprise but are not limited to azetidinyl, oxetanyl, tetrahydrofuranyl, piperidinyl, piperazinyl, 4-piperidone group, tetrahydropyranyl. Polycyclic heterocycloalkyl comprises spiro, fused and bridged heterocyclyl, the spiro, fused and bridged heterocycloalkyl is optionally linked with other groups by single bond, or further linked with other cycloalkyl rings and heterocyclic rings by any two or more atoms on the ring.

The term "aryl" refers to an all carbon monocyclic ring or fused polycyclic ring (i.e., a ring that share adjacent carbon atom pairs) group with a conjugated π electron system, which is aromatic cyclic hydrocarbon compound group. When the number of carbon atoms is limited in front of the aryl, for example, C6-C12 aryl means that the aryl has 6-12 ring carbon atoms, such as phenyl and naphthyl.

The term "arylene" refers to a group formed by the loss of one hydrogen atom of the aryl, the aryl is as defined above. When the number of carbon atoms is limited in front of the arylene, for example, C6-C12 arylene means that the arylene has 6-12 ring carbon atoms. Representative examples comprise but are not limited to phenylene and naphthylene, or the like.

The term "heteroaryl" refers to aromatic heterocyclic ring group having one to more (preferably 1, 2, 3 or 4) heteroatoms, the heteroaryl can be monocyclic ring (monocyclic), or polycyclic ring (bicyclic, tricyclic or polycyclic) fused together or covalently connected. Each of heterocyclic ring having heteroatom can have one or more (e.g., 1, 2, 3, 4) heteroatoms independently selected from the group consisting of oxygen, sulfur and nitrogen. When the number of members is limited in front of the heteroaryl, it refers to the number of ring atoms of the heteroaryl, for example, 5-12 membered heteroaryl refers to a heteroaryl having 5-12 ring atoms. Representative examples comprise but are not limited to pyrrolyl, pyrazolyl, imidazolyl, oxazolyl, isoxazolyl, thiazolyl, thiadiazolyl, isothiazolyl, furanyl, pyridyl, pyrazinyl, pyrimidinyl, pyridazinyl, triazolyl and tetrazolyl, etc.

The term "heteroarylene" refers to a group formed by the loss of one hydrogen atom of the heteroaryl, the heteroaryl is as defined above. When the number of carbon atoms is limited in front of the heteroarylene, for example, C6-C12 heteroarylene means that the heteroarylene has 6-12 ring carbon atoms. Representative examples comprise but are not limited to pyrrolylene, pyrazolylene, imidazolylene, triazolylene and oxazolylene, or the like.

As used herein, when used alone or as part of other substituents, the term "methylsulfonyl" is

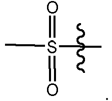

In present invention, it should be understood that all substituents are unsubstituted, unless explicitly described herein as "substituted". The term "substituted" means that one or more hydrogen atoms on the specified group are substituted by specified substituent. The specific substituent is the substituent as described above, or the substituent in each example. Preferably, the "substituted" means that one or more (preferably 1, 2, 3, or 4) hydrogen atoms on the group are substituted by a substituent selected from the group consisting of C1-C8 alkyl, C3-C8 cycloalkyl, C1-C8 haloalkyl (e.g., trifluoromethyl), C3-C8 halocycloalkyl, halogen, nitro, -CN, hydroxyl, sulfhydryl, amino, C1-C8 alkoxyl, C1-C8 alkylthio, C3-C8 cycloalkoxyl, C3-C8 cycloalkylthio, C1-C8 haloalkoxyl, C1-C8 haloalkylthio, C6-C12 aryl, 5-10 membered heteroaryl, methylsulfonyl, sulfonyl. Unless otherwise specified, each substituted group can have a substituent selected from a specified group at any substituted position of the group, the substitution can be the same or different at each substituted position.

In the present invention, the term "prevention" refers to a method of preventing the occurrence of disease and/or its accompanying symptoms, or protecting a subject from getting disease.

In the present invention, the term "treatment" comprises delaying and terminating the progression of the disease, or eliminating the disease, and it does not require 100% inhibition, elimination and reversal. In some embodiments, compared to the level observed in the absence of the mitochondrial oxidative phosphorylation pathway inhibitor of the present invention, the mitochondrial oxidative phosphorylation pathway inhibitor of the present invention alleviates, inhibits and/or reverses related diseases (e.g., tumor) and its accompanying symptoms such as by at least about 10%, at least about 30%, at least about 50%, or at least about 80%, or 100%.

### Mitochondrial oxidative phosphorylation pathway inhibitor

Oxidative Phosphorylation (OXPHOS) is one of the most important pathways in mitochondria, which utilizes NADH and FADH derived from tricarboxylic acid cycle and fat oxidation, etc to produce ATP. The mitochondrial oxidative phosphorylation pathway is composed of more than 90 proteins, which form five protein complexes, complexes I, II, III, IV and V. The first four protein complexes (complexes I, II, III and IV), also known as the electron transport chain, receive electrons from electron donors NADH and FADH and transfer them to oxygen. In the process of electron transfer, hydrogen ions are pumped from the mitochondrial inner membrane to the intermembrane space between the mitochondrial inner membrane and the mitochondrial outer membrane, thereby forming a hydrogen ion gradient and potential difference inside and outside the inner membrane. The energy stored in the mitochondria membrane potential drives complex V in the oxidative phosphorylation pathway to produce ATP. Studies have shown that the mitochondrial oxidative phosphorylation pathway is very important for cell growth and is related to many diseases such as tumors, immune-related diseases and neurodegenerative diseases. Inhibiting the mitochondrial oxidative phosphorylation pathway can treat tumors, immune related diseases and neurodegenerative diseases, especially tumor cells with high malignancy and stem cell properties are extremely dependent on this pathway for survival, inhibiting this pathway can effectively kill such tumor cells, thereby solving the problem of related malignant cancer recurrence.

### NNMT gene

In the present invention, the English name of NNMT is Nicotinamide N-Methyltransferase. Different databases have different identification numbers for NNMT gene as follows: HGNC: 7861; Entrez Gene: 4837; Ensembl: ENSG00000166741; OMIM: 600008; UniProtKB: P40261

According to version GCF_000001405.25 (GRCh37.p13) of human genome, the NNMT gene is located at 114,128,528 bp to 114,184,258 bp on human chromosome 11, the total length of DNA sequence of NNMT gene is 55,731 bp, the NNMT gene comprises promoter region, exon region and intron region, the transcription start site of NNMT gene is at site 114,166,535 bp.

The promoter region of NNMT gene is the nucleotide sequence from the 114164535 bp to 114167034 bp on human chromosome 11, i.e. the sequence from 2000 bp before the transcription start site (bold section) to the transcription start site itself and 499 bp after the transcription start site (underlined section) in NNMT gene, the total length of promoter region of NNMT gene is 2500 bp, The nucleotide sequence of the promoter region of NNMT gene is as shown in SEQ ID NO: 1 as follows:

SEQ ID NO: 1:
TATCCAAGAGCTATCAGCACTCCCATGTTTATTGTAGCACTGTTCACAATA
GCCAAGATTTGGAAGTACTCTAAGTGTCCATTAGCAGATGAATGGATAAAGAC
AATGTGGTAATACACATAATGGAGTACTATTCAGTCATAAAGAAGAATTAGAT
CCTGTCATTTGCAATAACATGGATGGAACTGGAGGTCATAATGTTGAGTGAAA
TAAACCAGGCACAGAAAGACAAACTTTGCATGTTCTCACTTATTTATGGGAGC
TAAAAACTAAAATAACTGAACTCACAGAGATAGAGAGTAGAAGGATGGTTACG
AGAGGATGGGAAGGGTAGCGAGGTGGGTAGGGGGGATGTGGGGATCATTAA
TGGGTATAAAAAATAGTTAGAGGCCAGGCGCAGTGGCTCACGCCTGTAATCC
CAGCACTTTGGGAGGCCGAGGTAGGCGGAACACCTGAGGAGTTCAAGACCAG
CCTGGCCAATATGATGAAACCCCGTCTCTACTAAAAATACAAAAATTAGCTGG
GCGTGATGGTGTGCACCTGTAGTCCCAGCTGCTTGGGAGGCTGAGGCAGGAG
AATCGCTGGAACCCAAGAGGTGAAGGTTGCAGTGAGCTGAGATCGCGTCACT
GCACTCCAGCCTGGGTGACAGAGTGAGACTCCACATCAAAAAAAAAAAAAA
AAAGTTAGAAAGATTGAATAAGACCTAATATTTGCTAGCACAACAGGGTGAATA
TAGTAAAAAATAATTTATTTGTACCTTCAAAAATAACTAGACAAGTATAATTGG
GTTGTTTGTAACACACAAAAAATAAGTACTTGAAGTGGTGGATACCCCATTTA
CCCTGATGTGATTATTTTGTATTGCAGGCCTCTATCAGAATATCTCATGTAACC
CATAAATATATACACCTACTCTGTACCCACAAAAAGTTTTTAAAAAGAAAAATA
AATAGCAACCGAAAAAAAAAGAGAGGGAGAAAAGAAAAAAGAAAAAAAAATC
AAGTGCCTGGCTGGGTAGAATAAATTCTAAGGCCACAATGTTACTGACCATGG
GTTTTTTGGCTCTCAGTGTATAGAAATTGACACAAGGCCAATAGTCTTCCCAA
ACATGCTTTACTGGAACTTACGCCCTGGCATAAGGGCCACAACAAAAGAGAGA
GCGAATTCTCTGGCTTGCTGACTCCTTGGAAAAAACCGGTAGGGATTTTTTTA
TTAGGCAAAGCACAGGAATTGACGTCAGAGGCAGGATGTGCTGCTGGGCAAA
GCATACGAGAAGTGGGGTATGCAGGTCAGCATTACTTGGTTGCAATGGTTATC
TTGAGGAATGGGCCAACTGGTGGTCTGGCCAGTGGCAACAAGGCTGTAAATC
AATTATTCAGCATTCCTTCCCAAGGTGGGACACCCGGCAACATTGTTTATCTC
CTAAGGCCAGTTCCTGGAATTAAGTGAAAGGATGACTAATGGACATGTTGTCA

GTGAGGTAGTGGTGTGGGTTTTGTGACCAGTGGGAATGCACGAAAGAATGCT
TTAGCGGGGAGTGAGCTGAAGCCAAGCCCCATCCCTACTCTGTCTCAAAGTGA
GTTCAGAAAAGGGGATTTAAAGAATTCTTTTTTTTTTTTTTTTTTTTTTTGAGA
CAGAGTCTTGCTCTGTCGCCCAGGCTGGAGTGCAGTGGCGCCATCTTGGCTC
ACTGCAAGCTCCGCCCCCCGGGTTCATGCCATTCTCCTGCCTCAGCCTCCCAA
GTAGCTGGGACTGCAGGTGCCTACCACCAAGCCCAGCTAATTTTTTGTATTTTT
TTTTTAGTAGAGACGGGGTTTCACCATGTTAGCCAGGATGGTCTCGATCTCC
TGACCTCGTGATCTGCCCGCCTTAGCCTCCCAAAGTGCTGGGATTACAGGCAT
GAGCCTCCGCCCCCGGCCTTAAATAATTCTTAAAGGAAGTAAAGTTAACTTTG
AAAGAACTATCAGGATTTGGATTGACTGAAAGGAGTGGGGAAGCTTAGGGAG
GAGGTGCTTGCCAGACACTGGGTCATGGCAGTGGTCGGTGAAGCTGCAGTTGCCTA
GGGCAGGGATGGAGAGAGAGTCTGGGCATGAGGAGAGGGTCTCGGGATGTTTGGC
TGGACTAGATTTTACAGAAAGCCTTATCCAGGCTTTTAAAATTACTCTTTCCAGACT
TCATCTGAGACTCCTTCTTCAGCCAACATTCCTTAGCCCTGAATACATTTCCTATCC
TCATCTTTCCCTTCTTTTTTTTCCTTTCTTTTACATGTTTAAATTTAAACCATTCTTCG
TGACCCCTTTTCTTGGGAGATTCATGGCAAGAACGAGAAGAATGATGGTGCTTGTT
AGGGGATGTCCTGTCTCTCTGAACTTTGGGGTCCTATGCATTAAATAATTTTCCTGA
CGAGCTCAAGTGCTCCCTCTGGTCTACAATCCCTGGCGGCTGGCCTTCATCCCTTGG
GCAAGCATTGCATACAGCTCATGGCCCTCCCTCTACCATACCC.

In the present invention, the nucleotide sites from 1050 bp before the transcription start site to 499 bp after the transcription start site in NNMT gene is sites 951-2500 of nucleotide sequence as shown in SEQ ID NO: 1.

In the present invention, the nucleotide sites from 1050 bp to 193 bp before the transcription start site in NNMT gene is sites 951-1808 of nucleotide sequence as shown in SEQ ID NO: 1.

In the present invention, the nucleotide sites from 840 bp to 469 bp before the transcription start site in NNMT gene is sites 1161-1532 of nucleotide sequence as shown in SEQ ID NO: 1.

In present invention, the site 114165695, site 114165730, site 114165769, site 114165804, site 114165938, site 114166050 and site 114166066 on the human chromosome 11 correspond to the nucleotide site in SEQ ID NO: 1 as shown in Table 1:

**Table 1**

| Site on the human chromosome 11 | Correspond to the nucleotide site in SEQ ID NO: 1 |
|---|---|
| site 114165695 | site 1161 |
| site 114165730 | site 1196 |
| site 114165769 | site 1235 |
| site 114165804 | site 1270 |
| site 114165938 | site 1404 |
| site 114166050 | site 1516 |
| site 114166066 | site 1532 |

### DNA methylation

DNA methylation is a form of chemical modification of DNA, which can change genetic performance under no change of DNA sequence. Many studies have shown that DNA methylation can cause changes in chromatin structure, DNA conformation, DNA stability and the way DNA interacts with protein, thereby regulating gene expression.

DNA methylation is one of the earliest discovered and most deeply studied epigenetic regulatory mechanisms. Broadly speaking, DNA methylation refers to the chemical modification process in which a specific base in the DNA sequence is modified with a methyl by covalent bonding with S-adenosyl methionine (SAM) as methyl donor under the catalysis of DNA methyltransferase (DNMT). This DNA methylation can occur at position C-5 of cytosine, position N-6 of adenine and position N-7 of guanine. DNA methylation in general studies mainly refers to the methylation process that occurs at the carbon atom of position C-5 on cytosine in CpG dinucleotides, the product is called 5-methylcytosine (5-mC). The 5-methylcytosine (5-mC) is the main form of DNA methylation in eukaryotic organisms such as plants and animals. DNA methylation, as a relatively stable modification state, can be passed on to new generations of DNA during DNA replication process under the action of DNA methyltransferase, which is an important epigenetic mechanism.

There are two types of DNA methylation reactions. One type is that the DNA with two unmethylated strands is methylated, which is called denovo methylation; The other type is that the unmethylated strand of double-stranded DNA with one methylated strand and one unmethylated strand is methylated, which is called maintenance methylation.

Typically, DNA methylation is the methylation of DNA CpG site. The distribution of CpG binucleotide is very uneven in the human genome, while CpG remains or is higher than normal level in some regions of the genome. The CpG site enrichment region (also known as CpG island) is mainly located in the promoter region and exon regions of the gene, which is a region rich in CpG dinucleotide. About 60% of the promoters of the gene contains CpG island. The CpG is the abbreviation of cytosine (C)-phosphate (p)-guanine (G).

Gene expression is regulated by various signaling pathways, transcription factors and epigenetic modifications in the cell. DNA methylation modification is an important way in which epigenetic modifications regulate gene expression. The level of DNA methylation in a specific gene region often affects the expression level of the gene. Compared to the regulation of gene expression by signal transduction pathways and transcription factors, the effect of DNA methylation modification on gene expression is more stable in epigenetic modification, which is not easily affected by the extracellular environment. DNA methylation modification can be easily and accurately detected using existing technologies, so the DNA methylation is ideal biomarkers.

### Mitochondrial oxidative phosphorylation pathway inhibitor and use thereof

The present invention provides a mitochondrial oxidative phosphorylation pathway inhibitor, the mitochondrial oxidative phosphorylation pathway inhibitor can be used for preventing and treating tumors.

In a preferred embodiment of the present invention, the mitochondrial oxidative phosphorylation pathway inhibitor comprises a compound of formula I, II, and/or III, or an optical isomer thereof, or a racemate thereof, or a solvate thereof, or a pharmaceutically acceptable salt thereof

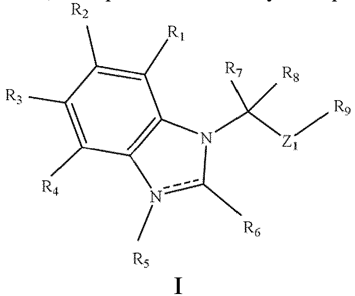

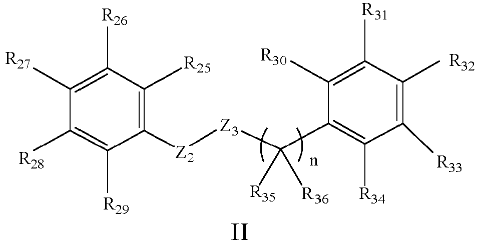

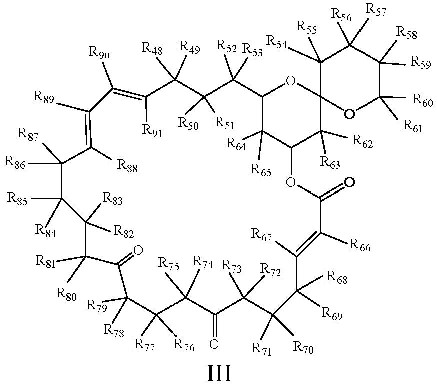

Specifically, the compound of formula I, II, and/or III is as described above in the first aspect of the present invention.

As used herein, the terms "compound of formula I of the present invention" and "compound of formula I" are used interchangeably, and refer to a compound of formula I, or an optical isomer thereof, or a racemate thereof, or a solvate thereof, or a pharmaceutically acceptable salt thereof. It should be understood that the term also comprises a mixture of the above components.

As used herein, the terms "compound of formula II of the present invention" and "compound of formula II " are used interchangeably, and refer to a compound of formula II, or an optical isomer thereof, or a racemate thereof, or a solvate thereof, or a pharmaceutically acceptable salt thereof. It should be understood that the term also comprises a mixture of the above components.

As used herein, the terms "compound of formula III of the present invention" and "compound of formula III " are used interchangeably, and refer to a compound of formula III, or an optical isomer thereof, or a racemate thereof, or a solvate thereof, or a pharmaceutically acceptable salt thereof. It should be understood that the term also comprises a mixture of the above components.

The term "pharmaceutically acceptable salt" refers to a salt formed by a compound of the present invention and an acid or a base, the salt is suitable for use as a drug. Pharmaceutically acceptable salts comprises inorganic salts and organic salts. A preferred type of salt is the salt formed by the compound of the present invention and an acid. Acids suitable for salt formation comprise but are not limited to inorganic acid such as hydrochloric acid, hydrobromic acid, hydrofluoric acid, hydriodic acid, sulfuric acid, nitric acid, phosphoric acid and the like; organic acid such as formic acid, acetic acid, propionic acid, oxalic acid, malonic acid, succinic acid, fumaric acid, maleic acid, lactic acid, malic acid, tartaric acid, citric acid, picric acid, methanesulfonic acid, benzenemethanesulfonic acid, benzenesulfonic acid and the like; and acidic amino acid such as aspartic acid and glutamic acid. A preferred type of salt is a metal salt formed by the compound of the present invention and a base. Suitable bases for salt formation comprise but are not limited to inorganic base such as sodium hydroxide, potassium hydroxide, sodium carbonate, sodium bicarbonate, sodium phosphate and the like; and organic base such as ammonia, triethylamine, diethylamine and the like.

The compound of formula I in present invention can be converted into its pharmaceutically acceptable salt by conventional methods. For example, a solution of corresponding acid can be added into the solution of above compounds, and the solvent is removed after the salt is formed, thereby forming the corresponding salt of the compound of the present invention.

Representively, the mitochondrial oxidative phosphorylation pathway inhibitors are selected from the following group:

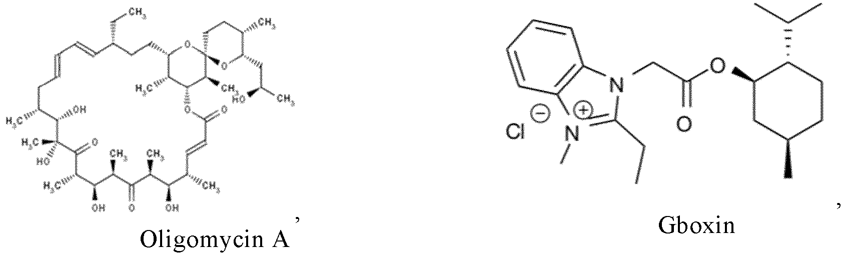

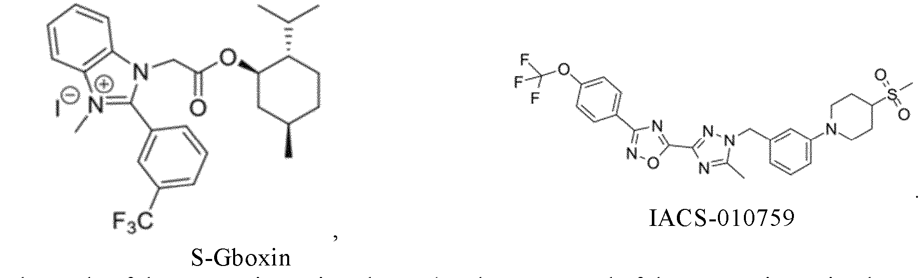

The study of the present invention shows that the compound of the present invention has more significant inhibitory effect on tumors with up-regulation of mitochondrial oxidative phosphorylation pathway, low or no expression of NNMT gene, high expression of DNA methylase, high expression of UHRFl, high methylation level of nucleotide site of NNMT gene, and/or high methylation level of DNA CpG site of NNMT gene. The tumor with up-regulation of mitochondrial oxidative phosphorylation pathway, low or no expression of NNMT gene, high expression of DNA methylase, high expression of UHRFl, high methylation level of nucleotide site of NNMT gene, and/or high methylation level of DNA CpG site of NNMT gene is sensitive to mitochondrial oxidative phosphorylation pathway inhibitor of present application.

### Tumor

The studies of the present invention shows the mitochondrial oxidative phosphorylation pathway inhibitor of present application can be used for preventing and treating tumors.

As used herein, the term "tumor " and "cancer" are used interchangeably.

In a preferred embodiment of the present invention, the tumor comprises tumor with up-regulation of mitochondrial oxidative phosphorylation pathway. Typically, the tumor with up-regulation of mitochondrial oxidative phosphorylation pathway is as described above in the first aspect of the present invention.

In a preferred embodiment of the present invention, the tumor comprises tumor with low or no expression of NNMT gene. Typically, the tumor with low or no expression of NNMT gene is as described above in the first aspect of the present invention.

In a preferred embodiment of the present invention, the tumor comprises tumor with high expression of DNA methylase. Typically, the tumor with high expression of DNA methylase is as described above in the first aspect of the present invention.

The DNA methylase of present invention comprises but is not limited to DNMT1, DNMT3a, DNMT3b, and combinations thereof. Preferably, the DNA methylase comprises DNMT1.

In a preferred embodiment of the present invention, the tumor comprises tumor with high expression of DNMT1. Typically, the tumor with high expression of DNMT1 is as described above in the first aspect of the present invention.

In a preferred embodiment of the present invention, the tumor comprises tumor with high expression of DNMT3a. Typically, the tumor with high expression of DNMT3a is as described above in the first aspect of the present invention.

In a preferred embodiment of the present invention, the tumor comprises tumor with high expression of DNMT3b. Typically, the tumor with high expression of DNMT3b is as described above in the first aspect of the present invention.

In a preferred embodiment of the present invention, the tumor comprises tumor with high expression of UHRF1 (ubiquitin-like with PHD and ring finger domain 1). Typically, the tumor with high expression of UHRF 1 is as described above in the first aspect of the present invention.

In a preferred embodiment of the present invention, the tumor comprises tumor with high methylation level of nucleotide site of NNMT gene. Typically, the tumor with high methylation level of nucleotide site of NNMT gene is as described above in the first aspect of the present invention.

In a preferred embodiment of the present invention, the tumor comprises tumor with high methylation level of DNA CpG site of NNMT gene. Typically, the tumor with high methylation level of DNA CpG site of NNMT gene comprises is as described above in the first aspect of the present invention.

Specifically, the tumor of present invention is as described above in the first aspect of the present invention.

In present invention, the corresponding tumor types of tumor cell lines are shown in Table 2:

**Table 2**

| Tumor cell line | The corresponding tumor types |
|---|---|
| NCI-H82 | Human small cell lung cancer cell |
| G-401 | human renal carcinoma Wilms cell |
| MDA-MB-453 | Breast cancer cell |
| WSU-DLCL2 | Human diffuse large B lymphoma cell |
| SU-DHL-2 | Large cell lymphoma cell |
| OCI-AML-3 | FAB M4 type acute myeloid leukemia |
| SW48 | Human colon adenocarcinoma cell |
| ATN-1 | T-cell leukemia cell |
| HCC15 | Non-small cell lung cancer cell |
| OCI-LY-19 | B-cell lymphoma cell |
| 22RV1 | Prostate cancer cell |
| MIA PaCa-2 | Pancreatic cancer cell |
| CCRF-CEM | Acute T-lymphocyte leukemia cell |
| HH | Skin T-cell lymphoma cell |
| OCI-AML-5 | M4 type acute myeloid leukemia. |
| G-402 | Renal carcinoma cell |
| HCC1806 | Breast cancer cell |
| BT-549 | Breast cancer cell |
| OCI-AML-4 | Acute myeloid leukemia cell |
| H9 | Lymphoma cell |
| Jurkat, Clone E6-1 | T lymphoma cell |
| G-361 | Melanoma cell |
| U-937 | Histiocytic lymphoma cell |
| SNU-398 | Hepatocellular carcinoma cell |
| NCI-H1048 | Small cell lung cancer cell |
| A-375 | Melanoma cell |
| D283 Med | Medulloblastoma cell |
| GAK | Melanoma cell |
| CHL-1 | Melanoma cell |
| NCI-H1155 | Non-small cell lung cancer cell |
| LS 180 | Colorectal adenocarcinoma cell |
| Daoy | Medulloblastoma cell |
| DU 145 | Brain metastatic prostate cancer cell |
| AM-38 | Glioblastoma multiforme cell |
| HCC70 | Grade 3 primary breast ductal carcinoma cell |
| PANC-1 | Pancreatic cancer cell |
| U-87 MG | Brain tumor cell |
| MJ | Human skin T lymphoma cell |
| Gp2D | Human colon cancer cell |
| SU.86.86 | Pancreatic cancer cell |
| NCI-H2081 | Small cell lung cancer cell |
| NCI-H1793 | Non-small cell lung cancer cell |
| ACHN | Renal carcinoma cell |
| U-251 MG | Neuroglioma cell |
| MDA-MB-231 | Breast cancer cell |
| NCI-H196 | Lung cancer cell |
| PC-3 | Prostate cancer cel |
| OCI-M1 | Acute myeloid leukemia cell |
| NCI-H1651 | Non-small cell lung cancer |
| C3A | Liver cancer cell |
| SNU-449 | Liver cancer cell |
| GB-1 | Glioblastoma cell |
| 769-P | Renal carcinoma cell |
| COLO 320HSR | Colorectal adenocarcinoma cell |
| CFPAC-1 | Pancreatic cancer cell |
| SF126 | Brain tumor cell |
| 786-O | Clear cell renal cell adenocarcinoma |

### Marker

The present invention provides a marker for determining whether a mitochondrial oxidative phosphorylation pathway inhibitor is suitable for use in the prevention and/or treatment of patient tumor, the marker comprises the expression level or activity of mitochondrial oxidative phosphorylation pathway, the expression level of NNMT gene, the expression level of DNA methylase, the expression level of UHRF1, the methylation level of nucleotide site of NNMT gene, and/or the methylation level of DNA CpG site of NNMT gene.

In a preferred embodiment of the present invention, the expression level or activity of mitochondrial oxidative phosphorylation pathway, the expression level of NNMT gene, the expression level of DNA methylase, the expression level of UHRF1, the methylation level of nucleotide site of NNMT gene, and/or the methylation level of DNA CpG site of NNMT gene are used as a marker for determining whether a mitochondrial oxidative phosphorylation pathway inhibitor is suitable for use in the prevention and/or treatment of patient tumor, the method comprises as follows:
the mitochondrial oxidative phosphorylation pathway inhibitor is suitable for use in the prevention and/or treatment of patient tumor with up-regulation of mitochondrial oxidative phosphorylation pathway, low or no expression of NNMT gene, high expression of DNA methylase, high expression of UHRFl, high methylation level of nucleotide site of NNMT gene, and/or high methylation level of DNA CpG site of NNMT gene; and/or
the mitochondrial oxidative phosphorylation pathway inhibitor is not suitable for use in the prevention and/or treatment of patient tumor with down-regulation of mitochondrial oxidative phosphorylation pathway, high expression of NNMT gene, low expression of DNA methylase, low expression of UHRFl, low methylation level of nucleotide site of NNMT gene, and/or low methylation level of DNA CpG site of NNMT gene.

Specifically, the tumor with up-regulation of mitochondrial oxidative phosphorylation pathway, low or no expression of NNMT gene, high expression of DNA methylase (e.g, NNMT1), high expression of UHRFl, high methylation level of nucleotide site of NNMT gene, and/or high methylation level of DNA CpG site of NNMT gene is as described above in the first aspect of the present invention.

Specifically, the tumor with down-regulation of mitochondrial oxidative phosphorylation pathway, high expression of NNMT gene, low expression of DNA methylase (e.g, NNMT1), low expression of UHRFl, low methylation level of nucleotide site of NNMT gene, and/or low methylation level of DNA CpG site of NNMT genee is as described above in the second aspect of the present invention.

### Composition or preparation, active ingredient combination, medical kit and administration routes

Preferably, the composition of the present invention is pharmaceutical composition. The compositions of the present invention can comprise a pharmaceutically acceptable carrier.

The term "pharmaceutically acceptable carrier" refers to one or more compatible solid, semi-solid, liquid or gel fillers, which are suitable for use in humans or animals and must have sufficient purity and sufficiently low toxicity. The "compatible" means each component and drug active ingredient in the pharmaceutical composition can be blended with each other without significantly reducing the efficacy.

It should be understood that the pharmaceutically acceptable carrier is not particularly limited in the present invention, the carrier can be selected from materials commonly used in the art, or can be obtained by a conventional method, or is commercially available. Some examples of pharmaceutically acceptable carriers are cellulose and its derivatives (e.g., methylcellulose, ethylcellulose, hydroxypropylmethylcellulose, sodium carboxymethylcellulose, etc.), gelatin, talc, solid lubricants (e.g., stearic acid, magnesium stearate), calcium sulfate, plant oil (e.g., soybean oil, sesame oil, peanut oil, olive oil, etc.), polyols (e.g., propylene glycol, glycerin, mannitol, sorbitol, etc.), emulsifier (e.g., Tween), wetting agent (e.g., sodium lauryl sulfate), buffer agent, chelating agent, thickener, pH regulator, transdermal enhancer, colorant, flavoring agent, stabilizer, antioxidant, preservative, bacteriostatic agent, pyrogen-free water, etc.

In a preferred embodiment of the present invention, the dosage form of the composition or preparation is a solid preparation, liquid preparation or semi-solid preparation.

In a preferred embodiment of the present invention, the dosage form of the composition or preparation is oral preparation, external preparation or injection preparation

In a preferred embodiment of the present invention, the dosage form of the composition or preparation is tablet, injection, infusion, paste, gel, solution, microsphere or film.

The pharmaceutical preparation should be matched with the mode of administration. The pharmaceutical preparation of the present invention can also be given together with other synergistic therapeutic drugs before, during or after the administration. When the pharmaceutical composition or preparation is administrated, a safe and effective amount of the drug is administered to a subject in need (e.g. human or non-human mammal). The safe and effective amount is usually at least about 10 µg/kg.bw, and does not exceed about 8 µg/kg.bw in most case, preferably, the dose is about 1-10 µg/kg.bw. Of course, the specific dose should also take into account the route of administration, the patient's health and other factors, which are within the skill range of skilled doctors.

### The main advantages of the present invention comprise:

The invention provides a marker for guiding precise administration of the mitochondrial oxidative phosphorylation pathway inhibitors, the marker can be used to effectively identify tumor patients sensitive to such anti-tumor drugs, improve treatment effect of drugs, and avoid administrating such drugs to tumor patients insensitive to such anti-tumor drugs, thus realizing the precise application of the mitochondrial oxidative phosphorylation pathway inhibitors.

The present invention has unexpectedly found the expression level or activity of mitochondrial oxidative phosphorylation pathway, the expression level of NNMT gene, the expression level of DNA methylase, the expression level of UHRF1, the methylation level of nucleotide site of NNMT gene, and/or the methylation level of DNA CpG site of NNMT gene can be used as a marker for determining whether the mitochondrial oxidative phosphorylation pathway inhibitor is suitable for use in the treatment of specific tumor.

The tumor with up-regulation of mitochondrial oxidative phosphorylation pathway, low or no expression of NNMT gene, high expression of DNA methylase, high expression of UHRFl, high methylation level of nucleotide site of NNMT gene, and/or high methylation level of DNA CpG site of NNMT gene is highly sensitive to the mitochondrial oxidative phosphorylation pathway inhibitors, ie, the mitochondrial oxidative phosphorylation pathway inhibitor has significant inhibitory effect on tumor with up-regulation of mitochondrial oxidative phosphorylation pathway, low or no expression of NNMT gene, high expression of DNA methylase, high expression of UHRFl, high methylation level of nucleotide site of NNMT gene, and/or high methylation level of DNA CpG site of NNMT gene. Moreover, the detection method of methylation level of DNA CpG site is stable and reliable, which is suitable for the development of molecular markers.

The present invention will be further illustrated below with reference to the specific examples. It should be understood that these examples are only to illustrate the invention but are not to limit the scope of the invention. The experimental methods with no specific conditions described in the following examples are generally performed under the conventional conditions, or according to the manufacturer's instructions. Unless indicated otherwise, parts and percentage are calculated by weight.

### Example

Oligomycin A, Gboxin, S-Gboxin and IACS-010759 were known mitochondrial oxidative phosphorylation pathway inhibitor.

The structural formula of Oligomycin A was as follows:

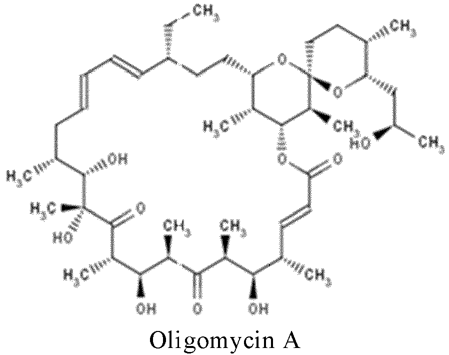

The structural formula of Gboxin was as follows:

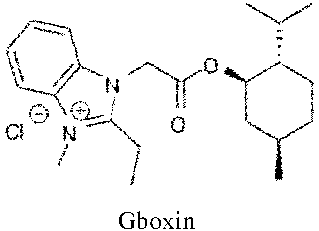

The structural formula of S-Gboxin was as follows:

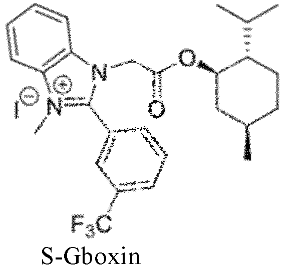

The structural formula of IACS-010759 was as follows:

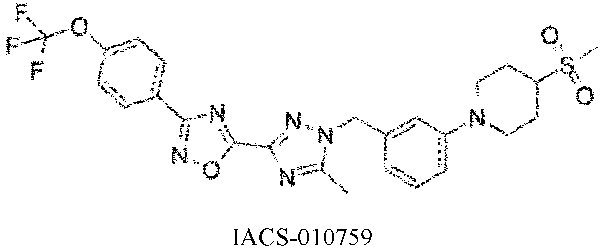

DNMT3a refered to DNA methyltransferase 3a, NCBI entrez gene: 1788; Uniprotkb/Swiss-port: Q9Y6K1.

DNMT3b refered to DNA methyltransferase 3b, NCBI entrez gene: 1789; Uniprotkb/Swiss-port: Q9UBC3.

DNMT1 refered to DNA methyltransferase 1, NCBI entrez gene: 1786; Uniprotkb/Swiss-port: P26358.

UHRF1 refered to ubiquitin-like with PHD and ring finger domain 1, NCBI entrez gene: 29128; Uniprotkb/Swiss-port:Q96T88.

NNMT refered to Nicotinamide N-Methyltransferase.

The nucleotide sequence of promoter region of NNMT gene was as shown in SEQ ID NO: 1.

The nucleotide sites from 1050 bp before the transcription start site to 499 bp after the transcription start site in NNMT gene was sites 951-2500 of nucleotide sequence as shown in SEQ ID NO: 1.

The nucleotide sites from 1050 bp to 193 bp before the transcription start site in NNMT gene was sites 951-1808 of nucleotide sequence as shown in SEQ ID NO: 1.

The nucleotide sites from 840 bp to 469 bp before the transcription start site in NNMT gene was sites 1161-1532 of nucleotide sequence shown in SEQ ID NO: 1.

### Example 1

### Experimental background:

The oxygen was mainly consumed by mitochondrial oxidative phosphorylation pathway in the cell, thus the determination of oxygen consumption rate (OCR) of mitochondria could directly reflect the activity of mitochondrial oxidative phosphorylation pathway. The oxygen consumption of NCI-H82 cell in the presence or absence of Oligomycin A, Gboxin, S-Gboxin and IACS-010759, mitochondrial oxidative phosphorylation pathway inhibitors, was detected using Seahorse XFe metabolic analyzer in the present Example.

### Experimental method and result:

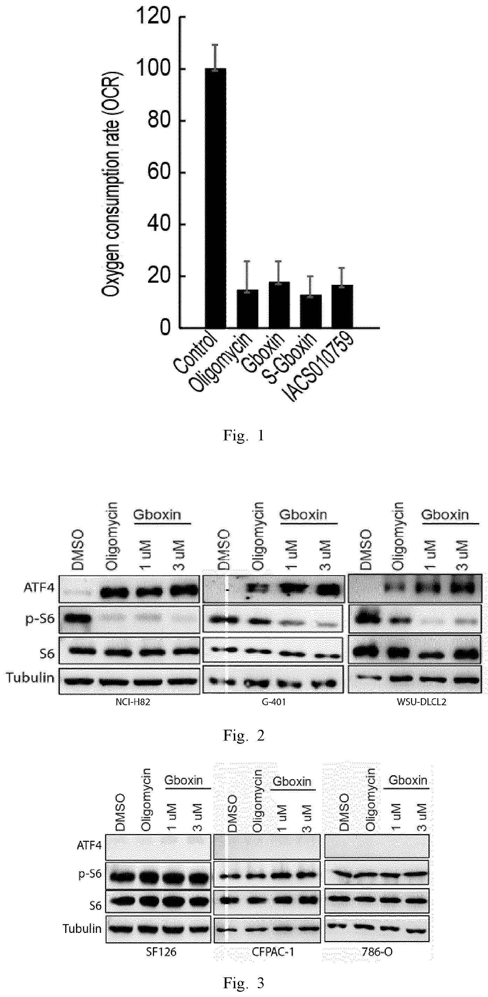

NCI-H82 cell (ATCC, No. HTB-175) was cultured in 10% FBS-containing RPMI1640 medium (+p/s), Oligomycin A (1µM), Gboxin (2µM), S-Gboxin (5µM) or IACS-010759(1µM), a mitochondrial oxidative phosphorylation pathway inhibitor, was added respectively. The control group in absence of mitochondrial oxidative phosphorylation pathway inhibitor was set. The detection of cell oxygen consumption was finished within 0.5 h. The experiment result was shown in Fig.1.

The Fig.1 showed that compared with the control group in absence of mitochondrial oxidative phosphorylation pathway inhibitor, the addition of small molecular compounds Oligomycin A, Gboxin, S-Gboxin and IACS-010759 could significantly inhibit the oxygen consumption of NCI-H82 cells, indicating Oligomycin A, Gboxin, S-Gboxin and IACS-010759 could effectively inhibit the mitochondrial oxidative phosphorylation pathway.

### Example 2

Cell lines derived from different tissues with different genotypes were randomly selected, the sensitivity of cell lines to Gboxin, a mitochondrial oxidative phosphorylation pathway inhibitor, was detected using cell activity detection reagent. The results showed that some cell lines were sensitive to Gboxin with low IC₅₀ value, while other cell lines were not sensitive to Gboxin with high IC₅₀ value.

### Experimental background:

Cell viability was detected using the Promega CellTiter-Glo kit, the kit reflected cell viability by directly measuring intracellular ATP content. The inhibitory effect (IC₅₀ value) of Gboxin, a mitochondrial oxidative phosphorylation pathway inhibitor, on different tumor cell lines was tested in the Example. The name, source, and culture condition of each tumor cell line were as follows:
Cell line NCI-H82 (ATCC, No. HTB-175) was cultured in 10% fetal bovine serum-containing RPMI1640 medium (+P/S).
Cell line G-401 (ATCC, No. CRL-1441) was cultured in 10% fetal bovine serum-containing McCoy's 5a medium (+P/S).
Cell line MDA-MB-453 (ATCC, No. HTB-131) was cultured in 10% fetal bovine serum-containing Leibovitz's L-15 medium (+P/S).
Cell line WSU-DLCL2 (DSMZ, No. ACC-575) was cultured in 10% fetal bovine serum-containing RPMI1640 medium (+P/S).
Cell line SU-DHL-2 (ATCC, No. CRL-2956) was cultured in 10% fetal bovine serum-containing RPMI1640 medium (+P/S).
Cell line OCI-AML-3 (DSMZ, No. ACC-582) was cultured in 20% fetal bovine serum-containing RPMI1640 medium (+P/S).
Cell line SW48 (ATCC, No. CCL-231) was cultured in 10% fetal bovine serum-containing Leibovitz's L-15 medium (+P/S).
Cell line ATN-1 (RIKEN, No. RBRC-RCB1440) was cultured in RPMI1640 medium containing 10% fetal bovine serum and 0.1mM NEAA (+P/S).
Cell line HCC15 (KCLB, No. 70015) was cultured in 10% fetal bovine serum-containing RPMI1640 medium (+P/S).
Cell line OCI-LY-19 (DSMZ, No. ACC-528) was cultured in 80-90% alpha-MEM medium containing 10-20% h.i. FBS (+P/S).
Cell line 22RV1 (ATCC, No. CRL-2505) was cultured in 10% fetal bovine serum-containing RPMI1640 medium (+P/S).
Cell line MIA PaCa-2 (ATCC, No. CRL-1420) was cultured in 10% fetal bovine serum-containing DMEM medium (+P/S).
Cell line CCRF-CEM (ATCC, No. CCL-119) was cultured in 10% fetal bovine serum-containing RPMI1640 medium (+P/S).
Cell line HH (ATCC, No. CRL-2105) was cultured in 10% fetal bovine serum-containing RPMI1640 medium (+P/S).
Cell line OCI-AML-5 (DSMZ, No. ACC-247) was cultured in alpha-MEM medium containing 20% fetal bovine serum and 10% volume fraction of 5637 cell line adjusted medium (+P/S).
Cell line G-402 (ATCC, No. CRL-1440) was cultured in 10% fetal bovine serum-containing McCoy's 5a medium (+P/S).
Cell line HCC1806 (ATCC, No. CRL-2335) was cultured in 10% fetal bovine serum-containing RPMI1640 medium (+P/S).
Cell line BT-549 (ATCC, No. HTB-122) was cultured in RPMI1640 medium containing 10% fetal bovine serum and 0.023 IU/ml human insulin (+P/S).
Cell line OCI-AML-4 (DSMZ, No. ACC-729) was cultured in alpha-MEM medium containing 20% fetal bovine serum and 20% volume fraction of 5637 cell line adjusted medium(+P/S).;
Cell line H9 (ATCC, No. HTB-176) was cultured in 10% fetal bovine serum-containing RPMI1640 medium (+P/S).
Cell line Jurkat, Clone E6-1 (ATCC, No. TIB-152) was cultured in 10% fetal bovine serum-containing RPMI1640 medium (+P/S).
Cell line G-361 (ATCC, No. CRL-1424) was cultured in 10% fetal bovine serum-containing McCoy's 5a medium (+P/S).
Cell line U-937 (ATCC, No. CRL-1593.2) was cultured in 10% fetal bovine serum-containing RPMI1640 medium (+P/S).
Cell line SNU-398 (ATCC, No. CRL-2233) was cultured in 10% fetal bovine serum-containing RPMI1640 medium (+P/S).
Cell line NCI-H1048 (ATCC, No. CRL-5853) was cultured in 5% fetal bovine serum-containing HITES medium (+P/S).
Cell line A-375 (ATCC, No. CRL-1619) was cultured in 10% fetal bovine serum-containing DMEM medium (+P/S).
Cell line D283 Med (ATCC, No. HTB-185) was cultured in 10% fetal bovine serum-containing EMEM medium (+P/S).
Cell line GAK (JCRB, No. JCRB0180) was cultured in 20% fetal bovine serum-containing Ham's F12 medium (+P/S)
Cell line CHL-1 (ATCC, No. CRL-9446) was cultured in 10% fetal bovine serum-containing DMEM medium (+P/S).
Cell line NCI-H1155 (ATCC, No. CRL-5818) was cultured in serum-free ACL-4 medium (+P/S).
Cell line LS 180 (ATCC, No. CL-187) was cultured in 10% fetal bovine serum-containing EMEM medium (+P/S).
Cell line Daoy (ATCC, No. HTB-186) was cultured in 10% fetal bovine serum-containing EMEM medium (+P/S).
Cell line DU 145 (ATCC, No. HTB-81) was cultured in 10% fetal bovine serum-containing EMEM medium (+P/S).
Cell line AM-38 (JCRB, No. IFO50492) was cultured in EMEM medium containing 20% heat-inactivated fetal bovine serum (+P/S).
Cell line HCC70 (ATCC, No. CRL-2315) was cultured in 10% fetal bovine serum-containing RPMI1640 medium (+P/S).
Cell line PANC-1 (ATCC, No. CRL-1469) was cultured in 10% fetal bovine serum-containing DMEM medium (+P/S).
Cell line U-87 MG (ATCC, No. HTB-14) was cultured in 10% fetal bovine serum-containing EMEM medium (+P/S).
Cell line MJ (ATCC, No. CRL-8294) was cultured 20% fetal bovine serum-containing IMDM medium (+P/S).
Cell line Gp2D (ECACC, No. 95090714) was cultured in 10% fetal bovine serum-containing DMEM medium (+P/S).
Cell line SU.86.86 (ATCC, No. CRL-1837) was cultured in 10% fetal bovine serum-containing RPMI1640 medium (+P/S).
Cell line NCI-H2081 (ATCC, No. CRL-5920) was cultured in 5% fetal bovine serum-containing HITES medium (+P/S).
Cell line NCI-H1793 (ATCC, No. CRL-5896) was cultured in 5% fetal bovine serum-containing HITES medium (+P/S).
Cell line ACHN (ATCC, No. CRL-1611) was cultured in 10% fetal bovine serum-containing EMEM medium (+P/S).
Cell line U-251 MG (ECACC, No. 9063001) was cultured in EMEM medium containing 2mM glutamine, 1% NEAA, 1mM sodium pyruvate (NaP) and 10% fetal bovine serum (+P/S).
Cell line MDA-MB-231 (ATCC, No. HTB-26) was cultured in 10% fetal bovine serum-containing Leibovitz's L-15 medium (+P/S).
Cell line NCI-H196 (ATCC, No. CRL-5823) was cultured in 10% fetal bovine serum-containing RPMI1640 medium (+P/S).
Cell line PC-3 (ATCC, No. CRL-1435) was cultured in 10% fetal bovine serum-containing F-12K medium (+P/S).
Cell line OCI-M1 (DSMZ, No. ACC-529) was cultured in 20% fetal bovine serum-containing IMDM medium (+P/S).
Cell line NCI-H1651 (ATCC, No. CRL-5884) was cultured in 10% fetal bovine serum-containing ACL-4 medium (+P/S).
Cell line C3A (ATCC, No. CRL-10741) was cultured in 10% fetal bovine serum-containing EMEM medium (+P/S).
Cell line SNU-449 (ATCC, No. CRL-2234) was cultured in 10% fetal bovine serum-containing RPMI1640 medium (+P/S).
Cell line GB-1 (JCRB, No. IFO50489) was cultured in 10% fetal bovine serum-containing DMEM medium (+P/S).
Cell line 769-P (ATCC, No. CRL-1933) was cultured in 10% fetal bovine serum-containing RPMI1640 medium (+P/S).
Cell line COLO 320HSR (ATCC, No. CCL-220.1) was cultured in 10% fetal bovine serum-containing RPMI1640 medium (+P/S).
Cell line CFPAC-1 (ATCC, No. CRL-1918) was cultured in 10% fetal bovine serum-containing IMDM medium (+P/S).
Cell line SF126 (JCRB, No. IFO50286) was cultured in 10% fetal bovine serum-containing EMEM medium (+P/S).
Cell line 786-O (ATCC, No. CRL-1932) was cultured in 10% fetal bovine serum-containing RPMI1640 medium (+P/S).

### Experimental method and result:

The above tumor cells were cultured in the relevant medium (+P/S), and incubated for 3h, then the gradient diluted Gboxin was added, and IC₅₀ (50% inhibiting concentration) was measured after 3-4 days of culture.

The experiment result was shown in Table 3 below:

**Table 3 IC₅₀ of Gboxin on different tumor cell lines (µM)**

| Cell line | IC50 (µM) |
|---|---|
| NCI-H82 | 0.398 |
| G-401 | 0.777 |
| MDA-MB-453 | 0.942 |
| WSU-DLCL2 | 0.682 |
| SU-DHL-2 | 0.97 |
| OCI-AML-3 | 1.46 |
| SW48 | 1.535 |
| ATN-1 | 1.55 |
| HCC15 | 2.26 |
| OCI-LY-19 | 2.51 |
| 22RV1 | 2.53 |
| MIA PaCa-2 | 2.57 |
| CCRF-CEM | 2.59 |
| HH | 2.67 |
| OCI-AML-5 | 2.74 |
| G-402 | 2.82 |
| HCC1806 | 3.18 |
| BT-549 | 3.28 |
| OCI-AML-4 | 3.34 |
| H9 | 3.34 |
| Jurkat, Clone E6-1 | 3.51 |
| G-361 | 3.88 |
| U-937 | 4.06 |
| SNU-398 | 4.17 |
| NCI-H1048 | 4.34 |
| A-375 | 4.65 |
| D283 Med | 4.81 |
| GAK | 7.05 |
| CHL-1 | 7.68 |
| NCI-H1155 | 8.84 |
| LS 180 | 8.90 |
| Daoy | 9.72 |
| DU 145 | 9.91 |
| AM-38 | 10.29 |
| HCC70 | 11.23 |
| PANC-1 | 11.99 |
| U-87 MG | 12.31 |
| MJ | 12.90 |
| Gp2D | 13.30 |
| SU.86.86 | 13.68 |
| NCI-H2081 | 13.92 |
| NCI-H1793 | 15.99 |
| ACHN | 16.57 |
| U-251 MG | 16.68 |
| MDA-MB-231 | 17.81 |
| NCI-H196 | 19.31 |
| PC-3 | 21.26 |
| OCI-M1 | 21.65 |
| NCI-H1651 | 22.36 |
| C3A | 25.74 |
| SNU-449 | >30.00 |
| GB-1 | >30.00 |
| 769-P | >30.00 |
| COLO 320HSR | >30.00 |
| CFPAC-1 | >30.00 |
| SF126 | >30.00 |
| 786-O | >30.00 |

| | |
|---|---|
| Note: IC₅₀ refered to 50% inhibiting concentration, ie, the concentration of the inhibitor when 50% inhibitory effect was achieved. | |

The Table 3 showed the sensitivity of different cells to the Gboxin, a mitochondrial oxidative phosphorylation pathway inhibitor, NCI-H82 (human small cell lung cancer cell), G-401 (human renal carcinoma Wilms cells), MDA-MB-453 (breast cancer cell), WSU-DLCL2 (human diffuse large B lymphoma cell) and SW48 (human colon adenocarcinoma cell) were sensitive to Gboxin with low IC₅₀ value, while GB-1 (human glioblastoma cell), CFPAC-1 (human pancreatic cancer cell), SF126 (human brain tumor cell), 786-O (clear cell renal cell adenocarcinoma) were not sensitive to Gboxin with high IC₅₀ value.

### Example 3

The sensitivity of cell lines NCI-H82, G-401, MDA-MB-453, WSU-DLCL2, SW48, GB-1, CFPAC-1, SF126 and 786-O to Oligomycin A or IACS-010759, a mitochondrial oxidative phosphorylation pathway inhibitor, was further tested using cell viability detection reagent. The Example 2 showed the cell lines NCI-H82, G-401, MDA-MB-453, WSU-DLCL2 and SW48 were sensitive to Gboxin and the cell lines GB-1, CFPAC-1, SF126 and 786-O were not sensitive to Gboxin, a mitochondrial oxidative phosphorylation pathway inhibitor.

### Experimental background:

Cell viability was detected using the Promega CellTiter-Glo kit, the kit reflected cell viability by directly measuring intracellular ATP content. In this experiment, the IC₅₀ values of Oligomycin A or IACS-010759, a mitochondrial oxidative phosphorylation pathway inhibitor, on different tumor cells was determined.

### Experimental method and result:

The different tumor cells were cultured in the relevant medium (+P/S) as shown in Example 2, and incubated for 3h, then the gradient diluted Oligomycin A or IACS-010759 was added respectively, and IC₅₀ (50% inhibiting concentration) of Oligomycin A or IACS-010759 on different tumor cells was measured after 3-4 days of culture.

The experiment result was shown in Table 4 below:

**Table 4 the inhibitory effect of of Oligomycin A or IACS-010759 on different tumor cell lines (IC50)**

| Sensitivity | Cell line | Oligomycin A IC₅₀(µM) | IACS-010759 IC₅₀(µM) |
|---|---|---|---|
| Sensitive group | NCI-H82 | 0.013 | 0.168 |
| | G-401 | 0.014 | 1.476 |
| | MDA-MB-453 | 0.014 | <0.01 |
| | SW48 | 0.014 | <0.01 |
| | WSU-DLCL2 | 0.647 | 3.56 |
| Insensitive group | CFPAC-1 | 15.096 | 55.67 |
| | 786-O | 13.642 | >50 |
| | GB-1 | 25.609 | >15 |
| | SF126 | 12.028 | >15 |

| | | | |
|---|---|---|---|
| Note: IC₅₀ refered to 50% inhibiting concentration, ie, the concentration of the inhibitor when 50% inhibitory effect was achieved. | | | |

The Table 4 showed the cell lines NCI-H82, G-401, MDA-MB-453, WSU-DLCL2 and SW48 sensitive to Gboxin was also sensitive to Oligomycin A or IACS-010759 with low IC₅₀ value, and the cell lines GB-1, CFPAC-1, SF126 and 786-O insensitive to Gboxin was also not sensitive to Oligomycin A or IACS-010759 with high IC₅₀ value.

### Example 4

The change of activity of ATF4 (Activating Transcription Factor 4) and mTOR (rapamycin target protein) pathways after the action of the mitochondrial oxidative phosphorylation pathway inhibitors such as small molecules Gboxin and Oligomycin A, etc., on the cell lines (NCI-H82, G-401 and WSU-DLCL2) sensitive to mitochondrial oxidative phosphorylation pathway inhibitors.

### Experimental background:

The inhibition of mitochondrial oxidative phosphorylation pathway could cause the activation of ATF4 stress pathway and the decrease of mTOR pathway activity, the activity of the mTOR pathway was reflected by the expression level of phosphorylated ribosomal protein S6 (ie, p-S6). The increased expression of ATF4 indicated the activation of ATF4 stress pathway, while the decreased expression of p-S6 protein indicated the activity of mTOR pathway was inhibited.

### Experimental method and result:

The tumor cells NCI-H82, G-401 and WSU-DLCL2 were cultured in the relevant 10% FBS-containing medium (+p/s), and incubated overnight, then 1 µ M Gboxin, 3 µ M Gboxin or 1 µ M Oligomycin A as shown in Fig.2 was added. After 12 h of incubation, the content of ATF4 and p-S6 protein was detected using Western Blot, the experiment result was shown in Fig.2.

The Fig.2 showed after the action of the mitochondrial oxidative phosphorylation pathway inhibitors such as small molecules Gboxin and Oligomycin A, etc., on the cell lines NCI-H82, G-401 and WSU-DLCL2, the ATF4 stress pathway was up-regulated, while the content of p-S6 protein decreased, the activity of mTOR pathway was inhibited. Thus the results indicated the oxidative phosphorylation pathway was active in cell lines NCI-H82, G-401 and WSU-DLCL2, and the NCI-H82, G-401 and WSU-DLCL2 cell lines were sensitive to oxidative phosphorylation pathway inhibitors.

### Example 5

The change of activity of ATF4 and mTOR pathways after the action of the mitochondrial oxidative phosphorylation pathway inhibitors such as small molecules Gboxin and Oligomycin A, etc., on the cell lines (SF126, CFPAC-1 and 786-O) insensitive to mitochondrial oxidative phosphorylation pathway inhibitors was detected using Western Blot test.

### Experimental background:

The inhibition of mitochondrial oxidative phosphorylation pathway could cause the activation of ATF4 stress pathway and the decrease of mTOR pathway activity, the activity of the mTOR pathway was reflected by the expression level of phosphorylated ribosomal protein S6 (ie, p-S6). The increased expression of ATF4 indicated the activation of ATF4 stress pathway, while the decreased expression of p-S6 protein indicated the activity of mTOR pathway was inhibited.

### Experimental method and result:

The cell lines SF126, CFPAC-1 and 786-O were cultured in the relevant 10% FBS-containing medium (+p/s), and incubated overnight, then 1 µ M Gboxin, 3 µ M Gboxin or 1 µ M Oligomycin A as shown in Fig.3 was added. After 12 h of incubation, the content of ATF4 and p-S6 protein was detected using Western Blot, the experiment result was shown in Fig.3.

The Fig.3 showed after the action of the mitochondrial oxidative phosphorylation pathway inhibitors such as small molecules Gboxin and Oligomycin A, etc., on the cell lines SF 126, CFPAC-1 and 786-O, the activity of ATF4 stress pathway and mTOR pathway had no significant changes, indicating the cell lines SF126, CFPAC-1 and 786-O were not sensitive to oxidative phosphorylation pathway inhibitors.

### Example 6

The gene transcription expression of cell lines (NCI-H82, G-401, MDA-MB-453, SW48 and WSU-DLCL2) sensitive to mitochondrial oxidative phosphorylation pathway inhibitors and cell lines (786-O, CFPAC-1, GB-1 and SF126) insensitive to mitochondrial oxidative phosphorylation pathway inhibitors was detected using bioinformatics.

### Experimental background:

The behavior and characteristics of cell were determined by the expressed genes. The mRNA transcription level of each gene in a cell could be accurately detected using the whole-genome gene transcription sequencing. Bioinformatics calculation and analysis of the mRNA transcription level of all gene could classify different cells based on the approximate degree of gene expression.

### Experimental method and result:

The whole genome mRNA transcriptional level of cell lines (NCI-H82, G-401, MDA-MB-453, SW48 and WSU-DLCL2) sensitive to mitochondrial oxidative phosphorylation pathway inhibitors and cell lines (786-O, CFPAC-1, GB-1 and SF126) insensitive to mitochondrial oxidative phosphorylation pathway inhibitors was detected, and then the similarity of mRNA transcriptome in each cell was calculated and analyzed using bioinformatics.

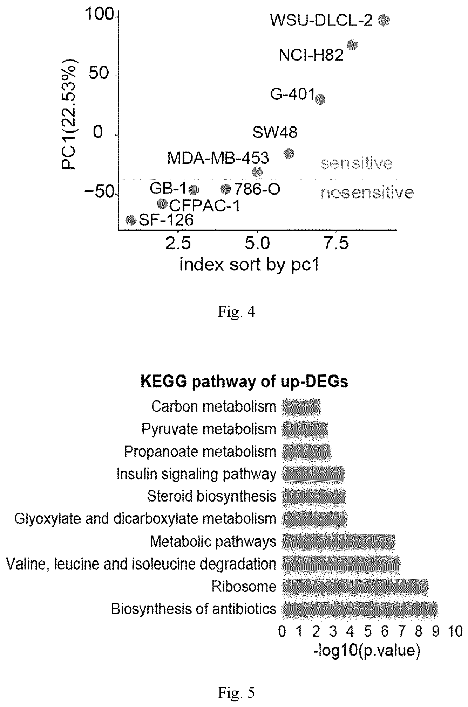

The experiment result was shown in Fig.4, wherein "pc" referred to principal component, and "pc1" referred to the degree of difference among cells by integrating the expression information of each gene.

The Fig.4 showed the cell lines (NCI-H82, G-401, MDA-MB-453, SW48 and WSU-DLCL2) sensitive to mitochondrial oxidative phosphorylation pathway inhibitors and cell lines (786-O, CFPAC-1, GB-1 and SF126) insensitive to mitochondrial oxidative phosphorylation pathway inhibitors were significantly different in gene transcription level.

### Example 7

The function of differentially expressed gene between cell lines (NCI-H82, G-401, MDA-MB-453, SW48 and WSU-DLCL2) sensitive to mitochondrial oxidative phosphorylation pathway inhibitors and cell lines (786-O, CFPAC-1, GB-1 and SF126) insensitive to mitochondrial oxidative phosphorylation pathway inhibitors was detected using bioinformatics.

### Experimental background:

The behavior and characteristics of cell were determined by the expressed genes, the differentially expressed gene among multiple cells often determined the different characteristics of these cells. The different characteristics of the cells could be obtained using bioinformatics calculation and analysis of the mRNA transcription level of differentially expressed gene among multiple cells.

### Experimental method and result:

The differentially expressed genes between cell lines (NCI-H82, G-401, MDA-MB-453, SW48 and WSU-DLCL2) sensitive to mitochondrial oxidative phosphorylation pathway inhibitors and cell lines (786-O, CFPAC-1, GB-1 and SF126) insensitive to mitochondrial oxidative phosphorylation pathway inhibitors were obtained using bioinformatics, then the function of the differentially expressed genes was analyzed to obtain the functional differences between the two groups of cells. The experiment result was shown in Fig.5.

The Fig.5 showed the differentially expressed up-regulated genes between cell lines (NCI-H82, G-401, MDA-MB-453, SW48 and WSU-DLCL2) sensitive to mitochondrial oxidative phosphorylation pathway inhibitors and cell lines (786-O, CFPAC-1, GB-1 and SF126) insensitive to mitochondrial oxidative phosphorylation pathway inhibitors were mainly in metabolism-realted pathways (e.g., carbon metabolism, pyruvate metabolism, propionic acid metabolism, glyoxylic acid and dicarboxylic acid metabolism, etc.,), indicating that there were significant differences in metabolism between the two groups of cells, and relevant metabolic pathways were up-regulated in sensitive cells.

### Example 8

The main differences in metabolic pathways between cell lines (NCI-H82, G-401, MDA-MB-453, SW48 and WSU-DLCL2) sensitive to mitochondrial oxidative phosphorylation pathway inhibitors and cell lines (786-O, CFPAC-1, GB-1 and SF126) insensitive to mitochondrial oxidative phosphorylation pathway inhibitors was detected using bioinformatics.

### Experimental background:

The Example 7 showed there were significant differences in metabolism-related pathways between cell lines (NCI-H82, G-401, MDA-MB-453, SW48 and WSU-DLCL2) sensitive to mitochondrial oxidative phosphorylation pathway inhibitors and cell lines (786-O, CFPAC-1, GB-1 and SF126) insensitive to mitochondrial oxidative phosphorylation pathway inhibitors. Cell metabolism was determined by the activity of multiple metabolic pathways (gene expression). A deep analysis of the different metabolic pathways between the two groups of cells could provide a deeper understanding of the differences in metabolism between the two groups of cells.

### Experimental method and result:

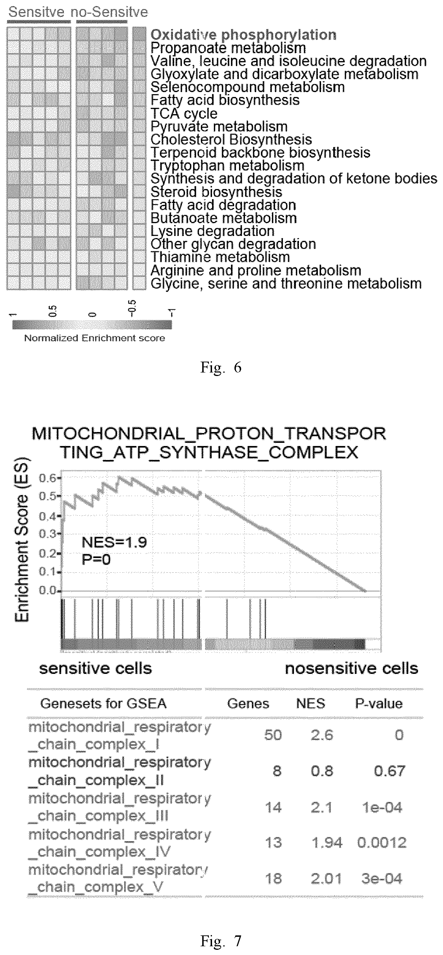

The differentially expressed genes were obtained between cell lines (NCI-H82, G-401, MDA-MB-453, SW48 and WSU-DLCL2) sensitive to mitochondrial oxidative phosphorylation pathway inhibitors and cell lines (786-O, CFPAC-1, GB-1 and SF126) insensitive to mitochondrial oxidative phosphorylation pathway inhibitors were obtained using bioinformatics. The differentially expressed genes involved in cell metabolism were selected to analyze the metabolic pathways in which these genes involved. The experiment result was shown in Fig.6.

The Fig.6 showed the most significant metabolic pathway of differential expression was mitochondrial oxidative phosphorylation pathway between cell lines (NCI-H82, G-401, MDA-MB-453, SW48 and WSU-DLCL2) sensitive to mitochondrial oxidative phosphorylation pathway inhibitors and cell lines (786-O, CFPAC-1, GB-1 and SF126) insensitive to mitochondrial oxidative phosphorylation pathway inhibitors, the metabolic pathways were up-regulated in sensitive cells.

### Example 9

The differences in protein complexes of oxidative phosphorylation pathway between cell lines (NCI-H82, G-401, MDA-MB-453, SW48 and WSU-DLCL2) sensitive to mitochondrial oxidative phosphorylation pathway inhibitors and cell lines (786-O, CFPAC-1, GB-1 and SF126) insensitive to mitochondrial oxidative phosphorylation pathway inhibitors was detected using bioinformatics.

### Experimental background:

The Example 8 showed there were significant differences in mitochondrial oxidative phosphorylation pathway between cell lines (NCI-H82, G-401, MDA-MB-453, SW48 and WSU-DLCL2) sensitive to mitochondrial oxidative phosphorylation pathway inhibitors and cell lines (786-O, CFPAC-1, GB-1 and SF126) insensitive to mitochondrial oxidative phosphorylation pathway inhibitors. The oxidative phosphorylation pathway was composed of five protein complexes containing more than 90 proteins.

### Experimental method and result:

The differentially expressed genes were obtained between cell lines (NCI-H82, G-401, MDA-MB-453, SW48 and WSU-DLCL2) sensitive to mitochondrial oxidative phosphorylation pathway inhibitors and cell lines (786-O, CFPAC-1, GB-1 and SF126) insensitive to mitochondrial oxidative phosphorylation pathway inhibitors were obtained using bioinformatics. The differentially expressed genes related to oxidative phosphorylation pathway protein complexes were analyzed, and the protein complexes of oxidative phosphorylation pathway expressed by these genes were obtained. The experiment result was shown in Fig.7.

The Fig.7 showed the differentially expressed genes in the oxidative phosphorylation pathway between cell lines (NCI-H82, G-401, MDA-MB-453, SW48 and WSU-DLCL2) sensitive to mitochondrial oxidative phosphorylation pathway inhibitors and cell lines (786-O, CFPAC-1, GB-1 and SF126) insensitive to mitochondrial oxidative phosphorylation pathway inhibitors were mainly in protein complexes I, III, IV, and V of oxidative phosphorylation pathway, these proteins were highly expressed in sensitive cells.

### Example 10

The membrane potential difference between cell lines (NCI-H82, G-401 and WSU-DLCL2) sensitive to mitochondrial oxidative phosphorylation pathway inhibitors and cell lines (786-O, CFPAC-1 and SF126) insensitive to mitochondrial oxidative phosphorylation pathway inhibitors were analyzed using mitochondrial membrane potential difference indicator.

### Experimental background:

Mitochondrial membrane potential difference could regulate various important mitochondrial functions such as mitochondrial protein transport, autophagy, and ATP synthesis. There were significant differences in the oxidative phosphorylation pathway between cell lines (NCI-H82, G-401 and WSU-DLCL2) sensitive to mitochondrial oxidative phosphorylation pathway inhibitors and cell lines (786-O, CFPAC-1and SF126) insensitive to mitochondrial oxidative phosphorylation pathway inhibitors, the differences also were reflected in mitochondrial membrane potential difference.

### Experimental method and result:

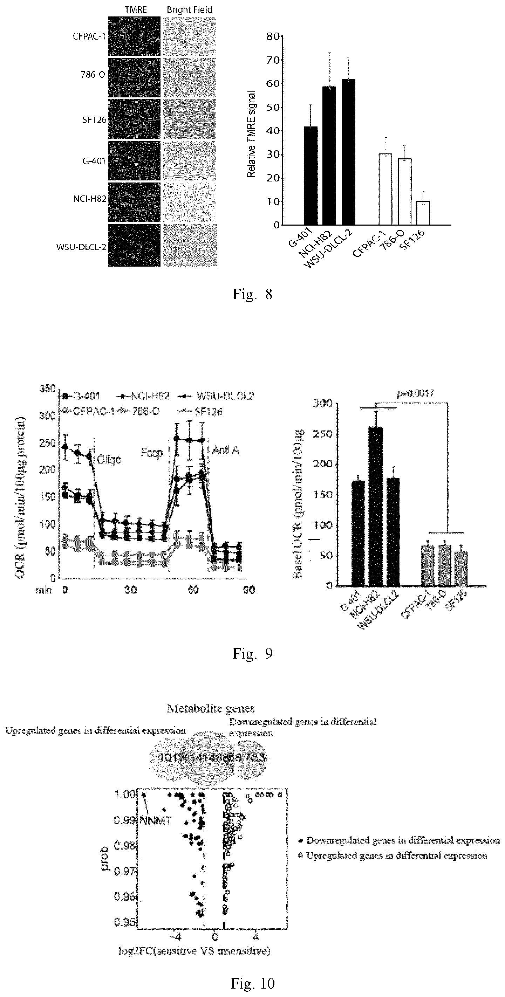

The membrane potential difference in cell lines (NCI-H82, G-401 and WSU-DLCL2) sensitive to mitochondrial oxidative phosphorylation pathway inhibitors and cell lines (786-O, CFPAC-1and SF126) insensitive to mitochondrial oxidative phosphorylation pathway inhibitors was detected using mitochondrial membrane potential difference indicator TMRE (tetramethylrhodamine, ethyl ester), the cells were cultured in normal state. The experiment result was shown in Fig.8.

The Fig.8 showed the membrane potential difference in cells (NCI-H82, G-401 and WSU-DLCL2) sensitive to mitochondrial oxidative phosphorylation pathway inhibitors was relatively high, the membrane potential difference in cells (786-O, CFPAC-1and SF126) insensitive to mitochondrial oxidative phosphorylation pathway inhibitors was relatively low, there were significant differences in the mitochondrial membrane potential difference between the two groups of cells.

### Example 11

The differences in oxygen consumption in cell lines (NCI-H82, G-401 and WSU-DLCL2) sensitive to mitochondrial oxidative phosphorylation pathway inhibitors and cell lines (786-O, CFPAC-1 and SF126) insensitive to mitochondrial oxidative phosphorylation pathway inhibitors were analyzed using Seahorse Cell Metabolism Analyzer.

### Experimental background:

More than 90% of the oxygen needed by cells was consumed by mitochondrial oxidative phosphorylation pathway, so the activity of oxidative phosphorylation pathway was closely related to the oxygen consumption level in cells. There were significant differences in the expression of oxidative phosphorylation pathway proteins in cell lines (NCI-H82, G-401 and WSU-DLCL2) sensitive to mitochondrial oxidative phosphorylation pathway inhibitors and cell lines (786-O, CFPAC-1 and SF126) insensitive to mitochondrial oxidative phosphorylation pathway inhibitors. Related proteins were highly expressed in sensitive cells, and these differences could also be reflected in oxygen consumption.

### Experimental method and result:

The oxygen consumption rate (OCR) in cell lines (NCI-H82, G-401 and WSU-DLCL2) sensitive to mitochondrial oxidative phosphorylation pathway inhibitors and cell lines (786-O, CFPAC-1 and SF126) insensitive to mitochondrial oxidative phosphorylation pathway inhibitors were analyzed according to the standard process of Seahorse Cell Metabolism Analyzer, the cells were cultured in normal state. The experiment result was shown in Fig.9.

The Fig.9 showed the oxygen consumption level in cell lines (NCI-H82, G-401 and WSU-DLCL2) sensitive to mitochondrial oxidative phosphorylation pathway inhibitors was significantly higher than that in cell lines (786-O, CFPAC-1 and SF126) insensitive to mitochondrial oxidative phosphorylation pathway inhibitors.

### Example 12

The genes with significant differences in expression in cell lines (NCI-H82, G-401, MDA-MB-453, SW48 and WSU-DLCL2) sensitive to mitochondrial oxidative phosphorylation pathway inhibitors and cell lines (786-O, CFPAC-1, GB-1 and SF126) insensitive to mitochondrial oxidative phosphorylation pathway inhibitors were screened using transcriptome and bioinformatics.

### Experimental background:

Precise therapy was the direction of modern drug research and development, and biomarkers provided excellent technical support for achieving precise therapy. A good biomarker should have the ability to clearly distinguish drug response and non-response. Most biomarkers were the expression of a certain gene or a group of genes.

### Experimental method and result:

Transcriptome sequencing was performed on cell lines (NCI-H82, G-401, MDA-MB-453, SW48 and WSU-DLCL2) sensitive to mitochondrial oxidative phosphorylation pathway inhibitors and cell lines (786-O, CFPAC-1, GB-1 and SF126) insensitive to mitochondrial oxidative phosphorylation pathway inhibitors. The genes with significant differences in expression in the two groups of cells were screen using bioinformatics. The experiment result was shown in Fig.10.

The Fig.10 showed there were significant differences in NNMT gene expression in cell lines (NCI-H82, G-401, MDA-MB-453, SW48 and WSU-DLCL2) sensitive to mitochondrial oxidative phosphorylation pathway inhibitors and cell lines (786-O, CFPAC-1, GB-1 and SF126) insensitive to mitochondrial oxidative phosphorylation pathway inhibitors. The expression of NNMT gene was low in cell sensitive to oxidative phosphorylation pathway inhibitors.

### Example 13

The feasibility of NNMT gene transcription level as a biomarker for determining the sensitivity to mitochondrial oxidative phosphorylation pathway inhibitors was studied in the cell level.

### Experimental background:

Precise therapy was the direction of modern drug research and development, and biomarkers provided excellent technical support for achieving precise therapy. A good biomarker should have the ability to clearly distinguish drug response and non-response, and have a positive or negative correlation with the corresponding biological characteristics.

### Experimental method and result:

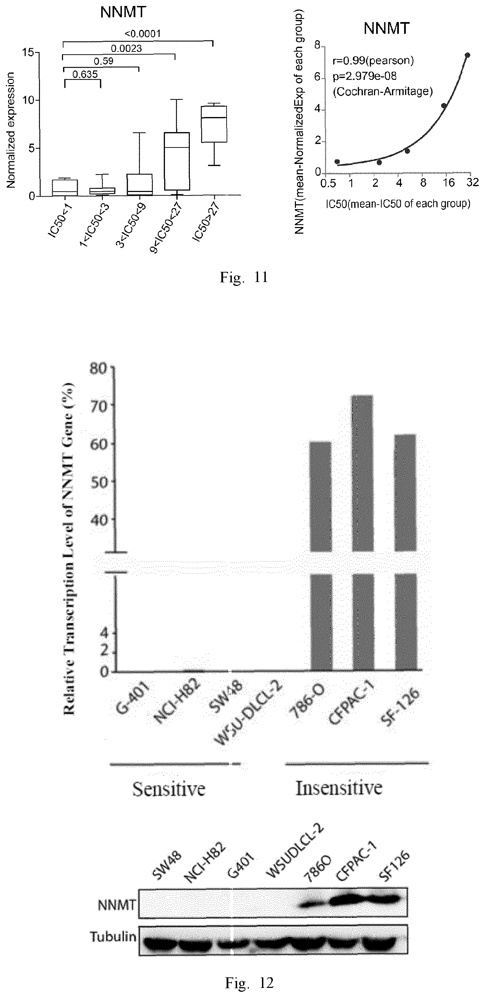

According to the sensitivity of fifty-seven tumor cells from different tissue sources and genotypes to Gboxin, a mitochondrial oxidative phosphorylation pathway inhibitor, in Example 2, the cells were divided into five groups according to the different IC₅₀ values as follows: Group 1: IC₅₀<1 µM; Group 2: 1 µM<IC₅₀<3 µM; Group 3: 3 µM <IC₅₀<9 µM; Group 4: 9 µM <IC₅₀<27µM; Group 5: 27 µM<IC₅₀. The mean transcription level of NNMT gene of all tumor cells in each group was measured, and the correlation between the transcription level of NNMT gene of tumor cells and the sensitivity of tumor cells to oxidative phosphorylation inhibitors was analyzed. The relationship between the inhibitory effect (IC₅₀) of Gboxin, a oxidative phosphorylation inhibitor, on tumor cells and the transcription level of NNMT gene was shown in Fig.11.

The Fig.11 showed the transcription level of NNMT gene was exponentially negative correlation with the sensitivity of tumor cells to Gboxin in each cell, the smaller the IC₅₀ was, the more sensitive of tumor cell to Gboxin was, indicating that the transcription level of NNMT gene of tumor cells was negative correlation with the sensitivity of tumor cells to mitochondrial oxidative phosphorylation pathway inhibitors, that is, the lower the transcription level of NNMT gene of the cell was, the higher the sensitivity of the tumor cell to mitochondrial oxidative phosphorylation pathway inhibitors was.

### Example 14

The NNMT gene in cells sensitive to mitochondrial oxidative phosphorylation pathway inhibitors and cells insensitive to mitochondrial oxidative phosphorylation pathway inhibitors was validated in terms of mRNA and protein expression levels.

### Experimental background:

Genes in cells usually performed their functions at the protein level. The experiment detected the mRNA and protein level of the NNMT gene.

### Experiment method and result:

The mRNA and protein of NNMT in tumor cell lines sensitive and insensitive to mitochondrial oxidative phosphorylation pathway inhibitors was measured using RT-qPCR and Western Blot. The experiment result was shown in Fig. 12.

The Fig.12 showed the mRNA and protein of the NNMT gene were lowly expressed in cells (NCI-H82, G-401, SW48, and WSU-DLCL2) sensitive to mitochondrial oxidative phosphorylation pathway inhibitors, while the mRNA and protein of the NNMT gene were highly expressed in cells (786-O, CFPAC-1 and SF126) insensitive to mitochondrial oxidative phosphorylation pathway inhibitors.

### Example 15

### Experiment method and result:

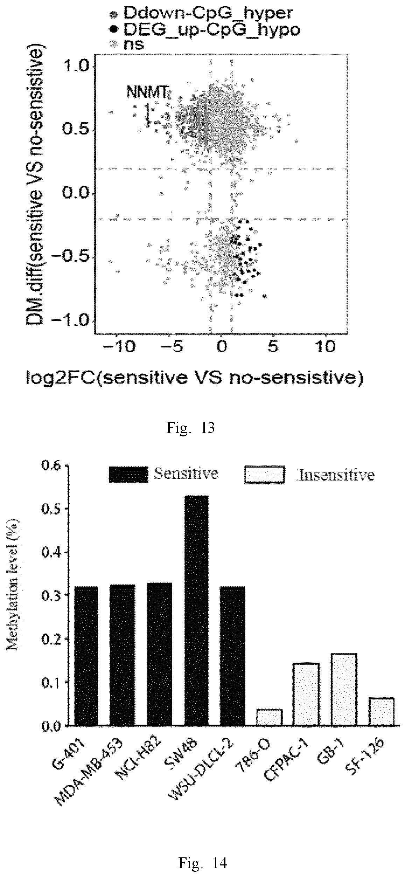

Whole genome transcription sequencing and DNA methylation sequencing were performed on cell lines (NCI-H82, G-401, MDA-MB-453, SW48 and WSU-DLCL2) sensitive to mitochondrial oxidative phosphorylation pathway inhibitors and cell lines (786-O, CFPAC-1 GB-1 and SF126) insensitive to mitochondrial oxidative phosphorylation pathway inhibitors, and the genes (Ddown CpG_super, upper left portion in Fig. 13) with low expression but high promoter methylation and the genes (DEG_up CpG_hydropo, lower right portion in Fig. 13) with high expression but low promoter methylation in cells sensitive to mitochondrial oxidative phosphorylation pathway inhibitors was identified using bioinformatics compared with that in cells insensitive to mitochondrial oxidative phosphorylation pathway inhibitors.

The experiment result was shown in Fig. 13, wherein the X-axis represented the ratio of the transcription expression level of a gene in the sensitive cells to the transcription expression level of the gene in the insensitive cells; the Y-axis represented the ratio of the methylation level of CpG in the promoter region of a gene of sensitive cells to the methylation level of CpG in the promoter region of a gene of insensitive cells.

The Fig.13 showed the promoter region of NNMT gene was highly methylated and lowly expressed in cell lines (NCI-H82, G-401, MDA-MB-453, SW48 and WSU-DLCL2) sensitive to mitochondrial oxidative phosphorylation pathway inhibitors, while the promoter region of NNMT gene was lowly methylated and highly expressed in cell lines (786-O, CFPAC-1, GB-1, and SF126) insensitive to mitochondrial oxidative phosphorylation pathway inhibitors.

### Example 16

The promoter region of NNMT gene, the region from 1050 bp before the transcription start site to 499 bp after the transcription start site in NNMT gene and the region from 1050 bp to 193 bp before the transcription start site in NNMT gene were subjected to bisulfite sequencing to measure methylation level of DNA CpG site in five tumor cell lines (NCI-H82, G-401, MDA-MB-453, SW48, and WSU-DLCL2) sensitive to mitochondrial oxidative phosphorylation pathway inhibitors such as Oligomycin A and Gboxin and four tumor cell lines (786-O, CFPAC-1, GB-1, and SF126) insensitive to mitochondrial oxidative phosphorylation pathway inhibitors such as Oligomycin A and Gboxin. Firstly, genomic DNA was subjected to bisulfite, unmethylated cytosine was deamined to form uracil, and methylated cytosine could not be deamined, so the methylation sites could be determined by comparing the sequencing samples treated with bisulfite to the sequencing samples treated without bisulfite, and the result was shown in Fig.14, Fig.15, and Fig.16.

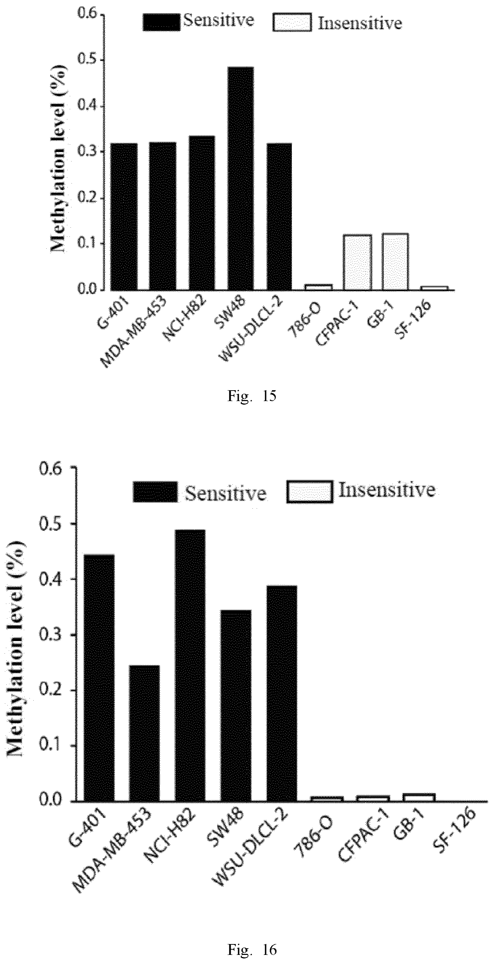

As shown in Fig.14 (the promoter region of NNMT gene), Fig.15 (the region from 1050 bp before the transcription start site to 499 bp after the transcription start site in NNMT gene) and Fig.16 (the region from 1050 bp to 193 bp before the transcription start site in NNMT gene), the mitochondrial oxidative phosphorylation pathway inhibitors had significantly stronger inhibitory effect on tumor cells with high methylation level of DNA CpG site in the promoter region of NNMT gene, the region from 1050 bp before the transcription start site to 499 bp after the transcription start site in NNMT gene and the region from 1050 bp to 193 bp before the transcription start site in NNMT gene, the mitochondrial oxidative phosphorylation pathway inhibitors had significantly weaker inhibitory effect on tumor cells with low methylation level of DNA CpG site in the promoter region of NNMT gene, the region from 1050 bp before the transcription start site to 499 bp after the transcription start site in NNMT gene and the region from 1050 bp to 193 bp before the transcription start site in NNMT gene, indicating the methylation level of DNA CpG site in the promoter region of NNMT gene, the region from 1050 bp before the transcription start site to 499 bp after the transcription start site in NNMT gene and the region from 1050 bp to 193 bp before the transcription start site in NNMT gene was positive correlation with the sensitivity of tumor cells to to mitochondrial oxygen phosphorylation pathway inhibitors.

### Example 17

The methylation level of specific DNA CpG sites from 840 bp (i.e., position 114165695 on human chromosome 11) to 469 bp (i.e., position 114166066 on human chromosome 11) before the transcription start site in NNMT gene in four tumor cell lines (NCI-H82, G-401, SW48, and WSU-DLCL2) sensitive to mitochondrial oxidative phosphorylation pathway inhibitors and three tumor cell lines (786-O, CFPAC-1, and SF126) insensitive to mitochondrial oxidative phosphorylation pathway inhibitors was studied.

Firstly, genomic DNA was subjected to bisulfite, unmethylated cytosine was deamined to form uracil, and methylated cytosine could not be deamined, so the methylation sites could be determined by comparing the sequencing samples treated with bisulfite with the sequencing samples treated without bisulfite, then PCR amplification and sequencing analysis were performed on the region using corresponding primers to measure the methylation level of CpG site in the DNA region.

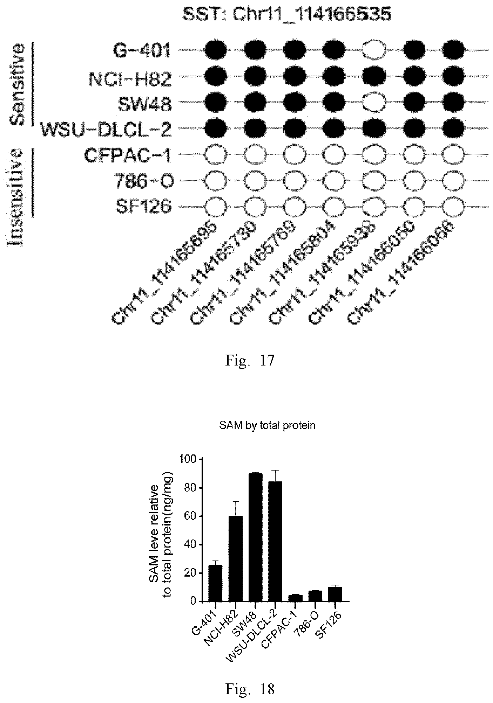

The study showed that almost all of the seven CpG sites (site 114165695, site 114165730, site 114165769, site 114165804, site 114165938, site 114166050 and site 114166066 on the human chromosome 11) in cell lines (G-401, SW48, NCI-H82, and WSU-DLCL2) sensitive to mitochondrial oxidative phosphorylation pathway inhibitors were methylated, while none of the above seven CpG sites in cell lines (CFPAC-1, 786-O and SF126) insensitive to mitochondrial oxidative phosphorylation pathway inhibitors were methylated, the methylation of related sites was shown in Fig.17.

The sites of the nucleotide sequence of SEQ ID NO: 1 corresponding to the site 114165695, site 114165730, site 114165769, site 114165804, site 114165938, site 114166050 and site 114166066 on the human chromosome 11 were as follows:

| The site on the human chromosome 11 | Corresponding to the sites of the nucleotide sequence of SEQ ID NO: 1 |
|---|---|
| site 114165695 | site 1161 |
| site 114165730 | site 1196 |
| site 114165769 | site 1235 |
| site 114165804 | site 1270 |
| site 114165938 | site 1404 |
| site 114166050 | site 1516 |
| site 114166066 | site 1532 |

### Example 18

The level of methylation donor S-adenosylmethionine (SAM) in cell lines sensitive and insensitiveo mitochondrial oxidative phosphorylation pathway inhibitors was detected using enzyme linked immunosorbent assay.

### Experiment method and result:

The level of methylation donor S-adenosylmethionine (SAM) in cell lines (NCI-H82, G-401, SW48, and WSU-DLCL2) sensitive to mitochondrial oxidative phosphorylation pathway inhibitors and cell lines (786-O, CFPAC-1, and SF126) insensitive to mitochondrial oxidative phosphorylation pathway inhibitors was detected using enzyme linked immunosorbent assay, the experiment result was shown in Fig.18.

The Fig.18 showed the level of methylation donor SAM in cell lines (NCI-H82, G-401, SW48 and WSU-DLCL2) sensitive to mitochondrial oxidative phosphorylation pathway inhibitors was significantly higher than that in cell lines (786-O, CFPAC-1 and SF126) insensitive to mitochondrial oxidative phosphorylation pathway inhibitors.

### Example 19

The methylation level of DNA in cell was maintained by DNA methylation enzymes DNMT3a, DNMT3b and DNMT1. The original methylation of DNA was performed with DNMT3a and DNMT3b, DNMT1 could replicate and maintain methylated DNA with the help of protein UHRF1 (ubiquitin-like with PHD and ring finger domain 1). The correlation between the expression of NNMT and the expression of DNMT1, UHRF1, DNMT3a and DNMT3b in tumors was determined in the Example.

### Experiment method and result:

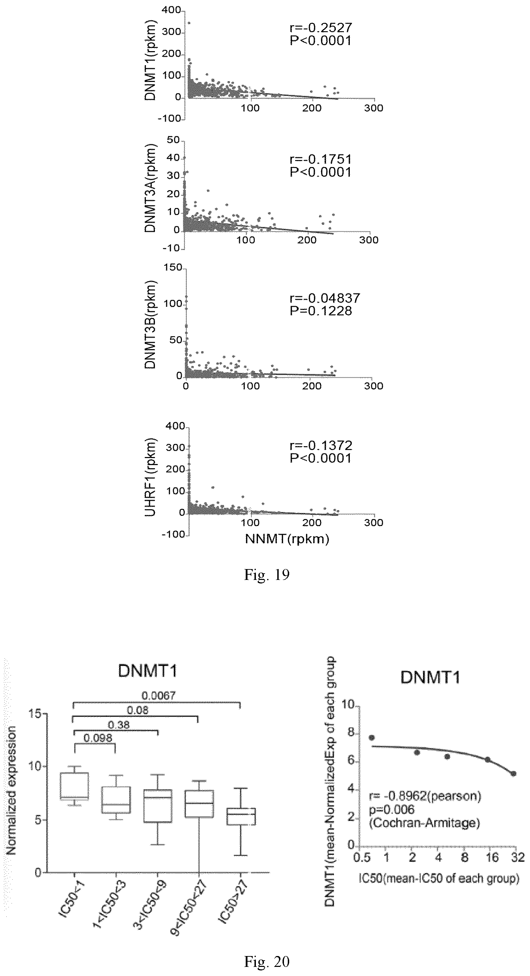

The expression of NNMT gene, DNMT1, UHRF1, DNMT3a, and DNMT3b in various cells were obtained from a public database (Cancer Cell Line Encyclopedia, CCLE, 1019 cells in total). Then, the correlation between the expression of NNMT and the expression of DNMT1, UHRF1, DNMT3a and DNMT3b in these cells was analyzed using bioinformatics, and the correlation between the expression level of NNMT gene and the expression level of DNMT1, UHRF1, DNMT3a and DNMT3b in each cell was analyzed, the experiment result was shown in Fig.19.

The Fig.19 showed the expression of NNMT was negative correlation with the expression of DNA methylase and UHRF1 in each cell.

### Example 20

The transcription level of DNMT1 (DNA Methyltransferase 1) gene as a biomarker for determining sensitivity of cell to mitochondrial oxidative phosphorylation pathways inhibitors was investigated at cell level .

### Experiment method and result:

According to the sensitivity of fifty-seven tumor cells from different tissue sources and genotypes to Gboxin, a mitochondrial oxidative phosphorylation pathway inhibitor, in Example 2, the cells were divided into five groups according to the different IC₅₀ values as follows: Group 1: IC₅₀<1 µM; Group 2: 1 µM<IC₅₀<3 µM; Group 3: 3 µM <IC₅₀<9 µM; Group 4: 9 µM <IC₅₀<27µM; Group 5: 27 µM<IC₅₀.

The mean transcriptional mRNA level of DNMT1 gene of all tumor cells in each group was measured, and the correlation between the transcription level of DNMT1 gene of each tumor cell and the sensitivity of the tumor cells to oxidative phosphorylation inhibitors was analyzed, the experiment result was shown in Fig.20.

The Fig.20 showed the transcription level of DNMT1 gene was exponentially positive correlation with the sensitivity of the cells to Gboxin, the smaller the IC₅₀ was, the more sensitive of tumor cell to Gboxin was, indicating the transcription level of DNMT1 gene was positive correlation with the sensitivity of related cells to mitochondrial oxidative phosphorylation pathway inhibitors, i.e, the higher the transcription level of DNMT1 gene in tumor cells was, the higher the sensitivity of the tumor cell to mitochondrial oxidative phosphorylation pathway inhibitors was.

### Example 21

The expression level of NNMT in cells was significantly negative correlation with the sensitivity of the cell to oxidative phosphorylation pathway inhibitors, while the expression level of DNMT1 gene was significantly positive correlation with the sensitivity of cell to oxidative phosphorylation pathway inhibitors. The role of NNMT and DNMT1 in the sensitivity of cells to oxidative phosphorylation pathway inhibitors was further determined.

### Experiment method and result:

The NCI-H82 cell overexpressing NNMT protein was obtained by inserting the NNMT gene into NCI-H82 cell using a viral vector. The expression of DNMT1 in NCI-H82 cells was knocked down using shRNA transfection. The changes in sensitivity of cells to mitochondrial oxidative phosphorylation pathway inhibitors (Gboxin, Oligomycin A) was investigated using intracellular ATP detection methods after overexpression of NNMT protein and/or knockdown of DNMT1 expression. The experiment result was shown in Fig.21 and Fig.22, wherein, "Vector" referred to NCI-H82 cell transfected with empty virus as control group.

The Fig.21 and Fig.22 showed after the NNMT protein of NCI-H82 cells sensitive to oxidative phosphorylation pathway inhibitors was overexpressed alone (ov-NNMT as shown in Figs. ) or the DNMT1 expression of NCI-H82 cells was knocked down using two different shRNA targeting the DNMT1 gene respectively (sh-DNMT1 # 1 referred to a shRNA targeting the DNMT1 gene, and the DNA sequence of sh-DNMT1 # 1 was GATCCGGCCCAATGAGACTGACATCAATT CAAGAGATTGATGTCAGTCTCATTGGGCTTTTTG (SEQ ID No: 2); the sh-DNMT1 # 2 was another shRNA targeting the DNMT1 gene, and the DNA sequence of sh-DNMT1 # 2 was GATCCGGGATGAGTCCATCAAGGAAGATTCAAGAGATCTTCCTTGATGGACTCATCCT TTTTTG (SEQ ID No: 3), the sensitivity of NCI-H82 cell to mitochondrial oxidative phosphorylation pathway inhibitors such as Gboxin and Oligomycin A decreased. After the NNMT protein was overexpressed and the expression of DNMT1 was knocked down in NCI-H82 cell simultaneously (ov-NNMT/sh-DNMT1#1 and ov-NNMT/sh-DNMT1#2 as shown in the Figs), the sensitivity of NCI-H82 tumor cells to mitochondrial inhibitors such as Gboxin and Oligomycin A decreased more significantly.

Compared to normal NCI-H82 (Vector), the NNMT protein content of NCI-H82 overexpressing NNMT protein (ov-NNMT) was detected using Western Blot assay, the result was shown in Fig.23. The Western Blot assay was used to detect the DNMT1 protein content of NCI-H82 with the knockdown of DNMT1 expression using two shRNA ((sh-DNMT1 # or sh-DNMT1 #2) compared to normal NCI-H82 (shVector), the result was shown in Fig. 24.

Therefore, the Example with the overexpression of NNMT protein and knockdown of DNMT1 in NCI-H82 cell further confirmed the level of NNMT expression in tumor cell was significantly negative correlation with the sensitivity of the tumor cell to oxidative phosphorylation pathway inhibitors, while the expression level of DNMT1 in tumor cells was significantly positive correlation with the sensitivity of the tumor cell to oxidative phosphorylation pathway inhibitors.

### Example 22

To verify whether cells sensitive to mitochondrial oxidative phosphorylation pathway inhibitors still maintained the sensitivity to oxidative phosphorylation pathway inhibitors in vivo, S-Gboxin was used to test the effectiveness in tumor-bearing mice subcutaneously inoculated with sensitive cells (NCI-H82) and insensitive cells (CFPAC-1).

### Experiment method and result:

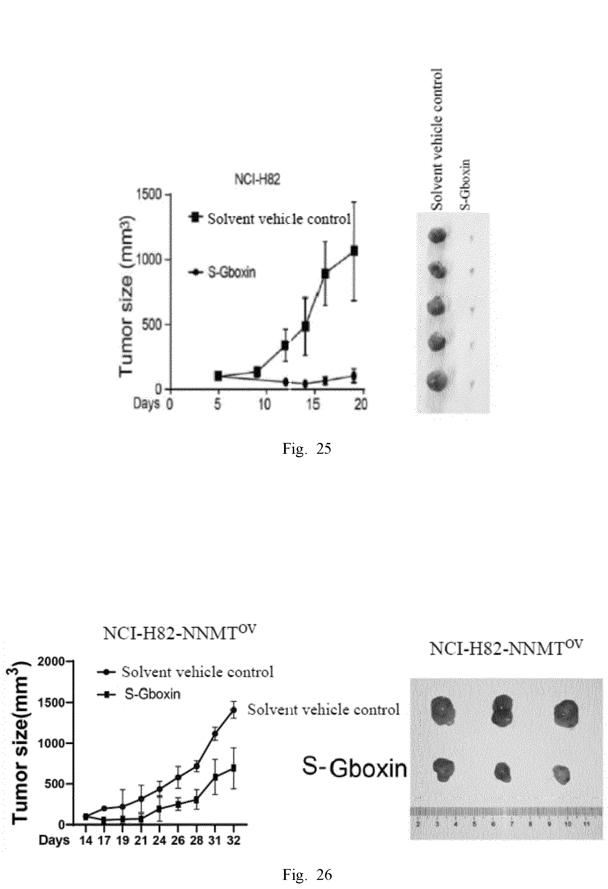

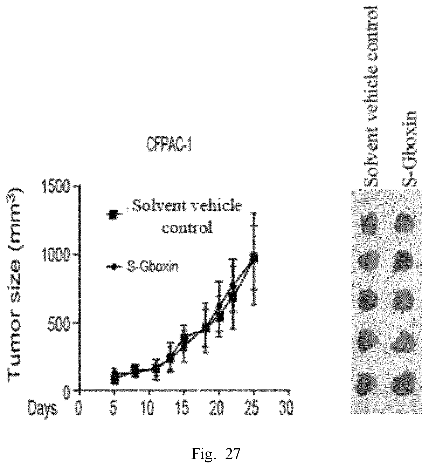

5x10⁶ of NCI-H82, NCI-H82-NNMT^{ov} (inserting the NNMT gene into NCI-H82 cell using a viral vector to overexpress NNMT protein) and CFPAC-1 tumors were subcutaneously inoculated in nude mice to establish tumor-bearing mice. Each group of tumor-bearing mice was injected intraperitoneally with the S-Gboxin (a mitochondrial oxidative phosphorylation pathway inhibition) at a dose of 10 mg/kg/day, the inhibition effect of S-Gboxin on the tumor was investigated. The mice in control group was injected intraperitoneally with solvent vehicle in the same method, and the tumor volume was calculated as follows: tumor volume=1/2 length x width ². The experiment result was shown in Fig.25, Fig.26, and Fig.27.

As shown in Fig.25, Fig.26, and Fig.27, the compound S-Gboxin could significantly inhibit the subcutaneous tumor of nude mice inoculated with sensitive cell NCI-H82, while the inhibitory effect of the compound S-Gboxin on the subcutaneous tumor of nude mice inoculated with NCI-H82-NNMT^{ov} was significantly weaker than that of the compound S-Gboxin on the subcutaneous tumor of nude mice inoculated with NCI-H82, and S-Gboxin had no significant inhibitory effect on the subcutaneous tumor of nude mice inoculated with insensitive cells CFPAC-1. The results suggested that the oxidative phosphorylation pathway inhibitors had a stronger inhibitory effect on tumor with low NNMT expression, ie, tumor with low NNMT expression was more sensitive to oxidative phosphorylation pathway inhibitors, while tumor with high NNMT expression was less sensitive to the oxidative phosphorylation pathway inhibitor.

It should be understood that those skilled in the art will be able to make various changes or modifications to the present invention after reading the teachings of the present invention, which also fall within the scope of the claims appended hereto.

## Claims

1. A mitochondrial oxidative phosphorylation pathway inhibitor for use in the prevention and/or treatment of a tumor;
wherein, the tumor comprises tumor with low or no expression of NNMT gene; and/or
the tumor comprises tumor with high expression of DNA methylase; and/or
the tumor comprises tumor with high expression of UHRFl; and/or
the tumor comprises tumor with high methylation level of nucleotide site of NNMT gene; and/or
the tumor comprises tumor with high methylation level of DNA CpG site of NNMT gene;
wherein, the low or no expression of NNMT gene means the ratio (E1/E0) of the expression level E1 of NNMT gene in the tumor cell with respect to the expression level E0 of NNMT gene in the same type of tumor cell with normal expression of NNMT gene is <1.0;
the tumor with high expression of DNA methylase means the ratio (A1/A0) of the expression level A1 of DNA methylase in the tumor cell with respect to the expression level A0 of DNA methylase in the same type of tumor cell with normal expression of DNA methylase is > 1.0;
the tumor with high expression of UHRF1 means the ratio (F1/F0) of the expression level F1 of UHRF1 in the tumor cell with respect to the expression level F0 of UHRF1 in the same type of tumor cell with normal expression of UHRF1 is > 1.0;
the high methylation level of nucleotide site of NNMT gene means the ratio (L1/L0) of the methylation level L1 of nucleotide site of NNMT gene in the tumor cell with respect to the methylation level L0 of nucleotide site of NNMT gene in the same type of tumor cell with normal methylation level of nucleotide site of NNMT gene is > 1.0;
the high methylation level of DNA CpG site of NNMT gene means the ratio (W1/W0) of the methylation level W1 of DNA CpG site of NNMT gene in the tumor cell with respect to the methylation level W0 of DNA CpG site of NNMT gene in the same type of tumor cell with normal methylation level of DNA CpG site of NNMT gene is > 1.0;
wherein, the mitochondrial oxidative phosphorylation pathway inhibitor is a compound of formula I-2, or an optical isomer thereof, or a racemate thereof, or a solvate thereof, or a pharmaceutically acceptable salt thereof; 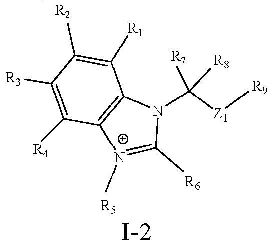
R₁, R₂, R₃, R₄, R₇ and R₈ are each independently hydrogen;
R₅ is C1-C6 alkyl;
R₆ is C1-C6 alkyl, substituted or unsubstituted C6-C8 aryl, or substituted or unsubstituted 5-8 membered heteroaryl, wherein, each "substituted" means that 1, 2, or 3 hydrogen atoms on the group are substituted by a substituent selected from the group consisting of C1-C4 haloalkyl;
R₉ is substituted or unsubstituted C5-C8 cycloalkyl, wherein, the "substituted" means that 1, 2, or 3 hydrogen atoms on the group are substituted by a substituent selected from the group consisting of C1-C6 alkyl;
Z₁ is 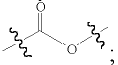
or, the mitochondrial oxidative phosphorylation pathway inhibitor is a compound of formula II, or an optical isomer thereof, or a racemate thereof, or a solvate thereof, or a pharmaceutically acceptable salt thereof; 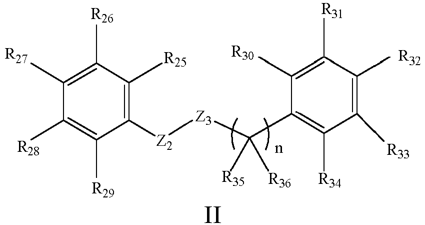
R₂₅, R₂₆, R₂₇, R₂₈ and R₂₉ are each independently hydrogen, or C1-C6 haloalkoxyl;
R₃₀, R₃₁, R₃₂, R₃₄, R₃₅ and R₃₆ are each independently hydrogen;
R₃₃ is substituted or unsubstituted 3-8 membered heterocycloalkyl, wherein, the "substituted" means that 1, 2, or 3 hydrogen atoms on the group are substituted by a substituent selected from the group consisting of methylsulfonyl, sulfonyl;
Z₂ and Z₃ are each independently substituted or unsubstituted C6-C8 arylene, or substituted or unsubstituted 3-8 membered heteroarylene, wherein, each "substituted" means that 1, 2, or 3 hydrogen atoms on the group are substituted by a substituent selected from the group consisting of C1-C4 alkyl;
n is 1;
or, the mitochondrial oxidative phosphorylation pathway inhibitor is a compound of formula III, or an optical isomer thereof, or a racemate thereof, or a solvate thereof, or a pharmaceutically acceptable salt thereof; 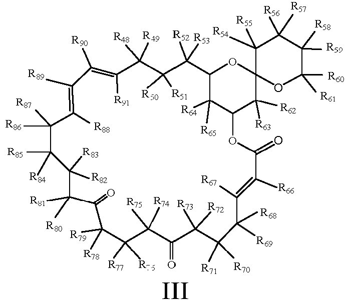
R₄₈, R₅₀, R₅₁, R₅₂, R₅₃, R₅₄, R₅₅, R₅₆, R₅₇, R₅₈, R₆₀, R₆₂, R₆₄, R₆₆, R₆₇, R₆₈, R₇₀, R₇₂, R₇₄, R₇₆, R₇₈, R₈₂, R₈₄, R₈₆, R₈₇, R₈₈, R₈₉, R₉₀ and R₉₁ are each independently hydrogen;
R₄₉ is C1-C6 alkyl;
R₅₉, R₆₃, R₆₅, R₆₉, R₇₃, R₇₅, R₇₉, R₈₀ and R₈₅ are each independently C1-C6 alkyl;
R₆₁ is hydroxyl-(C1-C6 alkyl)-;
R₇₁, R₇₇, R₈₁ and R₈₃ are each independently hydroxyl.

2. A mitochondrial oxidative phosphorylation pathway inhibitor for use according to claim 1, the mitochondrial oxidative phosphorylation pathway inhibitor is a compound of formula I-2, or an optical isomer thereof, or a racemate thereof, or a solvate thereof, or a pharmaceutically acceptable salt thereof; 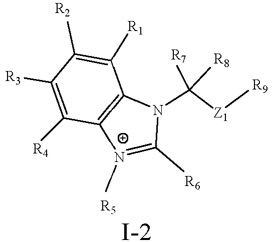
R₁, R₂, R₃, R₄, R₇ and R₈ are each independently hydrogen;
R₅ is hydrogen, methyl, ethyl, propyl, or butyl;
R₆ is hydrogen, methyl, ethyl, propyl, butyl, or 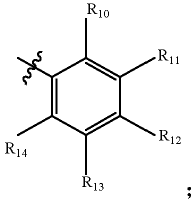
R9 is 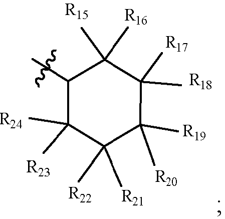
R₁₀, R₁₁, R₁₂, R₁₃ and R₁₄ are each independently hydrogen, or C1-C4 haloalkyl;
R₁₅, R₁₆, R₁₇, R₁₈, R₁₉, R₂₀, R₂₁, R₂₂, R₂₃ and R₂₄ are each independently hydrogen, or C1-C6 alkyl;
Z₁ is 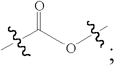
or, the mitochondrial oxidative phosphorylation pathway inhibitor is a compound of formula II, or an optical isomer thereof, or a racemate thereof, or a solvate thereof, or a pharmaceutically acceptable salt thereof; 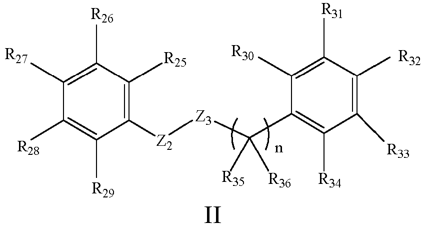
R₂₅, R₂₆, R₂₈, R₂₉, R₃₀, R₃₁, R₃₂, R₃₄, R₃₅ and R₃₆ are each independently hydrogen;
R₂₇ is C1-C4 haloalkoxyl;
R₃₃ is substituted or unsubstituted hexahydropyridyl, wherein, the "substituted" means that 1, 2, or 3 hydrogen atoms on the group are substituted by a substituent selected from the group consisting of methylsulfonyl, sulfonyl;
Z₂ and Z₃ are each independently substituted or unsubstituted oxadiazolylene, or substituted or unsubstituted triazolylene, wherein, each "substituted" means that one hydrogen atom on the group is substituted by a substituent selected from the group consisting of C1-C4 alkyl;
n is 1;
or, the mitochondrial oxidative phosphorylation pathway inhibitor is a compound of formula III, or an optical isomer thereof, or a racemate thereof, or a solvate thereof, or a pharmaceutically acceptable salt thereof; 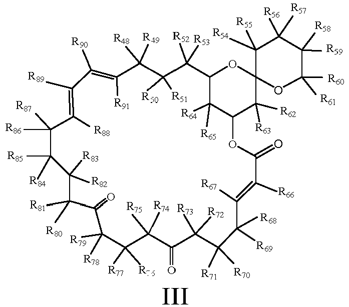
R₄₈, R₅₀, R₅₁, R₅₂, R₅₃, R₅₄, R₅₅, R₅₆, R₅₇, R₅₈, R₆₀, R₆₂, R₆₄, R₆₆, R₆₇, R₆₈, R₇₀, R₇₂, R₇₄, R₇₆, R₇₈, R₈₂, R₈₄, R₈₆, R₈₇, R₈₈, R₈₉, R₉₀ and R₉₁ are each independently hydrogen;
R₄₉ is methyl, ethyl, propyl, or butyl;
R₅₉, R₆₃, R₆₅, R₆₉, R₇₃, R₇₅, R₇₉, R₈₀ and R₈₅ are each independently methyl, ethyl, propyl, or butyl;
R₆₁ is hydroxyl-(C1-C4 alkyl)-;
R₇₁, R₇₇, R₈₁ and R₈₃ are each independently hydroxyl.

3. A mitochondrial oxidative phosphorylation pathway inhibitor for use according to claim 1, the mitochondrial oxidative phosphorylation pathway inhibitor is a compound of formula I-2, or an optical isomer thereof, or a racemate thereof, or a solvate thereof, or a pharmaceutically acceptable salt thereof; 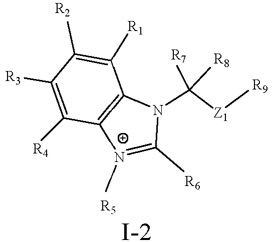
R₁, R₂, R₃, R₄, R₇ and R₈ are each independently hydrogen;
R₅ is methyl;
R₆ is propyl, or 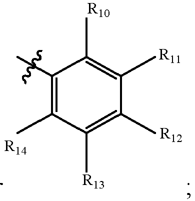
R₉ is 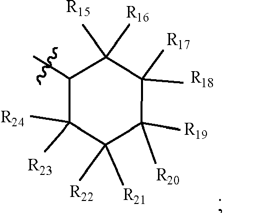
R₁₀, R₁₁, R₁₂ and R₁₄ are each independently hydrogen;
R₁₃ is trifluoromethyl;
R₁₅, R₁₇, R₁₈, R₁₉, R₂₀, R₂₁, R₂₃ and R₂₄ are each independently hydrogen;
R₁₆ is propyl;
R₂₂ is methyl;
Z₁ is 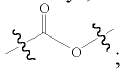
or, the mitochondrial oxidative phosphorylation pathway inhibitor is a compound of formula II, or an optical isomer thereof, or a racemate thereof, or a solvate thereof, or a pharmaceutically acceptable salt thereof; 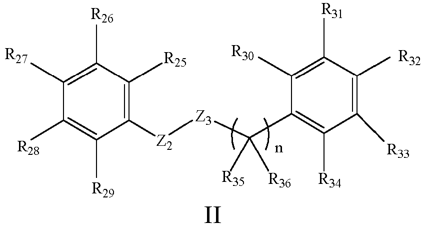
R₂₅, R₂₆, R₂₈, R₂₉, R₃₀, R₃₁, R₃₂, R₃₄, R₃₅ and R₃₆ are each independently hydrogen;
R₂₇ is trifluoromethyl-O-;
R₃₃ is 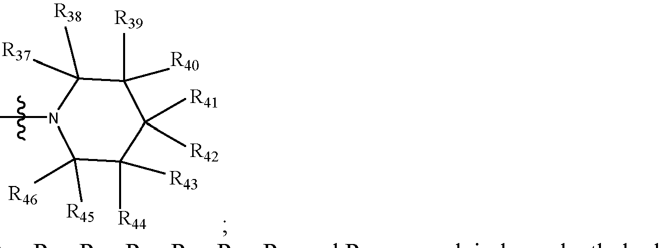
R₃₇, R₃₈, R₃₉, R₄₀, R₄₁, R₄₃, R₄₄, R₄₅ and R₄₆ are each independently hydrogen;
R₄₂ is methylsulfonyl;
Z₂ is 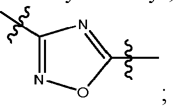
Z₃ is 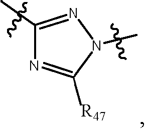 R₄₇ is methyl;
n is 1;
or, the mitochondrial oxidative phosphorylation pathway inhibitor is a compound of formula III, or an optical isomer thereof, or a racemate thereof, or a solvate thereof, or a pharmaceutically acceptable salt thereof; 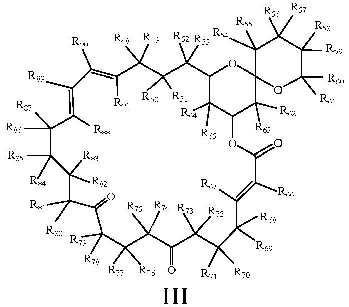
R₄₈, R₅₀, R₅₁, R₅₂, R₅₃, R₅₄, R₅₅, R₅₆, R₅₇, R₅₈, R₆₀, R₆₂, R₆₄, R₆₆, R₆₇, R₆₈, R₇₀, R₇₂, R₇₄, R₇₆, R₇₈, R₈₂, R₈₄, R₈₆, R₈₇, R₈₈, R₈₉, R₉₀ and R₉₁ are each independently hydrogen;
R₄₉ is ethyl;
R₅₉, R₆₃, R₆₅, R₆₉, R₇₃, R₇₅, R₇₉, R₈₀ and R₈₅ are each independently methyl;
R₆₁ is hydroxy-propyl-;
R₇₁, R₇₇, R₈₁ and R₈₃ are each independently hydroxyl.

4. A mitochondrial oxidative phosphorylation pathway inhibitor for use according to claim 1, wherein the tumor is human tumor;
the NNMT gene is human NNMT gene;
the expression comprises protein expression and/or mRNA expression; and/or
the DNA methylase is selected from the group consisting of DNMT1, DNMT3a, DNMT3b, and combinations thereof.

5. A mitochondrial oxidative phosphorylation pathway inhibitor for use according to claim 1, wherein the low or no expression of NNMT gene means the ratio (E1/E0) of the expression level E1 of NNMT gene in the tumor cell to the expression level E0 of NNMT gene in the same type of cell is ≤ 0.7;
the tumor with high expression of DNA methylase means the ratio (A1/A0) of the expression level A1 of DNA methylase in the tumor cell to the expression level A0 of DNA methylase in the same type of cell is ≥ 1.2;
the tumor with high expression of UHRF1 means the ratio (F1/F0) of the expression level F1 of UHRF1 in the tumor cell to the expression level F0 of UHRF1 in the same type of cell is ≥ 1.2;
the high methylation level of nucleotide site of NNMT gene means the ratio (L1/L0) of the methylation level L1 of nucleotide site of NNMT gene in the tumor cell to the methylation level L0 of nucleotide site of NNMT gene in the same type of cell is ≥ 1.2; and/or
the high methylation level of DNA CpG site of NNMT gene means the ratio (W1/W0) of the methylation level W1 of DNA CpG site of NNMT gene in the tumor cell to the methylation level W0 of DNA CpG site of NNMT gene in the same type of cell is ≥ 1.2.

6. A mitochondrial oxidative phosphorylation pathway inhibitor for use according to claim 1, wherein the low or no expression of NNMT gene means the ratio (E1/E0) of the expression level E1 of NNMT gene in the tumor cell to the expression level E0 of NNMT gene in the same type of cell is ≤ 0.5;
the tumor with high expression of DNA methylase means the ratio (A1/A0) of the expression level A1 of DNA methylase in the tumor cell to the expression level A0 of DNA methylase in the same type of cell is ≥ 2;
the tumor with high expression of UHRF1 means the ratio (F1/F0) of the expression level F1 of UHRF1 in the tumor cell to the expression level F0 of UHRF1 in the same type of cell is ≥ 2;
the high methylation level of nucleotide site of NNMT gene means the ratio (L1/L0) of the methylation level L1 of nucleotide site of NNMT gene in the tumor cell to the methylation level L0 of nucleotide site of NNMT gene in the same type of cell is ≥ 2; and/or
the high methylation level of DNA CpG site of NNMT gene means the ratio (W1/W0) of the methylation level W1 of DNA CpG site of NNMT gene in the tumor cell to the methylation level W0 of DNA CpG site of NNMT gene in the same type of cell is ≥ 2.

7. A mitochondrial oxidative phosphorylation pathway inhibitor for use according to claim 1, wherein the low or no expression of NNMT gene means the ratio (E1/E0) of the expression level E1 of NNMT gene in the tumor cell to the expression level E0 of NNMT gene in the same type of cell is ≤ 0.3;
the tumor with high expression of DNA methylase means the ratio (A1/A0) of the expression level A1 of DNA methylase in the tumor cell to the expression level A0 of DNA methylase in the same type of cell is ≥ 5;
the tumor with high expression of UHRF1 means the ratio (F1/F0) of the expression level F1 of UHRF1 in the tumor cell to the expression level F0 of UHRF1 in the same type of cell is ≥ 5;
the high methylation level of nucleotide site of NNMT gene means the ratio (L1/L0) of the methylation level L1 of nucleotide site of NNMT gene in the tumor cell to the methylation level L0 of nucleotide site of NNMT gene in the same type of cell is ≥ 5; and/or
the high methylation level of DNA CpG site of NNMT gene means the ratio (W1/W0) of the methylation level W1 of DNA CpG site of NNMT gene in the tumor cell to the methylation level W0 of DNA CpG site of NNMT gene in the same type of cell is ≥ 5.

8. A mitochondrial oxidative phosphorylation pathway inhibitor for use according to claim 1, wherein the low or no expression of NNMT gene means the ratio (E1/E0) of the expression level E1 of NNMT gene in the tumor cell to the expression level E0 of NNMT gene in the same type of cell is ≤ 0.1;
the tumor with high expression of DNA methylase means the ratio (A1/A0) of the expression level A1 of DNA methylase in the tumor cell to the expression level A0 of DNA methylase in the same type of cell is ≥ 10;
the tumor with high expression of UHRF1 means the ratio (F1/F0) of the expression level F1 of UHRF1 in the tumor cell to the expression level F0 of UHRF1 in the same type of cell is ≥ 10;
the high methylation level of nucleotide site of NNMT gene means the ratio (L1/L0) of the methylation level L1 of nucleotide site of NNMT gene in the tumor cell to the methylation level L0 of nucleotide site of NNMT gene in the same type of cell is ≥ 10; and/or
the high methylation level of DNA CpG site of NNMT gene means the ratio (W1/W0) of the methylation level W1 of DNA CpG site of NNMT gene in the tumor cell to the methylation level W0 of DNA CpG site of NNMT gene in the same type of cell is ≥ 10.

9. A mitochondrial oxidative phosphorylation pathway inhibitor for use according to claim 1, wherein the high methylation level of nucleotide site of NNMT gene means the methylation level (M%) of nucleotide site of NNMT gene in the tumor cell is ≥ 3% and ≤ M1%, wherein M1 is any positive integer from 3 to 100;
the methylation level of nucleotide site of NNMT gene refers to the ratio of the number of methylated nucleotides to the number of all nucleotides in the NNMT gene;
the methylation level of nucleotide site of NNMT gene comprises the methylation level of nucleotide site in promoter region of NNMT gene;
the methylation level of nucleotide site of NNMT gene comprises the methylation level of the nucleotide sites from 1050 bp before the transcription start site to 499 bp after the transcription start site in NNMT gene;
the methylation level of nucleotide site of NNMT gene comprises the methylation level of the nucleotide sites from 1050 bp to 193 bp before the transcription start site in NNMT gene;
the methylation level of nucleotide site of NNMT gene comprises the methylation level of the nucleotide sites from 840 bp to 469 bp before the transcription start site in NNMT gene;
the methylation level of nucleotide site of NNMT gene comprises the methylation level of nucleotide sites between any two sites (including the two sites) selected from group consisting of site 114165695, site 114165730, site 114165769, site 114165804, site 114165938, site 114166050 and site 114166066 on human chromosome 11;
the methylation level of nucleotide site of NNMT gene comprises the methylation level of nucleotide sites selected from group consisting of site 114165695 on human chromosome 11, site 114165730 on human chromosome 11, site 114165769 on human chromosome 11, site 114165804 on human chromosome 11, site 114165938 on human chromosome 11, site 114166050 on human chromosome 11, site 114166066 on human chromosome 11, and combinations thereof;
the methylation level of nucleotide site of NNMT gene comprises the methylation level of nucleotide sites between any two sites (including the two sites) selected from group consisting of site 1161, site 1196, site 1235, site 1270, site 1404, site 1516 and site 1532 in nucleotide sequence of SEQ ID NO: 1;
the methylation level of nucleotide site of NNMT gene comprises the methylation level of nucleotide sites selected from group consisting of site 1161 in SEQ ID NO: 1, site 1196 in SEQ ID NO: 1, site 1235 in SEQ ID NO: 1, site 1270 in SEQ ID NO: 1, site 1404 in SEQ ID NO: 1, site 1516 in SEQ ID NO: 1, site 1532 in SEQ ID NO: 1, and combinations thereof;
the high methylation level of DNA CpG site of NNMT gene means the methylation level (M%) of DNA CpG site of NNMT gene in the tumor cell is ≥ 3% and ≤ M2%, wherein M2 is any positive integer from 3 to 100;
the methylation level of DNA CpG site of NNMT gene refers to the ratio of the number of methylated CpG nucleotides to the number of all nucleotides in the NNMT gene;
the methylation level of DNA CpG site of NNMT gene refers to the ratio of the number of methylated CpG nucleotides to the number of all CpG nucleotides in the NNMT gene;
the methylation level of DNA CpG site of NNMT gene comprises the methylation level of DNA CpG site in promoter region of NNMT gene;
the methylation level of DNA CpG site of NNMT gene comprises the methylation level of the DNA CpG sites from 1050 bp before the transcription start site to 499 bp after the transcription start site in NNMT gene;
the methylation level of DNA CpG site of NNMT gene comprises the methylation level of the DNA CpG sites from 1050 bp to 193 bp before the transcription start site in NNMT gene;
the methylation level of DNA CpG site of NNMT gene comprises the methylation level of the DNA CpG sites from 840 bp to 469 bp before the transcription start site in NNMT gene;
the methylation level of DNA CpG site of NNMT gene comprises the methylation level of DNA CpG sites between any two sites (including the two sites) selected from group consisting of site 114165695, site 114165730, site 114165769, site 114165804, site 114165938, site 114166050 and site 114166066 on human chromosome 11;
the methylation level of DNA CpG site of NNMT gene comprises the methylation level of nucleotide sites selected from group consisting of site 114165695 on human chromosome 11, site 114165730 on human chromosome 11, site 114165769 on human chromosome 11, site 114165804 on human chromosome 11, site 114165938 on human chromosome 11, site 114166050 on human chromosome 11, site 114166066 on human chromosome 11, and combinations thereof;
the methylation level of DNA CpG site of NNMT gene comprises the methylation level of nucleotide sites between any two sites (including the two sites) selected from group consisting of site 1161, site 1196, site 1235, site 1270, site 1404, site 1516 and site 1532 in nucleotide sequence of SEQ ID NO: 1; and/or
the methylation level of DNA CpG site of NNMT gene comprises the methylation level of nucleotide sites selected from group consisting of site 1161 in SEQ ID NO: 1, site 1196 in SEQ ID NO: 1, site 1235 in SEQ ID NO: 1, site 1270 in SEQ ID NO: 1, site 1404 in SEQ ID NO: 1, site 1516 in SEQ ID NO: 1, site 1532 in SEQ ID NO: 1, and combinations thereof.

10. A mitochondrial oxidative phosphorylation pathway inhibitor for use according to claim 9, wherein M1 is 5, 10, 15, 20, 25, 30, 35, 40, 45, 50, 55, 60, 65, 70, 80, 85, 90, 95 or 100;
M2 is 5, 10, 15, 20, 25, 30, 35, 40, 45, 50, 55, 60, 65, 70, 80, 85, 90, 95 or 100;
the sites from 1050 bp before the transcription start site to 499 bp after the transcription start site in NNMT gene is sites 951-2500 of nucleotide sequence as shown in SEQ ID NO: 1;
the sites from 1050 bp to 193 bp before the transcription start site in NNMT gene is sites 951-1808 of nucleotide sequence as shown in SEQ ID NO: 1; and/or
the sites from 840 bp to 469 bp before the transcription start site in NNMT gene is sites 1161-1532 of nucleotide sequence as shown in SEQ ID NO: 1.

11. A mitochondrial oxidative phosphorylation pathway inhibitor for use according to claim 1, wherein the tumor is selected from the group consisting of lung cancer, renal carcinoma, breast cancer, colon cancer, rectal cancer, colorectal cancer, lymphoma, leukemia, pancreatic cancer, brain tumor, liver cancer, prostate cancer, melanoma, and combinations thereof.

12. A mitochondrial oxidative phosphorylation pathway inhibitor for use according to claim 11, wherein the lung cancer is selected from the group consisting of non-small cell lung cancer, small cell lung cancer, metastatic lung cancer, and combinations thereof;
the colon cancer comprises colon adenocarcinoma;
the rectal cancer comprises rectal adenocarcinoma;
the colorectal cancer comprises colorectal adenocarcinoma;
the lymphoma is selected from the group consisting of B-cell lymphoma, T-cell lymphoma, skin T-cell lymphoma, large cell lymphoma, histiocytic lymphoma, and combinations thereof;
the lymphoma comprises diffuse large B-cell lymphoma;
the brain tumor is selected from the group consisting of glioblastoma, neuroglioma, and combination thereof;
the brain tumor comprises brain medulloblastoma;
the brain tumor comprises glioblastoma multiforme;
the renal carcinoma is selected from the group consisting of clear cell renal cell adenocarcinoma, metastatic renal carcinoma, and combination thereof;
the leukemia is selected from the group consisting of T-lymphocyte leukemia, myeloid leukemia, and combinations thereof;
the prostate cancer is selected from the group consisting of metastatic prostate cancer;
the breast cancer is selected from the group consisting of breast ductal carcinoma, metastatic breast cancer, and combinations thereof; and/or
the pancreatic cancer comprises liver-metastatic pancreatic cancer.

13. A mitochondrial oxidative phosphorylation pathway inhibitor for use according to claim 1, the mitochondrial oxidative phosphorylation pathway inhibitor is selected from the following group:

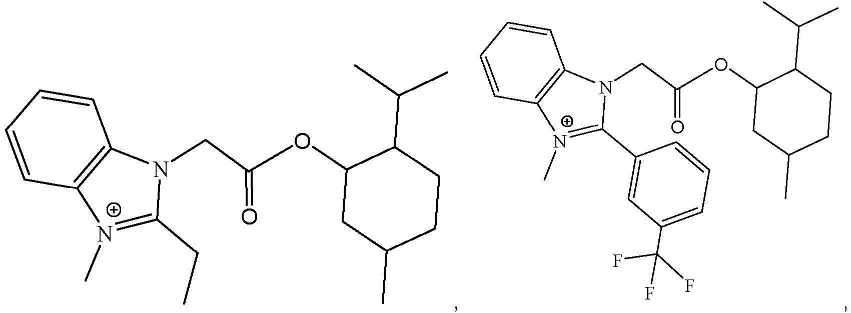

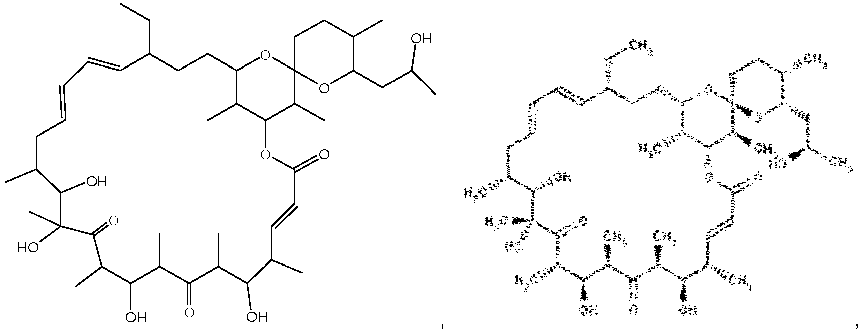

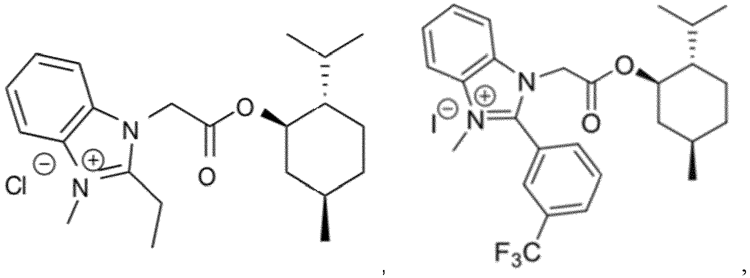

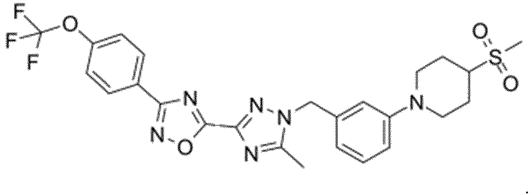

## Patentansprüche

1. Verwendung eines Inhibitors des mitochondrialen oxidativen Phosphorylierungswegs zur Vorbeugung und/oder Behandlung eines Tumors;
wobei der Tumor einen Tumor mit niedriger oder keiner Expression des NNMT-Gens umfasst; und/oder
der Tumor einen Tumor mit hoher Expression von DNA-Methylase umfasst; und/oder
der Tumor einen Tumor mit hoher Expression von UHRF1 umfasst; und/oder
der Tumor einen Tumor mit hohem Methylierungsgrad der Nukleotidstelle des NNMT-Gens umfasst; und/oder
der Tumor einen Tumor mit hohem Methylierungsgrad der DNA-CpG-Stelle des NNMT-Gens umfasst;
wobei die niedrige oder keine Expression des NNMT-Gens bedeutet, dass das Verhältnis (E1/E0) des Expressionsniveaus E1 des NNMT-Gens in der Tumorzelle im Vergleich zum Expressionsniveau E0 des NNMT-Gens in Tumorzell desselben Typs mit normaler Expression des NNMT-Gens < 1,0 ist;
der Tumor mit hoher Expression von DNA-Methylase bedeutet, dass das Verhältnis (A1/A0) des Expressionsniveaus A1 der DNA-Methylase in der Tumorzelle im Vergleich zum Expressionsniveau A0 der DNA-Methylase in Tumorzell desselben Typs mit normaler Expression der DNA-Methylase > 1,0 ist;
der Tumor mit hoher Expression von UHRF1 bedeutet, dass das Verhältnis (F1/F0) des Expressionsniveaus F1 von UHRF1 in der Tumorzelle im Vergleich zum Expressionsniveau F0 von UHRF1 in Tumorzell desselben Typs mit normaler Expression von UHRF1 > 1,0 ist;
der hohe Methylierungsgrad der Nukleotidstelle des NNMT-Gens bedeutet, dass das Verhältnis (L1/L0) des Methylierungsgrads L1 der Nukleotidstelle des NNMT-Gens in der Tumorzelle im Vergleich zum Methylierungsgrad L0 der Nukleotidstelle des NNMT-Gens in Tumorzell desselben Typs mit normalem Methylierungsgrad > 1,0 ist;
der hohe Methylierungsgrad der DNA-CpG-Stelle des NNMT-Gens bedeutet, dass das Verhältnis (W1/W0) des Methylierungsgrads W1 der DNA-CpG-Stelle des NNMT-Gens in der Tumorzelle im Vergleich zum Methylierungsgrad W0 der DNA-CpG-Stelle des NNMT-Gens in Tumorzell desselben Typs mit normalem Methylierungsgrad > 1,0 ist;
wobei der Inhibitor des mitochondrialen oxidativen Phosphorylierungswegs eine Verbindung der Formel I-2, oder ein optisches Isomer davon, oder ein Racemat davon, oder ein Solvat davon, oder ein pharmakologisch verträgliches Salz davon ist; 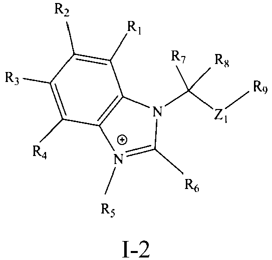
wobei R₁, R₂, R₃, R₄, R₇ und R₈ jeweils unabhängig für Wasserstoff stehen;
R₅ für C1-C6-Alkyl steht;
R₆ für C1-C6-Alkyl, substituiertes oder unsubstituiertes C6-C8-Aryl, substituiertes oder unsubstituiertes 5- bis 8-gliedriges Heteroaryl, wobei 'substituiert' bedeutet, dass 1, 2 oder 3 Wasserstoffatom der Gruppe durch einen Substituenten ersetzt sind, ausgewählt aus der folgenden Gruppe, umfassend C1-C4-Halogenalkyl;
R₉ für ubstituiertes oder unsubstituiertes C5-C8-Cycloalkyl, wobei 'substituiert' bedeutet, dass 1, 2 oder 3 Wasserstoffatom der Gruppe durch einen Substituenten ersetzt sind, ausgewählt aus der folgenden Gruppe, umfassend C1-C6-Alkyl;
Z₁ für 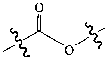 steht;
oder der Inhibitor des mitochondrialen oxidativen Phosphorylierungswegs eine Verbindung der Formel II, oder ein optisches Isomer davon, oder ein Racemat davon, oder ein Solvat davon,oder ein pharmakologisch verträgliches Salz davon ist; 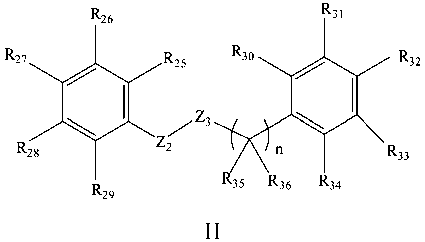
R₂₅, R₂₆, R₂₇, R₂₈ und R₂₉ jeweils unabhängig für Wasserstoff oder C1-C6-Halogenalkyloxy stehen;
R₃₀, R₃₁, R₃₂, R₃₄, R₃₅ und R₃₆ jeweils unabhängig für Wasserstoff stehen;
R₃₃ für substituiertes oder unsubstituiertes 3- bis 8-gliedriges Heterocycloalkyl steht, wobei 'substituiert' bedeutet, dass 1, 2 oder 3 Wasserstoffatome der Gruppe durch einen Substituenten ersetzt sind, ausgewählt aus der folgenden Gruppe, umfassend Mesyl, Sulfonyl;
Z₂ und Z₃ jeweils unabhängig für substituiertes oder unsubstituiertes C6-C8-Arylendiyl, substituiertes oder unsubstituiertes 3- bis 8-gliedriges Heteroarylendiyl stehen, wobei 'substituiert' bedeutet, dass 1, 2 oder 3 Wasserstoffatom der Gruppe durch einen Substituent ersetzt sind, ausgewählt aus der folgenden Gruppe, umfassend C-C4-Alkyl;
n für 1 steht;
oder der Inhibitor des mitochondrialen oxidativen Phosphorylierungswegs eine Verbindung der Formel III, oder ein optisches Isomer davon, oder ein Racemat davon,oder ein Solvat davon, oder ein pharmakologisch verträgliches Salz davon ist; 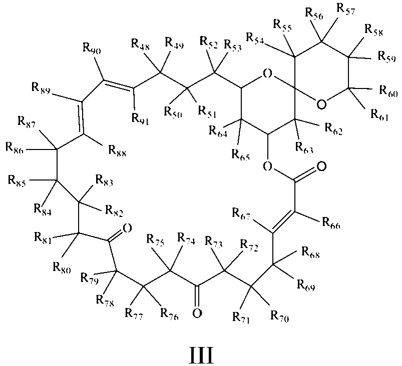
R₄₈, R₅₀, R₅₁, R₅₂, R₅₃, R₅₄, R₅₅, R₅₆, R₅₇, R₅₈, R₆₀, R₆₂, R₆₄, R₆₆, R₆₇, R₆₈, R₇₀, R₇₂, R₇₄, R₇₆, R₇₈, R₈₂, R₈₄, R₈₆, R₈₇, R₈₈, R₈₉, R₉₀ und R₉₁ jeweils unabhängig für Wasserstoff stehen;
R₄₉ für C1-C6-Alkyl steht;
R₅₉, R₆₃, R₆₅, R₆₉, R₇₃, R₇₅, R₇₉, R₈₀ und R₈₅ jeweils unabhängig für C1-C6-Alkyl stehen; R₆₁ für Hydroxyl, C1-C6-Alkyl steht;
R₇₁, R₇₇, R₈₁ und R₈₃ jeweils unabhängig für Hydroxyl stehen.

2. Verwendung des Inhibitors des mitochondrialen oxidativen Phosphorylierungswegs gemäß Anspruch 1, wobei der Inhibitor des mitochondrialen oxidativen Phosphorylierungswegs eine Verbindung der Formel I-2, oder ein optisches Isomer davon, oder ein Racemat davon, oder ein Solvat davon,oder ein pharmakologisch verträgliches Salz davon ist; 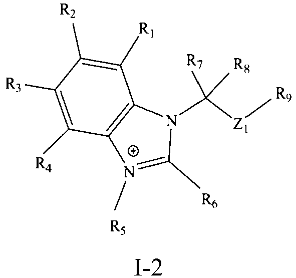
wobei R₁, R₂, R₃, R₄, R₇ und R₈ jeweils unabhängig für Wasserstoff stehen;
R₅ für Wasserstoff, Methyl, Ethyl, Propyl oder Butyl steht;
R₆ für Wasserstoff, Methyl, Ethyl, Propyl, Butyl oder 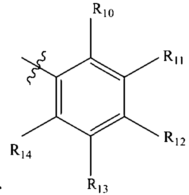 steht;
R₉ für 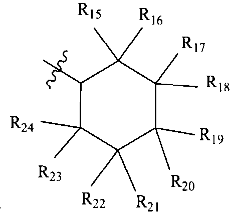 steht;
R₁₀, R₁₁, R₁₂, R₁₃ und R₁₄ jeweils unabhängig für Wasserstoff, C1-C4-Halogenalkyl stehen;
R₁₅, R₁₆, R₁₇, R₁₈, R₁₉, R₂₀, R₂₁, R₂₂, R₂₃ und R₂₄ jeweils unabhängig für Wasserstoff, oder C1-C6-Alkyl stehen;
Z₁ für 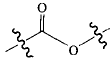 steht;
oder der Inhibitor des mitochondrialen oxidativen Phosphorylierungswegseine Verbindung der Formel II, oder ein optisches Isomer davon, oder ein Racemat davon, oder ein Solvat davon, oder ein pharmakologisch verträgliches Salz davon; 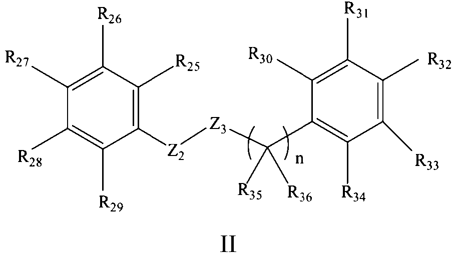
R₂₅, R₂₆, R₂₈, R₂₉, R₃₀, R₃₁, R₃₂, R₃₄, R₃₅ und R₃₆ jeweils unabhängig für Wasserstoff stehen;
R₂₇ für C1-C4-Halogenalkoxy steht;
R₃₃ für substituiertes oder unsubstituiertes Hexahydropyridinyl steht, wobei 'substituiert' bedeutet, dass 1, 2 oder 3 Wasserstoffatom der Gruppe durch einen Substituenten ersetzt sind, ausgewählt aus der folgenden Gruppe,umfassend Mesyl, Sulfonyl;
Z₂ und Z₃ jeweils unabhängig für substituiertes oder unsubstituiertes Oxadiazolyliden oder
substituiertes oder unsubstituiertes Triazolyl stehen, wobei 'substituiert' bedeutet, dass 1, 2 oder 3 Wasserstoffatome der Gruppe durch einen Substituenten ersetzt sind, ausgewählt aus der folgenden Gruppe, umfassend C1-C4-Alkyl;
n für 1 steht;
oder der Inhibitor des mitochondrialen oxidativen Phosphorylierungswegs eine Verbindung der Formel III, oder ein optisches Isomer davon, oder ein Racemat davon,oder ein Solvat davon, oder ein pharmakologisch verträgliches Salz davon ist; 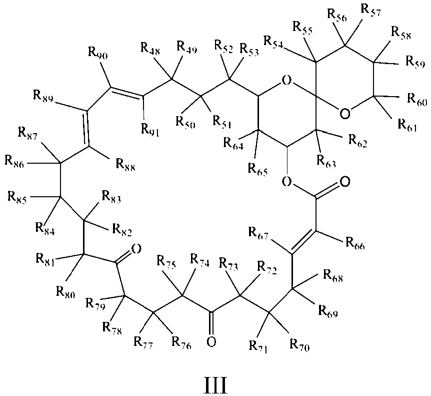
R₄₈, R₅₀, R₅₁, R₅₂, R₅₃, R₅₄, R₅₅, R₅₆, R₅₇, R₅₈, R₆₀, R₆₂, R₆₄, R₆₆, R₆₇, R₆₈, R₇₀, R₇₂, R₇₄, R₇₆, R₇₈, R₈₂, R₈₄, R₈₆, R₈₇, R₈₈, R₈₉, R₉₀ und R₉₁ jeweils unabhängig für Wasserstoff stehen;
R₄₉ für Methyl, Ethyl, Propyl oder Butyl steht;
R₅₉, R₆₃, R₆₅, R₆₉, R₇₃, R₇₅, R₇₉, R₈₀ und R₈₅ jeweils unabhängig für Methyl, Ethyl, Propyl oder Butyl stehen;
R₆₁ für Hydroxyl, (C1-C4-Alkyl) steht;
R₇₁, R₇₇, R₈₁ und R₈₃ jeweils unabhängig für Hydroxyl stehen.

3. Verwendung des Inhibitors des mitochondrialen oxidativen Phosphorylierungswegs gemäß Anspruch 1, wobei der Inhibitor des mitochondrialen oxidativen Phosphorylierungswegseine Verbindung der Formel I-2, oder ein optisches Isomer davon, oder ein Racemat davon, oder ein Solvat davon, oder ein pharmakologisch verträgliches Salz davon ist; 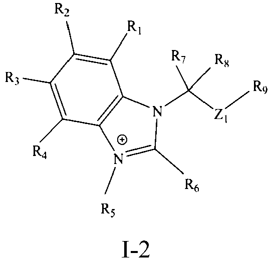
wobei R₁, R₂, R₃, R₄, R₇ und R₈ jeweils unabhängig für Wasserstoff stehen;
R₅ für Methyl steht;
R₆ für Propyl, oder 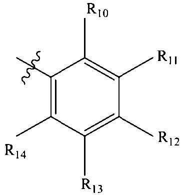 steht;
R₉ für 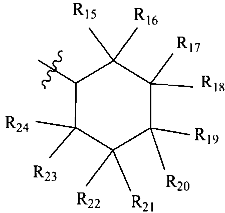 steht;
R₁₀, R₁₁, R₁₂ und R₁₄ jeweils unabhängig für Wasserstoff stehen;
R₁₃ für Trifluormethyl steht;
R₁₅, R₁₇, R₁₈, R₁₉, R₂₀, R₂₁, R₂₃ und R₂₄ jeweils unabhängig für Wasserstoff stehen;
R₁₆ für Propyl steht;
R₂₂ für Methyl steht;
Z₁ für Methyl 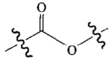 steht;
oder der Inhibitor des mitochondrialen oxidativen Phosphorylierungswegseine Verbindung der Formel II, oder ein optisches Isomer davon, oder ein Racemat davon, oder ein Solvat davon, oder ein pharmakologisch verträgliches Salz davon ist; 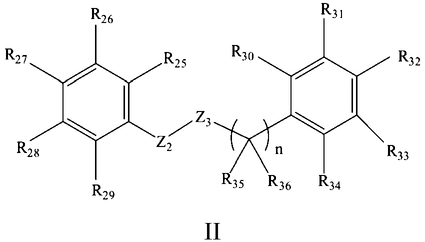
R₂₅, R₂₆, R₂₈, R₂₉, R₃₀, R₃₁, R₃₂, R₃₄, R₃₅ und R₃₆ jeweils unabhängig für Wasserstoff stehen;
R₂₇ für Trifluormethyl-O- steht;
R₃₃ für 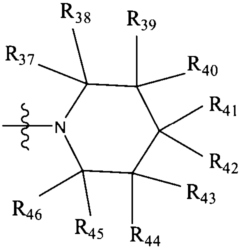 steht;
R₃₇, R₃₈, R₃₉, R₄₀, R₄₁, R₄₃, R₄₄, R₄₅ und R₄₆ jeweils unabhängig für Wasserstoff stehen;
R₄₂ für Methylsulfonyl steht;
Z₂ für 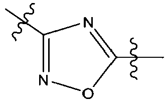 steht;
Z₃ für 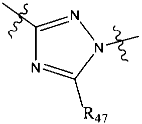 steht, R₄₇ für Methyl steht;
n für 1 steht;
oder der Inhibitor des mitochondrialen oxidativen Phosphorylierungswegseine Verbindung der Formel III, oder ein optisches Isomer davon, oder ein Racemat davon, oder ein Solvat davon, oder ein pharmakologisch verträgliches Salz davon ist; 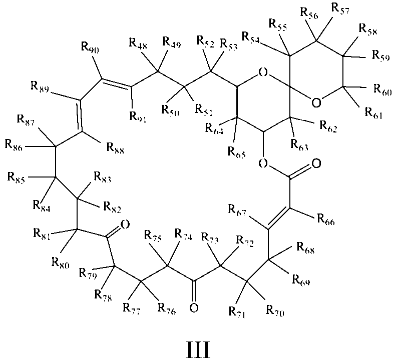
R₄₈, R₅₀, R₅₁, R₅₂, R₅₃, R₅₄, R₅₅, R₅₆, R₅₇, R₅₈, R₆₀, R₆₂, R₆₄, R₆₆, R₆₇, R₆₈, R₇₀, R₇₂, R₇₄, R₇₆, R₇₈, R₈₂, R₈₄, R₈₆, R₈₇, R₈₈, R₈₉, R₉₀ und R₉₁ jeweils unabhängig für Wasserstoff stehen;
R₄₉ für Ethyl steht;
R₅₉, R₆₃, R₆₅, R₆₉, R₇₃, R₇₅, R₇₉, R₈₀ und R₈₅ jeweils unabhängig für Methyl stehen;
R₆₁ für Hydroxyl, Propyl steht;
R₇₁, R₇₇, R₈₁ und R₈₃ jeweils unabhängig für Hydroxyl stehen.

4. Verwendung des Inhibitors des mitochondrialen oxidativen Phosphorylierungswegs gemäß Anspruch 1 **dadurch gekennzeichnet, dass** der Tumor ein menschlicher Tumor ist;
das NNMT-Gen ein menschliches NNMT-Gen ist;
die Expression Proteinexpression und/oder mRNA-Expression umfasst; und/oder
die DNA-Methylase aus der Gruppe ausgewählt ist, die NMT1, DNMT3a, DNMT3b oder eine Kombination davon umfasst.

5. Verwendung des Inhibitors des mitochondrialen oxidativen Phosphorylierungswegs gemäß Anspruch 1 **dadurch gekennzeichnet, dass** die niedrige oder keine Expression des NNMT-Gens bedeutet, dass das Verhältnis (E1/E0) des Expressionsniveaus E1 des NNMT-Gens in der Tumorzelle im Vergleich zum Expressionsniveau E0 des NNMT-Gens in Tumorzell desselben Typs mit normaler Expression des NNMT-Gens ≤ 0,7 ist;
der Tumor mit hoher Expression von DNA-Methylase bedeutet, dass das Verhältnis (A1/A0) des Expressionsniveaus A1 der DNA-Methylase in der Tumorzelle im Vergleich zum Expressionsniveau A0 der DNA-Methylase in Tumorzell desselben Typs mit normaler Expression der DNA-Methylase ≥ 1,2 ist;
der Tumor mit hoher Expression von UHRF1 bedeutet, dass das Verhältnis (F1/F0) des Expressionsniveaus F1 von UHRF1 in der Tumorzelle im Vergleich zum Expressionsniveau F0 von UHRF1 in Tumorzell desselben Typs mit normaler Expression von UHRF1 ≥ 1,2 ist;
der hohe Methylierungsgrad der Nukleotidstelle des NNMT-Gens bedeutet, dass das Verhältnis (L1/L0) des Methylierungsgrads L1 der Nukleotidstelle des NNMT-Gens in der Tumorzelle im Vergleich zum Methylierungsgrad L0 der Nukleotidstelle des NNMT-Gens in Tumorzell desselben Typs mit normalem Methylierungsgrad ≥ 1,2 ist; und/oder
der hohe Methylierungsgrad der DNA-CpG-Stelle des NNMT-Gens bedeutet, dass das Verhältnis (W1/W0) des Methylierungsgrads W1 der DNA-CpG-Stelle des NNMT-Gens in der Tumorzelle im Vergleich zum Methylierungsgrad W0 der DNA-CpG-Stelle des NNMT-Gens in Tumorzell desselben Typs mit normalem Methylierungsgrad ≥ 1,2 ist.

6. Verwendung des Inhibitors des mitochondrialen oxidativen Phosphorylierungswegs gemäß Anspruch 1 **dadurch gekennzeichnet, dass** die niedrige oder keine Expression des NNMT-Gens bedeutet, dass das Verhältnis (E1/E0) des Expressionsniveaus E1 des NNMT-Gens in der Tumorzelle im Vergleich zum Expressionsniveau E0 des NNMT-Gens in Tumorzell desselben Typs mit normaler Expression des NNMT-Gens ≤ 0,5 ist;
der Tumor mit hoher Expression von DNA-Methylase bedeutet, dass das Verhältnis (A1/A0) des Expressionsniveaus A1 der DNA-Methylase in der Tumorzelle im Vergleich zum Expressionsniveau A0 der DNA-Methylase in Tumorzell desselben Typs mit normaler Expression der DNA-Methylase ≥ 2 ist;
der Tumor mit hoher Expression von UHRF1 bedeutet, dass das Verhältnis (F1/F0) des Expressionsniveaus F1 von UHRF1 in der Tumorzelle im Vergleich zum Expressionsniveau F0 von UHRF1 in Tumorzell desselben Typs mit normaler Expression von UHRF1 ≥ 2 ist;
der hohe Methylierungsgrad der Nukleotidstelle des NNMT-Gens bedeutet, dass das Verhältnis (L1/L0) des Methylierungsgrads L1 der Nukleotidstelle des NNMT-Gens in der Tumorzelle im Vergleich zum Methylierungsgrad L0 der Nukleotidstelle des NNMT-Gens in Tumorzell desselben Typs mit normalem Methylierungsgrad ≥ 2 ist; und/oder
der hohe Methylierungsgrad der DNA-CpG-Stelle des NNMT-Gens bedeutet, dass das Verhältnis (W1/W0) des Methylierungsgrads W1 der DNA-CpG-Stelle des NNMT-Gens in der Tumorzelle im Vergleich zum Methylierungsgrad W0 der DNA-CpG-Stelle des NNMT-Gens in Tumorzell desselben Typs mit normalem Methylierungsgrad ≥ 2 ist.

7. Verwendung des Inhibitors des mitochondrialen oxidativen Phosphorylierungswegs gemäß Anspruch 1 **dadurch gekennzeichnet, dass** die niedrige oder keine Expression des NNMT-Gens bedeutet, dass das Verhältnis (E1/E0) des Expressionsniveaus E1 des NNMT-Gens in der Tumorzelle im Vergleich zum Expressionsniveau E0 des NNMT-Gens in Tumorzell desselben Typs mit normaler Expression des NNMT-Gens ≤ 0,3 ist;
der Tumor mit hoher Expression von DNA-Methylase bedeutet, dass das Verhältnis (A1/A0) des Expressionsniveaus A1 der DNA-Methylase in der Tumorzelle im Vergleich zum Expressionsniveau A0 der DNA-Methylase in Tumorzell desselben Typs mit normaler Expression der DNA-Methylase ≥ 5 ist;
der Tumor mit hoher Expression von UHRF1 bedeutet, dass das Verhältnis (F1/F0) des Expressionsniveaus F1 von UHRF1 in der Tumorzelle im Vergleich zum Expressionsniveau F0 von UHRF1 in Tumorzell desselben Typs mit normaler Expression von UHRF1 ≥ 5 ist;
der hohe Methylierungsgrad der Nukleotidstelle des NNMT-Gens bedeutet, dass das Verhältnis (L1/L0) des Methylierungsgrads L1 der Nukleotidstelle des NNMT-Gens in der Tumorzelle im Vergleich zum Methylierungsgrad L0 der Nukleotidstelle des NNMT-Gens in Tumorzell desselben Typs mit normalem Methylierungsgrad ≥ 5 ist; und/oder
der hohe Methylierungsgrad der DNA-CpG-Stelle des NNMT-Gens bedeutet, dass das Verhältnis (W1/W0) des Methylierungsgrads W1 der DNA-CpG-Stelle des NNMT-Gens in der Tumorzelle im Vergleich zum Methylierungsgrad W0 der DNA-CpG-Stelle des NNMT-Gens in Tumorzell desselben Typs mit normalem Methylierungsgrad ≥ 5 ist.

8. Verwendung des Inhibitors des mitochondrialen oxidativen Phosphorylierungswegs gemäß Anspruch 1 **dadurch gekennzeichnet, dass** die niedrige oder keine Expression des NNMT-Gens bedeutet, dass das Verhältnis (E1/E0) des Expressionsniveaus E1 des NNMT-Gens in der Tumorzelle im Vergleich zum Expressionsniveau E0 des NNMT-Gens in Tumorzell desselben Typs mit normaler Expression des NNMT-Gens ≤ 0,1 ist;
der Tumor mit hoher Expression von DNA-Methylase bedeutet, dass das Verhältnis (A1/A0) des Expressionsniveaus A1 der DNA-Methylase in der Tumorzelle im Vergleich zum Expressionsniveau A0 der DNA-Methylase in Tumorzell desselben Typs mit normaler Expression der DNA-Methylase ≥ 10 ist;
der Tumor mit hoher Expression von UHRF1 bedeutet, dass das Verhältnis (F1/F0) des Expressionsniveaus F1 von UHRF1 in der Tumorzelle im Vergleich zum Expressionsniveau F0 von UHRF1 in Tumorzell desselben Typs mit normaler Expression von UHRF1 ≥ 10 ist;
der hohe Methylierungsgrad der Nukleotidstelle des NNMT-Gens bedeutet, dass das Verhältnis (L1/L0) des Methylierungsgrads L1 der Nukleotidstelle des NNMT-Gens in der Tumorzelle im Vergleich zum Methylierungsgrad L0 der Nukleotidstelle des NNMT-Gens in Tumorzell desselben Typs mit normalem Methylierungsgrad ≥ 10 ist; und/oder
der hohe Methylierungsgrad der DNA-CpG-Stelle des NNMT-Gens bedeutet, dass das Verhältnis (W1/W0) des Methylierungsgrads W1 der DNA-CpG-Stelle des NNMT-Gens in der Tumorzelle im Vergleich zum Methylierungsgrad W0 der DNA-CpG-Stelle des NNMT-Gens in Tumorzell desselben Typs mit normalem Methylierungsgrad ≥ 10 ist.

9. Verwendung des Inhibitors des mitochondrialen oxidativen Phosphorylierungswegs gemäß Anspruch 1 **dadurch gekennzeichnet, dass** der hohe Methylierungsgrad der Nukleotidstelle des NNMT-Gens bedeutet, dass der Methylierungsgrad(M%) der Nukleotidstelle des NNMT-Gens in Tumorzellen ≥ 3% und ≤ M1% beträgt, wobei M1 eine beliebige positive Ganzzahl zwischen 3 und 100 ist;
der Methylierungsgrad der Nukleotidstelle des NNMT-Gens sich auf das Verhältnis der Anzahl methylierter Nukleotide in der NNMT-Genregion zur Gesamtzahl der Nukleotide in der NNMT-Genregion bezieht;
der Methylierungsgrad der Nukleotidstelle des NNMT-Gens den Methylierungsgrad der Nukleotidstelle in der Promotorregion des NNMT-Gens umfasst;
der Methylierungsgrad der Nukleotidstelle des NNMT-Gens den Methylierungsgrad der Nukleotidstelle im Bereich von 1050bp vom Transkriptionsstartpunkt bis 499bp nach dem Transkriptionsstartpunkt des NNMT-Gens umfasst;
der Methylierungsgrad der Nukleotidstelle des NNMT-Gens den Methylierungsgrad der Nukleotidstelle im Bereich von 1050bp vom Transkriptionsstartpunkt bis 193bp vor dem Transkriptionsstartpunkt des NNMT-Gens umfasst;
der Methylierungsgrad der Nukleotidstelle des NNMT-Gens den Methylierungsgrad der Nukleotidstelle im Bereich von 840bp vom Transkriptionsstartpunkt bis 469bp vom Transkriptionsstartpunkt des NNMT-Gens umfasst;
der Methylierungsgrad der Nukleotidstellen des NNMT-Gens den Methylierungsgrad der Nukleotidstellen im Bereich zwischen zwei beliebigen Stellen(einschließlich dieser beiden Stellen selbst), ausgewählt aus den Positionen 114165695, 114165730, 114165769, 114165804, 114165938, 114166050 und 114166066 des menschlichen Chromosoms 11, umfasst;
der Methylierungsgrad der Nukleotidstellen des NNMT-Gens den Methylierungsgrad der Nukleotidstellenstellen umfasst, die aus der folgenden Gruppe ausgewählt sind, die Position 114165695 des menschlichen Chromosoms 11, Position 114165730 des menschliches Chromosom 11, Position 114165769 des menschliches Chromosom 11, Position 114165804 des menschliches Chromosom 11, Position 114165938 des menschliches Chromosom 11, Position 114166050 des menschliches Chromosom 11, Position 114166066 des menschliches Chromosom 11 oder eine Kombination davon umfasst;
der Methylierungsgrad der Nukleotidstellen des NNMT-Gens der Methylierungsgrad der Nukleotidstelle im Bereich zwischen zwei beliebigen Stellen (einschließlich dieser beiden Stellen selbst), ausgewählt aus den Positionen 1161, 1196, 1235, 1270, 1404, 1516 und 1532 der Nukleotidsequenz in SEQ ID NO: 1, umfasst;
der Methylierungsgrad der DNA-CpG-Stellen des NNMT-Gens der Methylierungsgrad der Nukleotidstelle, ausgewählt aus den Positionen 1161, 1196, 1235, 1270, 1404, 1516 und 1532 der Nukleotidsequenz in SEQ ID NO: 1 oder einer Kombination davon, umfasst;
der hoher Methylierungsgrad der DNA-CpG-Stelle des NNMT-Gens bedeutet, dass der Methylierungsgrad (M%) in Tumorzellen ≥ 3 % und ≤ M2 % beträgt, wobei M2 eine beliebge positive Ganzzahl zwischen 3 und 100 ist;
der Methylierungsgrad der DNA-CpG-Stelle des NNMT-Gens sich auf das Verhältnis der Anzahl methylierter CpG-Nukleotide in der NNMT-Genregion zur Gesamtzahl der Nukleotide in der NNMT-Genregion bezieht;
der Methylierungsgrad der DNA-CpG-Stellen des NNMt-Gens sich auf das Verhältnis der Anzahl methylierter CpG-Nukleotide zur Gesamtzahl der CpG-Nukleotide in der NNMT-Genregion bezieht;
der Methylierungsgrad der DNA-CpG-Stellen des NNMT-Gens den Methylierungsgrad der DNA-CpG-Stellen in der Promotorregion des NNMT-Gens umfasst;
der Methylierungsgrad der DNA-CpG-Stellen des NNMT-Gens den Methylierungsgrad der DNA-CpG-Stellen im Bereich von 1050bp vom Transkriptionsstartpunkt bis 499bp nach dem Transkriptionsstartpunkt des NNMT-Gens umfasst;
der Methylierungsgrad der DNA-CpG-Stellen des NNMT-Gens den Methylierungsgrad der DNA-CpG-Stellen im Bereich von 1050bp vom Transkriptionsstartpunkt bis 193bp vom Transkriptionsstartpunkt des NNMT-Gens umfasst;
der Methylierungsgrad der DNA-CpG-Stellen des NNMT-Gens den Methylierungsgrad der DNA-CpG-Stellen im Bereich von 840bp vom Transkriptionsstartpunkt bis 469bp vom Transkriptionsstartpunkt des NNMT-Gens umfasst;
der Methylierungsgrad der DNA-CpG-Stellen des NNMT-Gens den Methylierungsgrad der DNA-CpG-Stellen im Bereich zwischen zwei beliebigen Stellen(einschließlich dieser beiden Stellen selbst), ausgewählt aus den Positionen 114165695, 114165730, 114165769, 114165804, 114165938, 114166050 und 114166066 des menschlichen Chromosoms 11, umfasst;
der Methylierungsgrad der DNA-CpG-Stellen des NNMT-Gens den Methylierungsgrad der Stellen, ausgewählt aus der folgenden Gruppe: Position 114165695 des menschlichen Chromosoms 11, Position 114165730 des menschliches Chromosom 11, Position 114165769 des menschliches Chromosom 11, Position 114165804 des menschliches Chromosom 11, Position 114165938 des menschliches Chromosom 11, Position 114166050 des menschliches Chromosom 11, Position 114166066 des menschliches Chromosom 11 oder eine Kombination davon, umfasst;
der Methylierungsgrad der DNA-CpG-Stellen des NNMT-Gens der Methylierungsgrad der DNA-CpG-Stellen im Bereich zwischen zwei beliebigen Stellen (einschließlich dieser beiden Stellen selbst), ausgewählt aus den Positionen 1161, 1196, 1235, 1270, 1404, 1516 und 1532 der Nukleotidsequenz in SEQ ID NO:1, umfasst;
der Methylierungsgrad der DNA-CpG- Stellen des NNMT-Gens der Methylierungsgrad der DNA-CpG-Stellen, ausgewählt aus den Positionen 1161, 1196, 1235, 1270, 1404, 1516 und 1532 der Nukleotidsequenz in SEQ ID NO: 1, oder einer Kombinationen davon, umfasst.

10. Verwendung des Inhibitors des mitochondrialen oxidativen Phosphorylierungswegs gemäß Anspruch 9 **dadurch gekennzeichnet, dass** M1 5, 10, 15, 20, 25, 30, 35, 40, 45, 50, 55, 60, 65, 70, 80, 85, 90, 95 oder 100 ist;
M2 5, 10, 15, 20, 25, 30, 35, 40, 45, 50, 55, 60, 65, 70, 80, 85, 90, 95 oder 100 ist;
die Nukleotidsequenz der NNMT-Gen-Promotorregion wie in SEQ ID NO: 1 dargestellt ist;
die Stelle von 1050bp vomTranskriptionsstartpunkt bis 499bp nach Transkriptionsstartpunkt des NNMT-Gens die Positionen 951-2500 der in SEQ ID NO: 1 dargestellten Nukleotidsequenz sind;
die Stelle von 1050bp vom Transkriptionsstartpunkt bis 499bp vom Transkriptionsstartpunkt des NNMT-Gens die Positionen 951-1808 der in SEQ ID NO: 1 dargestellten Nukleotidsequenz sind;
die Stelle von 1050bp vom Transkriptionsstartpunkt und 499bp vom Transkriptionsstartpunkt des NNMT-Gens die Positionen 1161-1532 der in SEQ ID NO: 1 dargestellten Nukleotidsequenz sind.

11. Verwendung des Inhibitors des mitochondrialen oxidativen Phosphorylierungswegs gemäß Anspruch 1 **dadurch gekennzeichnet, dass** der Tumor aus der Gruppe ausgewählt ist, die Lungenkrebs, Nierenkrebs, Brustkrebs, Dickdarmkrebs, Mastdarmkrebs, kolorektalem Krebs, Lymphom, Leukämie, Bauchspeicheldrüsenkrebs, Hirntumor, Leberkrebs, Prostatakrebs, Melanom oder eine Kombination davon umfasst.

12. Verwendung des Inhibitors des mitochondrialen oxidativen Phosphorylierungswegs gemäß Anspruch 11 **dadurch gekennzeichnet, dass** der Lungenkrebs aus der Gruppe ausgewählt ist, die nicht-kleinzelligen Lungenkrebs, kleinzelligen Lungenkrebs, metastasierten Lungenkrebs, oder eine Kombination davon umfasst;
der Kolonkrebs das Kolonadenokarzinom umfasst;
der Rektumkrebs das Rektumadenokarzinom umfasst;
der Kolorektalkrebs das kolorektale Adenokarzinom umfasst;
das Lymphom aus der Gruppe ausgewählt ist, die B-Zell-Lymphom, T-Zell-Lymphom, kutanes T-Zell-Lymphom, großzelliges Lymphom, histiozytisches Lymphom oder eine Kombination davon umfasst;
das Lymphom das diffus großzellige B-Zell-Lymphom umfasst;
der Hirntumor aus der Gruppe ausgewählt ist, die Glioblastom, neurogliales Gliom oder eine Kombination davon umfasst;
der Hirntumor ein Medulloblastom umfasst;
der Hirntumor ein Glioblastoma multiforme umfasst;
der Nierenkrebs aus der Gruppe ausgewählt ist, die klarzelliges Nierenzellkarzinom, metastasierendes Nierenzellkarzinom oder eine Kombination davon umfasst;
die Leukämie aus der Gruppe ausgewählt ist, die T-Zell-Leukämie, myeloische Leukämie oder eine Kombination davon umfasst;
der Prostatakrebs aus der Gruppe ausgewählt ist, die metastasierenden Prostatakrebs umfasst;
der Brustkrebs aus der Gruppe ausgewählt ist, die duktales Mamakarzinom, metastasierenden Brustkrebs oder eine Kombination davon umfasst; und/oder
der Bauchspeicheldrüsenkrebs hepatisch metastasierenden Pankreaskrebs umfasst.

13. Verwendung des Inhibitors des mitochondrialen oxidativen Phosphorylierungswegs gemäß Anspruch 1 **dadurch gekennzeichnet, dass** der Inhibitor des mitochondrialen oxidativen Phosphorylierungswegs aus der folgenden Gruppe ausgewählt ist;

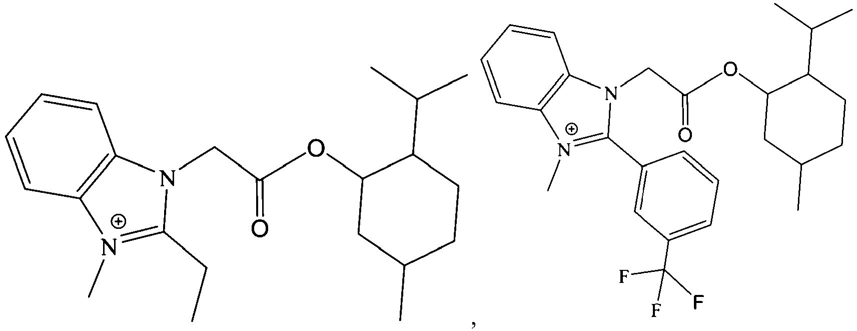

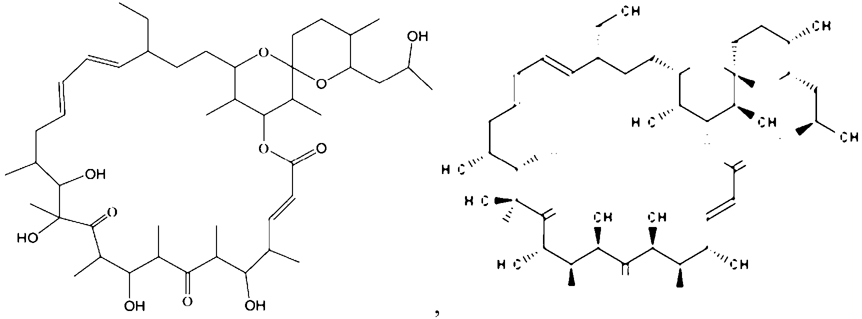

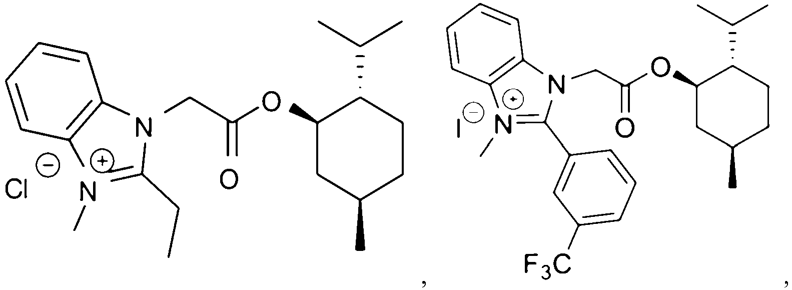

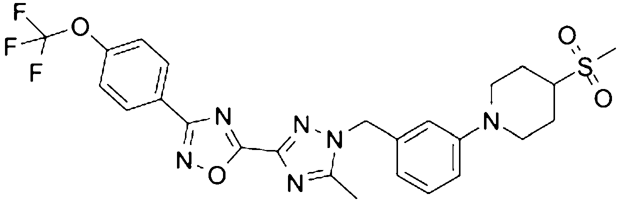

## Revendications

1. Inhibiteur de voie de phosphorylation oxydative mitochondriale destiné à être utilisé pour prévenir et/ou traiter une tumeur;
dans lequel, la tumeur comprend une tumeur avec une expression faible ou nulle d'un gène NNMT; et/ou
la tumeur comprend une tumeur avec une expression élevée d'une méthylase d'ADN; et/ou
la tumeur comprend une tumeur présentant une expression élevée de UHRF 1; et/ou
la tumeur comprend une tumeur présentant un niveau élevé de méthylation d'un site nucléotidique du gène NNMT; et/ou
la tumeur comprend une tumeur présentant un niveau élevé de méthylation d'un site CpG d'ADN du gène NNMT;
dans lequel, la faible expression ou l'absence d'expression du gène NNMT signifie que un rapport (E1/E0) entre un niveau d'expression E1 du gène NNMT dans une cellule tumorale et un niveau d'expression E0 du gène NNMT dans le même type de cellule tumorale présentant une expression normale du gène NNMT est < 1,0;
la tumeur présentant une expression élevée de la méthylase d'ADN désigne un rapport (A1/A0) entre un niveau d'expression A1 de la méthylase d'ADN dans une cellule tumorale et un niveau d'expression A0 de la méthylase d'ADN dans le même type de cellule tumorale avec une expression normale de la méthylase d'ADN est > 1,0;
la tumeur présentant une expression élevée de l'UHRF1 signifie que un rapport (F1/F0) entre un niveau d'expression F1 de UHRF1 dans une cellule tumorale et une niveau d'expression F0 de l'UHRF1 dans le même type de cellule tumorale présentant une expression normale de UHRF1 est > 1,0;
le niveau élevé de méthylation du site nucléotidique du gène NNMT signifie que un rapport (L1/L0) entre un niveau de méthylation L1 du site nucléotidique du gène NNMT dans une cellule tumorale et un niveau de méthylation L0 du site nucléotidique du gène NNMT dans le même type de cellule tumorale présentant un niveau normal de méthylation du site nucléotidique du gène NNMT est > 1,0;
le niveau élevé de méthylation du site CpG d'ADN du gène NNMT signifie que un rapport (W1/W0) entre un niveau de méthylation W1 du site CpG d'ADN du gène NNMT dans une cellule tumorale et un niveau de méthylation W0 du site CpG d'ADN du gène NNMT dans le même type de cellule tumorale présentant un niveau normal de méthylation du site CpG d'ADN du gène NNMT est > 1,0;
dans lequel l'inhibiteur de voie de phosphorylation oxydative mitochondriale est un composé de formule I-2, ou un isomère optique de celui-ci, ou un racémate de celui-ci, ou un solvate de celui-ci, ou un sel pharmaceutiquement acceptable de celui-ci; 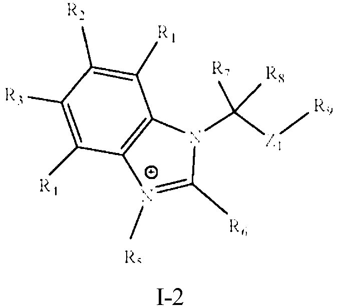
R₁, R₂, R₃, R₄, R₇ et R₈ sont chacun indépendamment un atome d'hydrogène;
R₅ est un groupe alkyle en C1-C6;
R₆ est un groupe alkyle en C1-C6, un groupe aryle en C6-C8 substitué ou non substitué, ou un groupe hétéroaryle à 5 à 8 chaînons substitué ou non substitué, dans lequel chaque «substitué» signifie que 1, 2 ou 3 atomes d'hydrogène sur un groupe sont substitués par un groupe halogénoalkyle en C1-C4;
R₉ est un groupe cycloalkyle en C5-C8 substitué ou non substitué, dans lequel « substitué » signifie que 1, 2 ou 3 atomes d'hydrogène sur un groupe sont substitués par un groupe alkyle en C1-C6;
Z₁ est 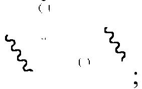
ou, dans lequel l'inhibiteur de voie de phosphorylation oxydative mitochondriale est un composé de formule II, ou un isomère optique de celui-ci, ou un racémate de celui-ci, ou un solvate de celui-ci, ou un sel pharmaceutiquement acceptable de celui-ci; 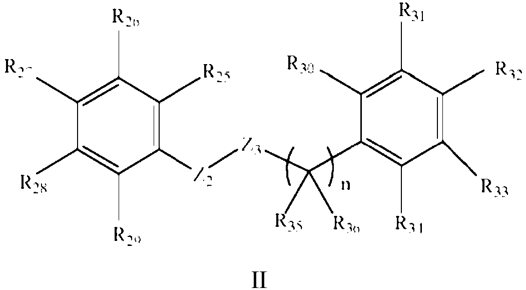
R₂₅, R₂₆, R₂₇, R₂₈ et R₂₉ sont chacun indépendamment un atome d'hydrogène ou un groupe halogénoalcoxy en C1-C6;
R₃₀, R₃₁, R₃₂, R₃₄, R₃₅ et R₃₆ sont chacun indépendamment un atome d'hydrogène;
R₃₃ est un groupe hétérocycloalkyle à 3 à 8 chaînons substitué ou non substitué, dans lequel «substitué» signifie que 1, 2 ou 3 atomes d'hydrogène sur un groupe sont substitués par un groupe méthylsulfonyle et un groupe sulfonyle;
Z₂ et Z₃ sont chacun indépendamment un groupe arylène en C6-C8 substitué ou non substitué, ou un groupe hétéroarylène à 3 à 8 chaînons substitué ou non substitué, dans lequel chaque «substitué» signifie que 1, 2 ou 3 atomes d'hydrogène sur un groupe sont substitués par un un groupe alkyle en C1-C4;
n est 1;
ou, dans lequel l'inhibiteur de voie de phosphorylation oxydative mitochondriale est un composé de formule III, ou un isomère optique de celui-ci, ou un racémate de celui-ci, ou un solvate de celui-ci, ou un sel pharmaceutiquement acceptable de celui-ci; 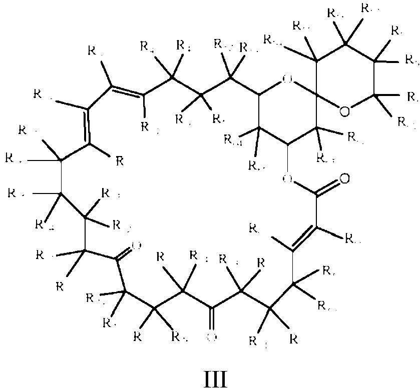
R₄₈, R₅₀, R₅₁, R₅₂, R₅₃, R₅₄, R₅₅, R₅₆, R₅₇, R₅₈, R₆₀, R₆₂, R₆₄, R₆₆, R₆₇, R₆₈, R₇₀, R₇₂, R₇₄, R₇₆, R₇₈, R₈₂, R₈₄, R₈₆, R₈₇, R₈₈, R₈₉, R₉₀ et R₉₁ sont chacun indépendamment un atome d'hydrogène;
R₄₉ est un groupe alkyle en C1-C6;
R₅₉, R₆₃, R₆₅, R₆₉, R₇₃, R₇₃, R₇₉, R₈₀ et R₈₅ sont chacun indépendamment un groupe alkyle en C1-C6;
R₆₁ est un groupe hydroxyle-(alkyle en C1-C6)-;
R₇₁, R₇₇, R₈₁ et R₈₃ sont chacun indépendamment un groupe hydroxyle.

2. Un inhibiteur de voie de phosphorylation oxydative mitochondriale selon la revendication 1, dans lequel l'inhibiteur de voie de phosphorylation oxydative mitochondriale étant un composé de formule I-2, ou un isomère optique de celui-ci, ou un racémate de celui-ci, ou un solvate de celui-ci, ou un sel pharmaceutiquement acceptable de celui-ci; 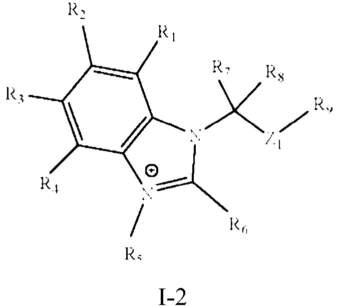
R₁, R₂, R₃, R₄, R₇ et R₈ sont chacun indépendamment un atome d'hydrogène;
R₅ est un groupe hydrogène, méthyle, éthyle, propyle ou butyle;
R₆ est un hydrogène, un méthyle, un éthyle, un propyle, un butyle ou 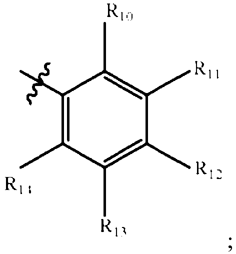
R₉ est 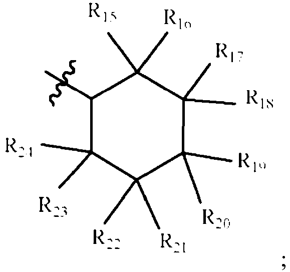
R₁₀, R₁₁, R₁₂, R₁₃ et R₁₄ sont chacun indépendamment un atome d'hydrogène ou un groupe halogénoalkyle en C1-C4;
R₁₅, R₁₆, R₁₇, R₁₈, R₁₉, R₂₀, R₂₁, R₂₂, R₂₃ et R₂₄ sont chacun indépendamment un atome d'hydrogène ou un groupe alkyle en C1-C6;
Z₁ est 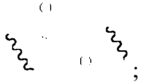
ou, dans lequel l'inhibiteur de voie de phosphorylation oxydative mitochondriale est un composé de formule II, ou un isomère optique de celui-ci, ou un racémate de celui-ci, ou un solvate de celui-ci, ou un sel pharmaceutiquement acceptable de celui-ci; 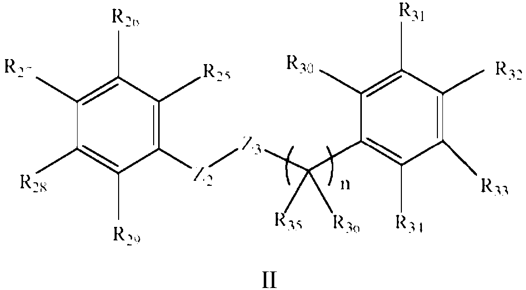
R₂₅, R₂₆, R₂₈, R₂₉, R₃₀, R₃₁, R₃₂, R₃₄, R₃₅ et R₃₆ sont chacun indépendamment un atome d'hydrogène;
R₂₇ est un groupe halogénoalcoxy en C1-C4;
R₃₃ est un groupe hexahydropyridyle substitué ou non substitué, dans lequel «substitué» signifie que 1, 2 ou 3 atomes d'hydrogène sur un groupe sont substitués par un groupe méthylsulfonyle, et un group sulfonyle;
Z₂ et Z₃ sont chacun indépendamment un groupe oxadiazolylène substitué ou non substitué, ou un groupe triazolylène substitué ou non substitué, dans lequel chaque terme «substitué» signifie qu'un atome d'hydrogène sur un groupe est substitué par ungroupe alkyle en C1-C4;
n est 1;
ou, dans lequel l'inhibiteur de voie de phosphorylation oxydative mitochondriale est un composé de formule III, ou un isomère optique de celui-ci, ou un racémate de celui-ci, ou un solvate de celui-ci, ou un sel pharmaceutiquement acceptable de celui-ci; 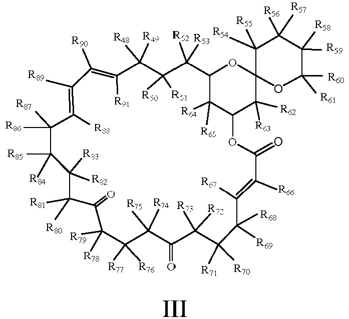
R₄₈, R₅₀, R₅₁, R₅₂, R₅₃, R₅₄, R₅₅, R₅₆, R₅₇, R₅₈, R₆₀, R₆₂, R₆₄, R₆₆, R₆₇, R₆₈, R₇₀, R₇₂, R₇₄, R₇₆, R₇₈, R₈₂, R₈₄, R₈₆, R₈₇, R₈₈, R₈₉, R₉₀ et R₉₁ représentent chacun indépendamment un atome d'hydrogène;
R₄₉ représente un groupe méthyle, éthyle, propyle ou butyle;
R₅₉, R₆₃, R₆₅, R₆₉, R₇₃, R₇₅, R₇₉, R₈₀ et R₈₅ représentent chacun indépendamment un groupe méthyle, éthyle, propyle ou butyle;
R₆₁ est un groupe hydroxyle-(alkyle en C1-C4)-;
R₇₁, R₇₇, R₈₁ et R₈₃ représentent chacun indépendamment un groupe hydroxyle.

3. Un inhibiteur de voie de phosphorylation oxydative mitochondriale selon la revendication 1, dans lequel l'inhibiteur de voie de phosphorylation oxydative mitochondriale étant un composé de formule I-2, ou un isomère optique de celui-ci, ou un racémate de celui-ci, ou un solvate de celui-ci, ou un sel pharmaceutiquement acceptable de celui-ci; 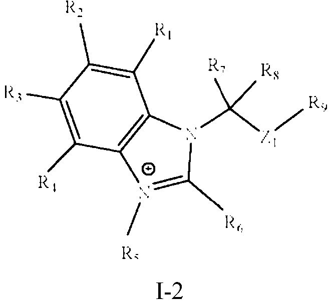
R₁, R₂, R₃, R₄, R₇ et R₈ représentent chacun indépendamment un atome d'hydrogène;
R₅ représente un groupe méthyle;
R₆ représente un groupe propyle; ou 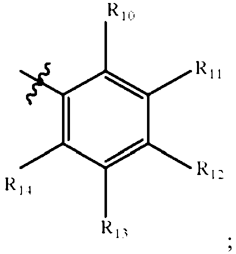
R₉ est 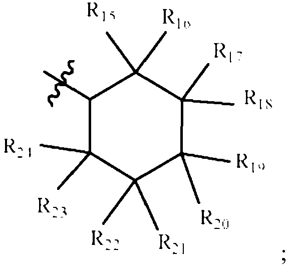
R₁₀, R₁₁, R₁₂ et R₁₄ représentent chacun indépendamment un atome d'hydrogène;
R₁₃ représente un groupe trifluorométhyle;
R₁₅, R₁₇, R₁₈, R₁₉, R₂₀, R₂₁, R₂₃ et R₂₄ représentent chacun indépendamment un atome d'hydrogène;
R₁₆ représente un groupe propyle;
R₂₂ est un groupe méthyle;
Z₁ est 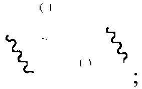
ou, dans lequel l'inhibiteur de voie de phosphorylation oxydative mitochondriale est un composé de formule II, ou un isomère optique de celui-ci, ou un racémate de celui-ci, ou un solvate de celui-ci, ou un sel pharmaceutiquement acceptable de celui-ci; 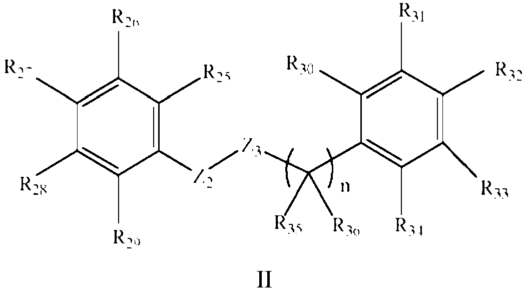
R₂₅, R₂₆, R₂₈, R₂₉, R₃₀, R₃₁, R₃₂, R₃₄, R₃₅ et R₃₆ représentent chacun indépendamment un atome d'hydrogène;
R₂₇ est trifluorométhyl-O-;
R₃₃ est 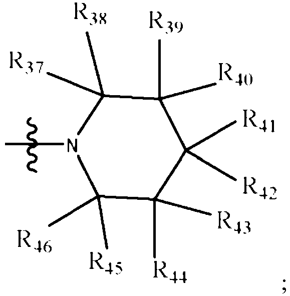
R₃₇, R₃₈, R₃₉, R₄₀, R₄₁, R₄₃, R₄₄, R₄₅ et R₄₆ représentent chacun indépendamment un atome d'hydrogène;
R₄₂ est un groupe méthylsulfonyle;
Z₂ est 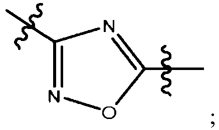
Z₃ est 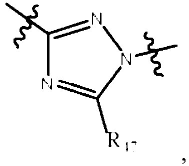 R₄₇ est un groupe méthyle;
n est 1;
ou, dans lequel l'inhibiteur de voie de phosphorylation oxydative mitochondriale est un composé de formule III, ou un isomère optique de celui-ci, ou un racémate de celui-ci, ou un solvate de celui-ci, ou un sel pharmaceutiquement acceptable de celui-ci; 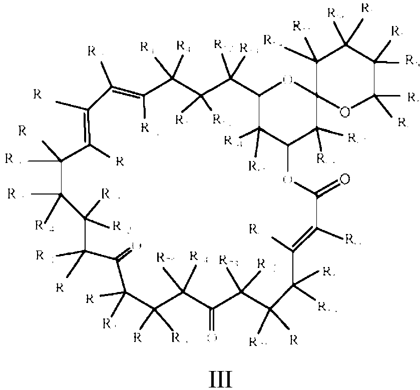
R₄₈, R₅₀, R₅₁, R₅₂, R₅₃, R₅₄, R₅₅, R₅₆, R₅₇, R₅₈, R₆₀, R₆₂, R₆₄, R₆₆, R₆₇, R₆₈, R₇₀, R₇₂, R₇₄, R₇₆, R₇₈, R₈₂, R₈₄, R₈₆, R₈₇, R₈₈, R₈₉, R₉₀ et R₉₁ représentent chacun indépendamment un atome d'hydrogène;
R₄₉ est un groupe éthyle;
R₅₉, R₆₃, R₆₅, R₆₉, R₇₃, R₇₅, R₇₉, R₈₀ et R₈₅ représentent chacun indépendamment un groupe méthyle;
R₆₁ est un groupe hydroxypropyle;
R₇₁, R₇₇, R₈₁ et R₈₃ sont chacun indépendamment un groupe hydroxyle.

4. Un inhibiteur de voie de phosphorylation oxydative mitochondriale selon la revendication 1, dans lequel la tumeur est une tumeur humaine;
le gène NNMT est le gène NNMT humain;
l'expression comprend l'expression de protéines et/ou l'expression d'ARNm; et/ou
la méthylase d'ADN est DNMT1, DNMT3a, DNMT3b et leurs combinaisons.

5. Un inhibiteur de voie de phosphorylation oxydative mitochondriale selon la revendication 1, dans lequel la faible expression ou l'absence d'expression du gène NNMT signifie que le rapport (E1/E0) entre le niveau d'expression E1 du gène NNMT dans la cellule tumorale et le niveau d'expression E0 du gène NNMT dans le même type de cellule est ≤ 0,7;
la tumeur présentant une expression élevée de la méthylase d'ADN signifie que le rapport (A1/A0) entre le niveau d'expression A1 de la méthylase d'ADN dans la cellule tumorale et le niveau d'expression A0 de la méthylase d'ADN dans le même type de cellule est ≥ 1,2;
la tumeur présentant une expression élevée de UHRF1 signifie que le rapport (F1/F0) entre le niveau d'expression F1 de UHRF1 dans la cellule tumorale et le niveau d'expression F0 de UHRF1 dans le même type de cellule est ≥ 1,2;
le niveau élevé de méthylation du site nucléotidique du gène NNMT signifie que le rapport (L1/L0) entre le niveau de méthylation L1 du site nucléotidique du gène NNMT dans la cellule tumorale et le niveau de méthylation L0 du site nucléotidique du gène NNMT dans le même type de cellule est ≥ 1,2; et/ou
le niveau élevé de méthylation du site CpG d'ADN du gène NNMT signifie que le rapport (W1/W0) entre le niveau de méthylation W1 du site CpG d'ADN du gène NNMT dans la cellule tumorale et le niveau de méthylation W0 du site CpG d'ADN du gène NNMT dans le même type de cellule est ≥ 1,2.

6. Un inhibiteur de voie de phosphorylation oxydative mitochondriale selon la revendication 1, dans lequel la faible expression ou l'absence d'expression du gène NNMT signifie que le rapport (E1/E0) entre le niveau d'expression E1 du gène NNMT dans la cellule tumorale et le niveau d'expression E0 du gène NNMT dans le même type de cellule est ≤ 0,5;
la tumeur présentant une expression élevée de la méthylase d'ADN signifie que le rapport (A1/A0) entre le niveau d'expression A1 de la méthylase d'ADN dans la cellule tumorale et le niveau d'expression A0 de la méthylase d'ADN dans le même type de cellule est ≥ 2;
la tumeur présentant une expression élevée de UHRF1 signifie que le rapport (F1/F0) entre le niveau d'expression F1 de UHRF1 dans la cellule tumorale et le niveau d'expression F0 de UHRF1 dans le même type de cellule est ≥ 2;
le niveau élevé de méthylation du site nucléotidique du gène NNMT signifie que le rapport (L1/L0) entre le niveau de méthylation L1 du site nucléotidique du gène NNMT dans la cellule tumorale et le niveau de méthylation L0 du site nucléotidique du gène NNMT dans le même type de cellule est ≥ 2; et/ou
le niveau élevé de méthylation du site CpG d'ADN du gène NNMT signifie que le rapport (W1/W0) entre le niveau de méthylation W1 du site CpG d'ADN du gène NNMT dans la cellule tumorale et le niveau de méthylation W0 du site CpG d'ADN du gène NNMT dans le même type de cellule est ≥ 2.

7. Un inhibiteur de voie de phosphorylation oxydative mitochondriale selon la revendication 1, dans lequel la faible expression ou l'absence d'expression du gène NNMT signifie que le rapport (E1/E0) entre le niveau d'expression E1 du gène NNMT dans la cellule tumorale et le niveau d'expression E0 du gène NNMT dans le même type de cellule est ≤ 0,3;
la tumeur présentant une expression élevée de la méthylase d'ADN signifie que le rapport (A1/A0) entre le niveau d'expression A1 de la méthylase d'ADN dans la cellule tumorale et le niveau d'expression A0 de la méthylase d'ADN dans le même type de cellule est ≥ 5;
la tumeur présentant une expression élevée de UHRF1 signifie que le rapport (F1/F0) entre le niveau d'expression F1 de UHRF1 dans la cellule tumorale et le niveau d'expression F0 de UHRF1 dans le même type de cellule est ≥ 5;
le niveau élevé de méthylation du site nucléotidique du gène NNMT signifie que le rapport (L1/L0) entre le niveau de méthylation L1 du site nucléotidique du gène NNMT dans la cellule tumorale et le niveau de méthylation L0 du site nucléotidique du gène NNMT dans le même type de cellule est ≥ 5; et/ou
le niveau élevé de méthylation du site CpG d'ADN du gène NNMT signifie que le rapport (W1/W0) entre le niveau de méthylation W1 du site CpG d'ADN du gène NNMT dans la cellule tumorale et le niveau de méthylation W0 du site CpG d'ADN du gène NNMT dans le même type de cellule est ≥ 5.

8. Un inhibiteur de voie de phosphorylation oxydative mitochondriale selon la revendication 1, dans lequel la faible expression ou l'absence d'expression du gène NNMT signifie que le rapport (E1/E0) entre le niveau d'expression E1 du gène NNMT dans la cellule tumorale et le niveau d'expression E0 du gène NNMT dans le même type de cellule est ≤ 0,1;
la tumeur présentant une expression élevée de la méthylase d'ADN signifie que le rapport (A1/A0) entre le niveau d'expression A1 de la méthylase d'ADN dans la cellule tumorale et le niveau d'expression A0 de la méthylase d'ADN dans le même type de cellule est ≥ 10;
la tumeur présentant une expression élevée de UHRF1 signifie que le rapport (F1/F0) entre le niveau d'expression F1 de UHRF1 dans la cellule tumorale et le niveau d'expression F0 de UHRF1 dans le même type de cellule est ≥ 10;
le niveau élevé de méthylation du site nucléotidique du gène NNMT signifie que le rapport (L1/L0) entre le niveau de méthylation L1 du site nucléotidique du gène NNMT dans la cellule tumorale et le niveau de méthylation L0 du site nucléotidique du gène NNMT dans le même type de cellule est ≥ 10; et/ou
le niveau élevé de méthylation du site CpG d'ADN du gène NNMT signifie que le rapport (W1/W0) entre le niveau de méthylation W1 du site CpG d'ADN du gène NNMT dans la cellule tumorale et le niveau de méthylation W0 du site CpG d'ADN du gène NNMT dans le même type de cellule est ≥ 10.

9. Un inhibiteur de voie de phosphorylation oxydative mitochondriale selon la revendication 1, dans lequel le niveau élevé de méthylation du site nucléotidique du gène NNMT signifie que le niveau de méthylation (M%) du site nucléotidique du gène NNMT dans la cellule tumorale est ≥ 3% et ≤ M1%, où M1 est un nombre entier positif compris entre 3 et 100;
le niveau de méthylation du site nucléotidique du gène NNMT fait référence au rapport entre un nombre de nucléotides méthylés et le nombre total de nucléotides dans le gène NNMT;
le niveau de méthylation du site nucléotidique du gène NNMT comprend un niveau de méthylation du site nucléotidique dans une région promotrice du gène NNMT;
le niveau de méthylation du site nucléotidique du gène NNMT comprend un niveau de méthylation des sites nucléotidiques situés entre 1050pb avant un site de début de transcription et 499pb après un site de début de transcription dans le gène NNMT;
le niveau de méthylation du site nucléotidique du gène NNMT comprend un niveau de méthylation des sites nucléotidiques situés entre 1050pb et 193pb avant un site de début de transcription dans le gène NNMT;
le niveau de méthylation du site nucléotidique du gène NNMT comprend un niveau de méthylation des sites nucléotidiques situés entre 840pb et 469pb avant un site de début de transcription dans le gène NNMT;
le niveau de méthylation du site nucléotidique du gène NNMT comprend un niveau de méthylation des sites nucléotidiques entre deux sites quelconques (y compris les deux sites) choisis parmi le site 114165695, le site 114165730, le site 114165769, le site 114165804, le site 114165938, le site 114166050 et le site 114166066 sur le chromosome humain 11;
le niveau de méthylation du site nucléotidique du gène NNMT comprend un niveau de méthylation des sites nucléotidiques choisis parmi le site 114165695 sur le chromosome humain 11, le site 114165730 sur le chromosome humain 11, le site 114165769 sur le chromosome humain 11, le site 114165804 sur le chromosome humain 11, le site 114165938 sur le chromosome humain 11, le site 114166050 sur le chromosome humain 11, le site 114166066 sur le chromosome humain 11, et leurs combinaisons;
le niveau de méthylation du site nucléotidique du gène NNMT comprend un niveau de méthylation des sites nucléotidiques entre deux sites quelconques (y compris les deux sites) choisis parmi le site 1161, le site 1196, le site 1235, le site 1270, le site 1404, le site 1516 et le site 1532 dans la séquence nucléotidique de SEQ ID N°:1;
le niveau de méthylation du site nucléotidique du gène NNMT comprend un niveau de méthylation des sites nucléotidiques choisis parmi le site 1161 dans SEQ ID N°:1, le site 1196 dans SEQ ID N°:1, le site 1235 dans SEQ ID N°:1, le site 1270 dans SEQ ID N°:1, le site 1404 dans SEQ ID N°:1, le site 1516 dans SEQ ID N°:1, le site 1532 dans SEQ ID N°:1, et leurs combinaisons;
le niveau élevé de méthylation du site CpG d'ADN du gène NNMT signifie que le niveau de méthylation (M %) du site CpG d'ADN du gène NNMT dans la cellule tumorale est ≥ 3% et ≤ M2%, où M2 est un nombre entier positif compris entre 3 et 100;
le niveau de méthylation du site CpG d'ADN du gène NNMT fait référence au rapport entre un nombre de nucléotides CpG méthylés et le nombre total de nucléotides dans le gène NNMT;
le niveau de méthylation du site CpG d'ADN du gène NNMT fait référence au rapport entre un nombre de nucléotides CpG méthylés et le nombre total de nucléotides CpG dans le gène NNMT;
le niveau de méthylation du site CpG d'ADN du gène NNMT comprend un niveau de méthylation du site CpG d'ADN dans une région promotrice du gène NNMT;
le niveau de méthylation du site CpG d'ADN du gène NNMT comprend un niveau de méthylation des sites CpG d'ADN situés entre 1050pb avant un site de début de transcription et 499pb après un site de début de transcription dans le gène NNMT;
le niveau de méthylation du site CpG d'ADN du gène NNMT comprend un niveau de méthylation des sites CpG d'ADN situés entre 1050pb et 193pb avant un site de début de transcription dans le gène NNMT;
le niveau de méthylation du site CpG d'ADN du gène NNMT comprend un niveau de méthylation des sites CpG d'ADN situés entre 840pb et 469pb avant un site de début de transcription dans le gène NNMT;
Le niveau de méthylation du site CpG d'ADN du gène NNMT comprend un niveau de méthylation des sites CpG d'ADN entre deux sites quelconques (y compris les deux sites) choisis parmi le site 114165695, le site 114165730, le site 114165769, le site 114165804, le site 114165938, le site 114166050 et le site 114166066 sur le chromosome humain 11;
le niveau de méthylation du site CpG d'ADN du gène NNMT comprend un niveau de méthylation des sites nucléotidiques choisis parmi le site 114165695 sur le chromosome humain 11, le site 114165730 sur le chromosome humain 11, le site 114165769 sur le chromosome humain 11, le site 114165804 sur le chromosome humain 11, le site 114165938 sur le chromosome humain 11, le site 114166050 sur le chromosome humain 11, le site 114166066 sur le chromosome humain 11, et leurs combinaisons;
le niveau de méthylation du site CpG d'ADN du gène NNMT comprend un niveau de méthylation des sites nucléotidiques entre deux sites quelconques (y compris les deux sites) choisis parmi le site 1161, le site 1196, le site 1235, le site 1270, le site 1404, le site 1516 et le site 1532 dans la séquence nucléotidique de SEQ ID N°:1; et/ou
le niveau de méthylation du site CpG d'ADN du gène NNMT comprend un niveau de méthylation des sites nucléotidiques choisis parmi le site 1161 dans SEQ ID N°:1, le site 1196 dans SEQ ID N°:1, le site 1235 dans SEQ ID N°:1, le site 1270 dans SEQ ID N°:1, le site 1404 dans SEQ ID N°:1, le site 1516 dans SEQ ID N°:1, le site 1532 dans SEQ ID N°:1, et des combinaisons de ceux-ci.

10. Un inhibiteur de voie de phosphorylation oxydative mitochondriale selon la revendication 9, dans lequel M1 est égal à 5, 10, 15, 20, 25, 30, 35, 40, 45, 50, 55, 60, 65, 70, 80, 85, 90, 95 ou 100;
M2 est égal à 5, 10, 15, 20, 25, 30, 35, 40, 45, 50, 55, 60, 65, 70, 80, 85, 90, 95 ou 100;
les sites situés entre 1050 pb avant un site de début de transcription et 499 pb après un site de début de transcription dans le gène NNMT correspondent aux sites 951-2500 de la séquence nucléotidique telle que représentée dans SEQ ID N°:1;
les sites situés entre 1050pb et 193pb avant un site de début de transcription dans le gène NNMT correspondent aux sites 951 à 1808 de la séquence nucléotidique telle que représentée dans SEQ ID N°:1; et/ou
les sites situés entre 840pb et 469pb avant un site de début de transcription dans le gène NNMT correspondent aux sites 1161 à 1532 de la séquence nucléotidique indiquée dans SEQ ID N°:1.

11. Un inhibiteur de voie de phosphorylation oxydative mitochondriale selon la revendication 1, dans lequel la tumeur est choisie parmi un cancer du poumon, un carcinome rénal, un cancer du sein, un cancer du côlon, un cancer rectal, un cancer colorectal, un lymphome, une leucémie, un cancer du pancréas, une tumeur cérébrale, un cancer du foie, un cancer de la prostate, un mélanome et leurs combinaisons.

12. Un inhibiteur de voie de phosphorylation oxydative mitochondriale selon la revendication 11, dans lequel le cancer du poumon est choisi parmi un cancer du poumon non à petites cellules, un cancer du poumon à petites cellules, un cancer du poumon métastatique et leurs combinaisons;
le cancer du côlon comprend un adénocarcinome du côlon;
le cancer rectal comprend un adénocarcinome rectal;
le cancer colorectal comprend un adénocarcinome colorectal;
le lymphome est choisi parmi un lymphome à cellules B, un lymphome à cellules T, un lymphome cutané à cellules T, un lymphome à grandes cellules, un lymphome histiocytaire et leurs combinaisons;
le lymphome comprend un lymphome diffus à grandes cellules B;
la tumeur cérébrale est choisie parmi un glioblastome, un neurogliome et leurs combinaisons;
la tumeur cérébrale comprend un médulloblastome cérébral;
la tumeur cérébrale comprend un glioblastome multiforme;
le carcinome rénal est choisi parmi un adénocarcinome rénal à cellules claires, un carcinome rénal métastatique et leurs combinaisons;
la leucémie est choisie parmi une leucémie à lymphocytes T, une leucémie myéloïde et leurs combinaisons;
le cancer de la prostate est choisi parmi un cancer métastatique de la prostate;
le cancer du sein est choisi parmi un carcinome canalaire du sein, un cancer du sein métastatique et leurs combinaisons; et/ou
le cancer du pancréas comprend un cancer du pancréas métastatique au foie.

13. Un inhibiteur de voie de phosphorylation oxydative mitochondriale selon la revendication 1, dans lequel l'inhibiteur de voie de phosphorylation oxydative mitochondriale est choisi parmi:
